(19) Europäisches Patentamt
European Patent Office
Office européen des brevets

(11) **EP 4 574 132 A1**

(12) **EUROPEAN PATENT APPLICATION**
published in accordance with Art. 153(4) EPC

(43) Date of publication:
**25.06.2025 Bulletin 2025/26**

(21) Application number: 23855169.1

(22) Date of filing: **17.08.2023**

(51) International Patent Classification (IPC):
*A61K 9/00* (2006.01)   *A61K 41/00* (2020.01)
*A61P 25/00* (2006.01)   *A61P 3/04* (2006.01)
*A61P 25/08* (2006.01)

(52) Cooperative Patent Classification (CPC):
A61K 9/00; A61K 41/00; A61P 3/04; A61P 25/00;
A61P 25/08

(86) International application number:
**PCT/KR2023/012209**

(87) International publication number:
**WO 2024/039203 (22.02.2024 Gazette 2024/08)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **17.08.2022 KR 20220102946**

(71) Applicants:
• **UIF (University Industry Foundation), Yonsei
University
Seoul 03722 (KR)**
• **Institute for Basic Science
Yuseong-gu
Daejeon 34126 (KR)**

(72) Inventors:
• **CHEON, Jinwoo
Seoul 08004 (KR)**

• **LEE, Jae-Hyun
Seoul 06640 (KR)**
• **KWAK, Minsuk
Gwangmyeong-si, Gyeonggi-do 14349 (KR)**
• **CHOI, Seo-Hyun
Seoul 04423 (KR)**
• **PARK, Chanhyun
Seoul 03782 (KR)**

(74) Representative: **Kellas, Fiona et al
Maucher Jenkins
Seventh Floor Offices
Artillery House
11-19 Artillery Row
London SW1P 1RT (GB)**

Remarks:
The complete document including Reference
Table(s) and the Sequence Listing(s) can be
downloaded from the EPO website

(54) **MAGNETIC TORQUER CONJUGANT-BASED DRUG**

(57)    The present invention relates to a Magnetic Torquer (m-Torquer) conjugate-based drug. Specifically, according to the present invention, an m-Torquer conjugate, comprising: (a) a magnetic nanoparticle that generates rotational force upon application of a controlled rotating magnetic field, wherein the working distance from the rotating magnetic field generator is at least 1 cm, preferably at least 2 cm, 10 cm, more preferably at least 20 cm, 30 cm, and most preferably at least 60 cm or 70 cm; (b) a binding moiety that binds to a drug target protein present in the biological lipid membrane; and (c) a linker or a direct attachment between the magnetic nanoparticle and the binding moiety, wherein, when the m-Torquer conjugate binds to the drug target protein, the rotational force generated by the magnetic nanoparticle upon application of a controlled rotating magnetic field is transmitted to the drug target protein, inducing a conformational change in the drug target protein. Accordingly, the m-Torquer conjugate-based drug may be used as a therapeutic agent that modulates biological signaling, structural integrity, metabolism, function, and regulation in the body.

EP 4 574 132 A1

[Fig. 19]

**Description**

**TECHNICAL FIELD**

The present invention relates to a Magnetic Torquer (m-Torquer) conjugate-based drug. Specifically, according to the present invention, an m-Torquer conjugate, comprising: (a) a magnetic nanoparticle that generates rotational force upon application of a controlled rotating magnetic field, wherein the working distance from the rotating magnetic field generator is at least 1 cm, preferably at least 2 cm, 10 cm, more preferably at least 20 cm, 30 cm, and most preferably at least 60 cm or 70 cm; (b) a binding moiety that binds to a drug target protein present in the biological lipid membrane; and (c) a linker or a direct attachment between the magnetic nanoparticle and the binding moiety, wherein, when the m-Torquer conjugate binds to the drug target protein, the rotational force generated by the magnetic nanoparticle upon application of a controlled rotating magnetic field is transmitted to the drug target protein, inducing a conformational change in the drug target protein. Accordingly, the m-Torquer conjugate-based drug may be used as a therapeutic agent that modulates biological signaling, structural integrity, metabolism, function, and regulation in the body.

[National R&D Program Supporting This Invention]

Project Identification Number: 1711170979
Project Number: IBS-R026-D1-2022-A00
Ministry: Ministry of Science and ICT
Project Management Agency: Institute for Basic Science (IBS)
Research Program Name: External Research Center Program
Research Project Name: Nano-Bio Systems Convergence Science (8/8)
Executing Institution: Institute for Basic Science (IBS)

**BACKGROUND ART**

Most metabolic processes in the body are driven by complex biochemical reactions. Traditional approaches to regulating metabolic functions using chemical drug-based therapies have played a crucial role in treating various diseases. However, since drugs administered orally or by injection circulate systemically through the bloodstream, they often lead to undesirable side effects in addition to their therapeutic effects.

Metabolism in the human body is an essential function for survival, requiring the coordinated activity of various physiological systems within organs to maintain homeostasis. Homeostatic regulation in response to external stimuli, such as food intake, temperature fluctuations, energy expenditure, and infections, is governed by the immune, endocrine, and nervous systems. For instance, cytokines are signaling proteins primarily secreted by immune cells that induce changes in immune function, such as inflammation. The production and secretion of cytokines represent a metabolic process that is regulated by neural circuits. When metabolic processes, including immune function, become dysregulated due to partial organ damage, functional impairment, or neural circuit malfunction, pathological conditions or diseases can arise.

Unlike traditional pharmacological approaches that regulate neural signaling through chemical drugs, emerging alternative therapies have been developed to modulate neural signals artificially using electrical stimulation and other modalities to treat or alleviate immune and metabolism-related disorders. These approaches are collectively referred to as *electroceuticals* or *bioelectronic medicine.* Electroceuticals can be defined as therapeutic interventions that restore or maintain homeostasis by stimulating neural circuits using electrical signals, light, ultrasound, or other physical stimuli, instead of drugs thereby regulating metabolic functions.

The nervous system in animals is divided into the central nervous system (CNS) and the peripheral nervous system (PNS).

The CNS consists of the brain and spinal cord and is responsible for integrating and processing information. The PNS, on the other hand, functions to transmit sensory information from sensory organs to the CNS and to relay commands from the CNS to effectors.

The brain plays a crucial role in receiving sensory inputs and generating motor outputs. Animals not only perceive their overall metabolic state but also process environmental stimuli through sensory organs, which are then transmitted to the body. Sensory inputs from the body's surface, muscles, joints, and internal organs generate motor responses. The process of linking sensory input to motor output constitutes the core of brain information processing. In this process, the majority of human cortical neurons function as interneurons, connecting sensory neurons and motor neurons.

The peripheral nervous system (PNS) refers to all components of the nervous system except the central nervous system (CNS). It consists of nerves extending from the CNS to various parts of the body, connecting the nerves of organs, skin, and limbs to the CNS. The PNS is divided into afferent nerves (which transmit sensory information to the brain and spinal cord) and efferent nerves (which convey motor commands from the brain and spinal cord to organs or muscles). Functionally, the PNS is further classified into the somatic nervous system (SNS) and the autonomic nervous system (ANS). The somatic nervous system refers to the involuntary control of smooth muscles and glands and the autonomic nervous system refers to the involuntary control of smooth muscles and glands. The somatic nervous system is responsible for transmitting sensory information to the brain and spinal cord and relaying motor commands from the CNS to muscle fibers. Neural signals transmitted through the SNS can be utilized in controlling artificial limbs and detecting artificial sensory input. The autonomic nervous system regulates visceral reflexes and maintains homeostasis by controlling key biological processes such as blood pressure, body temperature, and metabolism. The ANS is further subdivided into the sympathetic nervous system and the parasympathetic nervous system. The sympathetic nervous system regulates physiological responses that help the body cope with environmental changes and potential threats by increasing heart rate, respiration, blood flow to muscles, sweat secretion, and pupil dilation. The parasympathetic nervous system counterbalances the sympathetic nervous system by slowing heart rate and respiration, reducing blood flow, and constricting pupils once the body is no longer in a stressful situation, thereby maintaining normal physiological functions. Additionally, the peripheral nerves play a critical role in connecting the brain and internal organs. Afferent peripheral nerve stimulation, which transmits visceral information to the brain, can be leveraged to modulate anxiety disorders.

When the function of the central nervous system (CNS), particularly the brain, is impaired, various neurological disorders such as stroke, depression, epilepsy, and Parkinson's disease may arise. In such cases, artificial neural stimulation can serve as a potential method to restore lost functions. This therapeutic approach is referred to as neuromodulation, and some of its effects have been clinically validated. For instance, deep brain stimulation (DBS) has been FDA-approved and is currently used as a medical intervention. However, apart from severe neurological disorders and total paralysis, implanting and stimulating neural electrodes in brain regions responsible for metabolism, sensation, and motor function remains controversial, particularly for individuals with intact cognitive function. This psychological resistance presents a significant barrier to the widespread adoption of such technologies. One of the key peripheral nerves, the vagus nerve, is connected to critical organs such as brown adipose tissue, the liver, and the pancreas. Compared to direct brain stimulation, neuromodulation via artificial stimulation of the peripheral nervous system presents a more acceptable alternative with less psychological resistance. Given this advantage, peripheral nerve stimulation-based neuromodulation is emerging as a promising avenue for the application of bioelectronic medicine (electroceuticals).

Since bioelectronic medicine (electroceuticals) requires implantation within the body, it must be equipped with a sufficient power source to sustain continuous operation of the physical stimulation source. Additionally, these devices must be designed to minimize functional impairments caused by electrode corrosion or immune responses within the dynamic internal environment of the body.

With recent discoveries regarding the biological effects of light, clinical studies investigating light-based therapies targeting specific regions in vivo have gained momentum. In parallel, bioelectronic medicine research has introduced various optical therapies, such as optogenetics and photodynamic therapy, leveraging advanced materials and structural innovations. Optogenetics, in particular, differs from conventional neural stimulation techniques by precisely targeting and modulating the function of highly localized neuronal populations. Compared to traditional electrical stimulation-based approaches, optogenetics offers significantly higher spatiotemporal resolution, making it an emerging therapeutic strategy for neurological disorders.

Another physical stimulation method used in bioelectronic medicine (electroceuticals) involves focused ultrasound stimulation. Ultrasound refers to sound waves with frequencies beyond the audible range, typically between 20 kHz and 2 MHz, which can be concentrated locally to induce specific biological responses through focused energy delivery. Notable applications include focused ultrasound thermal ablation for brain tumors, focused ultrasound neurosurgery for movement disorders such as Parkinson's disease and essential tremor, and focused ultrasound-based interventions for psychiatric disorders. Recently, clinical studies have been actively exploring the use of low-intensity transcranial focused ultrasound (LIFU) in Alzheimer's disease patients. These studies employ microbubble ultrasound contrast agents combined with intravenous administration and low-intensity transcranial focused ultrasound to temporarily open the blood-brain barrier (BBB), thereby promoting neuroplasticity, brain glucose metabolism, and cognitive function.

Many cells regulate their physiological functions through mechanisms such as modulating ion permeability across the cell membrane, releasing neurotransmitters at the presynaptic nerve fiber terminals, utilizing intracellular $Ca^{2+}$ for muscle contraction, and secreting catecholamines from the adrenal medulla.

Excitable cells, such as neurons and muscle cells, exhibit changes in their electrical membrane properties when appropriately stimulated. Specifically, when cations move into the negatively charged intracellular space, an excitation wave known as an action potential is generated. The link between membrane voltage changes and physiological responses involves dipolar molecular conformational changes in membrane-resident polar molecules, which subsequently modulate the opening and closing of ion channels. These structural transitions alter membrane ion permeability, leading to the generation of a gating current, which governs the dynamic regulation of ion flux across the cell membrane.

Ion channels are pore-forming proteins that primarily facilitate the generation and transmission of electrical signals in the membranes of cells constituting organs such as the brain, nervous system, cardiovascular system, and contractile muscles.

Extensive research is actively being conducted on the development of drugs targeting various ion channels and the study of diseases caused by ion channel mutations using gene replication, recombination, and expression of ion channel proteins. Moreover, several anesthetics, including volatile anesthetics, are known to induce anesthesia by modulating the function of voltage-gated or ligand-gated ion channels.

Various methods have been attempted to inhibit neural activity. Approaches involving pharmacological agents, electrical stimulation, or magnetic field-based stimulation have been extensively studied and are currently employed in clinical therapies. However, these three conventional methods share a fundamental limitation: they lack specificity for targeting distinct neuronal populations.

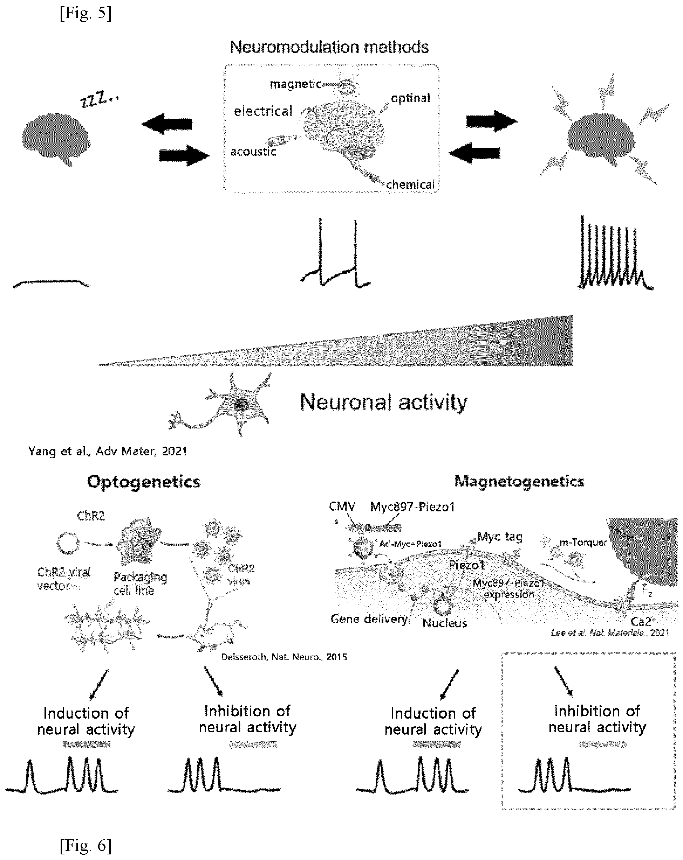

Optogenetic and chemogenetic approaches, using biological methods, have recently been developed as methods for cell-type-specific neuromodulation by inducing the expression of ion transport membrane proteins in targeted neuronal populations (Fig. 5). Optogenetics, which employs light to activate ion transport membrane proteins, offers the advantages of high-speed modulation and precise target specificity. However, due to the low optical transparency of most mammalian tissues, delivering light into deep brain regions often requires relatively invasive procedures, posing a significant limitation for in vivo applications.

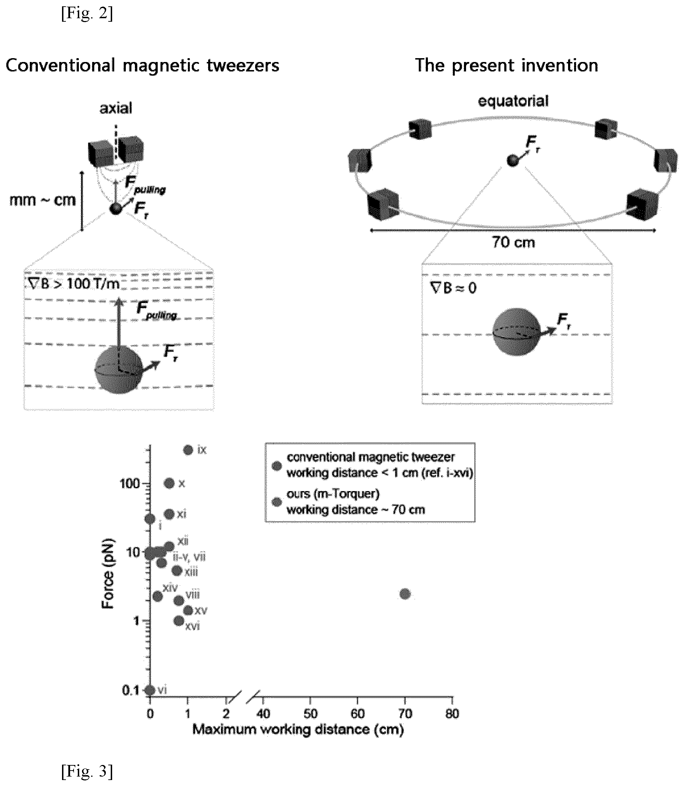

In contrast, magnetic fields can penetrate bone and tissue without significant loss of magnetic force, enabling the remote delivery of magnetic stimulation to ion transport membrane proteins and magnetic nanoparticles present in vivo, even when the magnetic field generator is positioned at a distance (~70 cm) from the target (Fig. 2). This characteristic suggests that magnetogenetics offers a less invasive alternative to optogenetics for modulating proteins in vivo, as it does not require surgical interventions that may cause damage to central nervous system structures such as the brain.

Chemogenetic approaches regulate neural activity by activating membrane proteins expressed in target cells via the administration of chemical compounds into the bloodstream, providing a relatively non-invasive method for neural modulation. However, a major limitation of chemogenetics is that the modulation of neural activity occurs over an extended period (approximately 30 minutes to 2 hours) following chemical administration, making it impossible to precisely control neural activity within a specific time window. In contrast, magnetogenetics enables the activation of ion transport membrane proteins expressed in target cells through the rotation of an externally applied magnetic field or an internal magnetic torque, allowing for the inhibition of neural activity with sub-second temporal precision.

Neural activity alternation through targeted stimulation delivery has emerged as a powerful tool in neuroscience. This approach to neuromodulation provides clinical strategies for restoring and enhancing brain function, offering therapeutic interventions for neurological and psychiatric disorders such as epilepsy, depression, traumatic brain injury, Parkinson's disease, and obesity. Neuroscientific research tools for the experimental interrogation of the nervous system provide insights into the functional connectivity of neural circuits and their roles in brain function. Over the past few decades, optogenetics has made significant advancements in the field of neuromodulation. This technology allows for versatile, cell-type-specific, light-based control of cellular activity with high temporal precision and resolution across various cell types, circuits, and brain structures. However, a major challenge in optogenetics is the limited tissue penetration of light, necessitating invasive implantation of optical fibers or intracranial light-emitting diodes (LEDs) for delivering illumination deep within the brain of living animals. Additionally, chemogenetic approaches, such as designer GPCR-based neuromodulation using designer drugs (DREADDs), offer a viable alternative to optogenetics. However, the prolonged systemic presence of chemical actuators in vivo results in poor temporal resolution.

The ability to achieve precise neuromodulation in a non-invasive manner has long been a major objective in neuroscience. Since the early 2000s, the development of reliable neuromodulation methods with cell-type specificity has established neuromodulation as an essential tool in modern neuroscience research for elucidating the functional organization of the nervous system. However, achieving both high spatiotemporal specificity and non-invasiveness remains a significant challenge.

## DETAILED DESCRIPTION OF THE INVENTION

### TECHNICAL PROBLEM

Remote regulation of cellular signaling has been a subject of interest for years, with principles proposed for controlling cellular signaling using light, ultrasound, and electric fields. However, these approaches have been limited by their insufficient penetration depth, restricting their practical applications.

Given their ability to penetrate deep tissues, magnetic fields have been investigated as a potential platform for remote biological signal modulation. However, the lack of suitably engineered magnetic particles and optimized magnetic field generators has significantly limited their practical application..

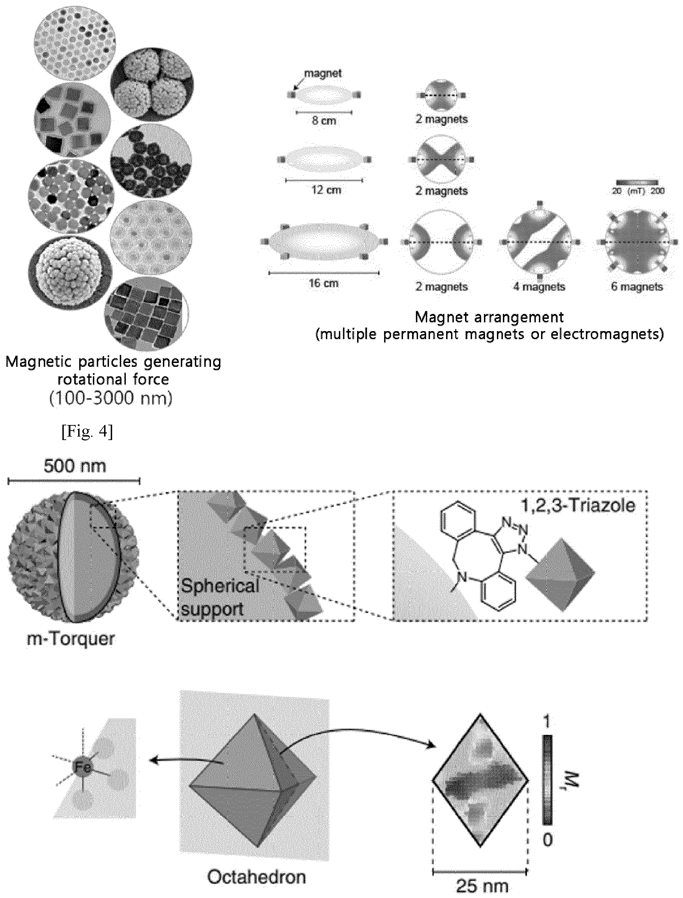

To address this issue, the inventors developed a solution involving magnetic nanoparticles and optimized magnet array configurations (Fig. 3), enabling neuromodulation at a working distance of approximately 70 cm using a rotating magnetic field in a non-invasive manner. Through this approach, the inventors demonstrated that the m-Torquer conjugates of the present invention, designed to function as pharmacological agents for physiological function regulation or behavioral modulation, successfully induced appetite suppression, reduced feeding behavior, and promoted weight loss in an obesity animal model. Furthermore, the m-Torquer conjugates were also shown to modulate social behaviors via neuromodulation. These findings provide the first evidence that the m-Torquer conjugates of the present invention can serve as locally targeted neuromodulatory agents that operate without physical tethering or invasive implantation.

## TECHNICAL SOLUTION

The first aspect of the present invention provides an m-Torquer conjugate-based drug, comprising: (a) a magnetic nanoparticle that generates rotational force upon application of a controlled rotating magnetic field, wherein the working distance from the rotating magnetic field generator is at least 1 cm, preferably at least 2 cm, 10 cm, more preferably at least 20 cm, 30 cm, and most preferably at least 60 cm or 70 cm; (b) a binding moiety that binds to a drug target protein present in the biological lipid membrane; and (c) a linker or a direct attachment between the magnetic nanoparticle and the binding moiety, wherein, when the m-Torquer conjugate binds to the drug target protein, the rotational force generated by the magnetic nanoparticle upon application of a controlled rotating magnetic field is transmitted to the drug target protein, inducing a conformational change in the drug target protein.

For example, the drug target protein present in the biological lipid membrane may be a cell surface protein.

For example, in the m-Torquer conjugate-based drug of the present invention, the m-Torquer conjugate can modulate the gating of ion channels embedded in the biological lipid membrane.

Accordingly, the m-Torquer conjugate-based drug of the present invention can be used as a physiological function-modulating drug, a behavior-regulating drug, or an anticancer agent by non-invasively delivering mechanical rotational force-based magnetic stimulation to targeted neural cells, secretory cells, muscle cells, or cancer cells upon the application of a rotating magnetic field.

Furthermore, the m-Torquer conjugate-based drug of the present invention can be used as a pain-modulating drug or a behavior-regulating drug by non-invasively delivering mechanical rotational force-based magnetic stimulation to targeted spinal cord neurons in the central nervous system upon the application of a rotating magnetic field.

Additionally, in the present invention, the drug target protein, to which the m-Torquer conjugate binds, can be expressed in specific cell types via a gene delivery carrier that enables targeted gene expression in a specific tissue region and cell population in vivo, thereby modulating the physiological or molecular functions of the target cells.

The second aspect (2-1) of the present invention provides a pharmaceutical composition for neuromodulation, comprising the m-Torquer conjugate-based drug of the first aspect.

For example, the m-Torquer conjugate-based drug may inhibit neuronal activity by activating anion transport membrane proteins.

For example, the m-Torquer conjugate-based drug may be used to treat epilepsy, attention-deficit/hyperactivity disorder (ADHD), chronic pain disorders, or to suppress pain perception.

The second aspect (2-2) of the present invention provides a pharmaceutical composition for weight loss or obesity treatment, comprising the m-Torquer conjugate-based drug of the first aspect.

The second aspect (2-3) of the present invention provides a pharmaceutical composition for physiological function regulation or behavioral modulation, comprising the m-Torquer conjugate-based drug of the first aspect.

The second aspect (2-4) of the present invention provides a pharmaceutical composition for the prevention or treatment of metabolic diseases, comprising the m-Torquer conjugate-based drug of the first aspect, as a drug capable of remotely and reversibly delivering mechanical torque-based magnetic stimulation over an extended period upon the application of a controlled rotating magnetic field from a rotating magnetic field generator.

The second aspect (2-5) of the present invention provides a pharmaceutical composition for inducing intracellular anion influx, comprising the m-Torquer conjugate-based drug of the first aspect.

The aforementioned pharmaceutical compositions of the present invention may be co-administered with a carrier that delivers the gene encoding a drug target protein, to which the m-Torquer conjugate can bind, thereby enabling targeted expression of the drug target protein in a specific cell type within a specific region in vivo. In particular, the m-Torquer conjugate-based drug of the first aspect can be used to induce activation and/or inhibition of specific neurons by expressing the drug target protein in targeted neurons within a specific region in vivo.

The third aspect of the present invention provides a kit comprising: (i) the m-Torquer conjugate-based drug of the first aspect; and (ii) a carrier engineered to deliver a gene encoding a drug target protein, to which the m-Torquer conjugate can bind, wherein the carrier enables selective expression of the drug target protein in a specific population of cells within a defined region in vivo.

The invention is further described below.

According to the present invention, the magnetic nanoparticle that generates rotational force upon application of a rotating magnetic field (also referred to as m-Torquer) is designed to harness the effects of a rotating magnetic field. Specifically, the m-Torquer nanoparticles are engineered to induce rotational force when subjected to a rotating magnetic field.

The magnetic stimulation associated with the m-Torquer conjugate of the present invention refers to a magneto-mechanical stimulation, wherein, when the m-Torquer conjugate binds to the drug target protein, the rotational force generated by the magnetic nanoparticle upon application of a controlled rotating magnetic field is transmitted to the drug target protein, inducing a conformational change in the target protein.

As used herein, neuromodulation refers to the process in which, when the m-Torquer conjugate binds to the drug target protein of a neural cell, the rotational force generated by the magnetic nanoparticle upon application of a controlled rotating magnetic field induces a conformational change in the drug target protein, thereby triggering the release of neural signals from the neural cell.

As used herein, a drug refers to any substance used for the diagnosis, cure, alleviation, treatment, or prevention of a disease (excluding food or medical devices) or for modulating biological signaling, structure, metabolism, function, or regulation in a living organism.

The m-Torquer conjugate-based drug of the present invention can be administered or injected and is not limited by the site or method of administration as long as it is applicable in vivo.

As used herein, the m-Torquer conjugate-based drug may refer to a pharmaceutical composition comprising the m-Torquer conjugate as an active pharmaceutical ingredient.

When the m-Torquer conjugate-based drug of the present invention is bound to a drug target protein, the rotational force generated by magnetic nanoparticles upon application of a controlled rotating magnetic field can induce conformational or biological activity changes in the drug target protein, which may be reversible.

Accordingly, in the present invention, the activity of the drug target protein can be modulated with a desired level of temporal precision and resolution, by delivering a magnetic stimulation pattern that governs the conformational change of the drug target protein bound to the m-Torquer conjugate.

In general, the in vivo efficacy and in vivo side effects of a drug are closely related to its absorption, distribution, metabolism, and excretion (ADME) properties. The ADME characteristics of a drug determine its pharmacokinetics, describing how the drug moves through the body and interacts with tissues and organs. In particular, the rate and route of excretion can impact both the drug's efficacy and side effects-rapid elimination may lead to a short half-life, reducing therapeutic effects, whereas slow elimination can cause drug accumulation, leading to toxicity.

In contrast, the rotational force generated by magnetic nanoparticles upon application of a controlled rotating magnetic field can induce a reversible conformational or biological activity change in the drug target protein. As a result, the efficacy of the m-Torquer conjugate-based drug of the present invention can be precisely controlled through magnetic stimulation of the target protein , following a predefined stimulation pattern, rather than being dependent on the drug's half-life.

The m-Torquer conjugate bound to a drug target protein in accordance with the present invention can function as a pharmacological agent that delivers localized magnetic stimulation to cells expressing the drug target protein in a freely moving animal within the space of a rotating magnetic field, without requiring physical restraints or invasive procedures.

The m-Torquer conjugate-based drug of the present invention may be used in combination with a carrier that facilitates the in vivo expression of the drug target protein, to which the m-Torquer conjugate can bind, in specific cells within a designated region of the body.

In this case, the carrier facilitates the expression of the drug target protein in the biological lipid membrane of specific cells within a designated region. Upon administration of the m-Torquer conjugate-based drug of the present invention, the m-Torquer conjugate binds to the drug target protein, and under the application of a controlled rotating magnetic field, the rotational force generated by the magnetic nanoparticle is locally transmitted to the drug target protein, inducing conformational changes.

In neuroscience research, where the nervous system is regulated by heterogeneous neuronal populations and complex neural circuits, cell-type-specific activation tools serve as versatile and innovative technologies for elucidating neurological disorders and fundamental neurophysiological mechanisms. A non-invasive, high-temporal-resolution neuromodulation technique capable of selectively modulating specific neuronal populations can be applied not only to control the neural activity of specific cell groups but also to transiently modulate specific neural projections during animal behavior.

The inventors have demonstrated for the first time that the m-Torquer conjugate-based drug according to the first aspect of the present invention enables magneto-mechanical stimulation of specific neuronal populations within the lateral hypothalamus (LH) in the deep brain region, thereby modulating behavior in freely moving animals (Example 2).

Specifically, Example 2, which utilizes the m-Torquer conjugate-based drug of the present invention, establishes a novel mode of action (MoA) in which that magneto-mechanical stimulation of specific LH neuronal populations in the deep brain region controls the behavior of freely moving animals. In Example 2, the inventors utilized the Cre-loxp system to selectively express magnetogenetic ion channels in LH neurons, which play a key role in feeding behavior regulation.

The study confirmed that cell-type specific expression of the target drug protein in Vgat (Slc32a1)-positive and Vglut2 (Slc17a6)-positive LH neurons, along with m-Torquer conjugate-based magneto-mechanical stimulation, could bidirectionally regulate feeding behavior. Moreover, the study demonstrated that transient modulation of feeding behavior confirmed reversibility. Furthermore, the results showed that sustained and repeated activation of specific LH neurons led to long-term anti-feeding effects, resulting in attenuation of obesity in obese mice. Lastly, the findings indicate that magnetogenetics can be adapted for various neuroscience studies, particularly for selective interrogation of the LH-VTA circuit in the context of social interactions under less restrictive conditions.

Example 2 establishes m-Torquer conjugate-based drugs as a significant neuromodulatory drug modality by employing a minimally invasive remote animal behavior control technology. This study utilizes a controlled rotating magnetic field generated by a rotating magnetic field generator, which induces rotational force in magnetic nanoparticles conjugated with a binding moiety targeting a drug target protein. Furthermore, Example 2 not only demonstrates a promising therapeutic approach for obesity by regulating feeding behavior and body weight, but also provides a versatile platform for controlling various animal behaviors, including social behavior.

Based on these findings, the present invention provides a new pharmacological modality, wherein a m-Torquer conjugate-based drug comprises (a) a magnetic nanoparticle that generates rotational force upon application of a controlled rotating magnetic field, wherein the working distance from the rotating magnetic field generator is at least 1 cm, preferably at least 2 cm, 10 cm, more preferably at least 20 cm, 30 cm, and most preferably at least 60 cm or 70 cm; (b) a binding moiety that binds to a drug target protein present in the biological lipid membrane; and (c) a linker or a direct connection between the magnetic nanoparticle and the binding moiety; and wherein, when the m-Torquer conjugate-based drug binds to the drug target protein, the rotational force generated by the magnetic nanoparticle upon application of a controlled rotating magnetic field is transmitted to the drug target protein, inducing a conformational change in the drug target protein.

Accordingly, when the drug target protein is expressed on the membrane of a neuronal cell, the m-Torquer conjugate of the present invention , upon binding to the drug target protein, can modulate neural circuits and behaviors in freely moving animals within the spatial region of a rotating magnetic field.

Additionally, the present invention provides that when a drug target protein is expressed on the cell membrane of a specific neuronal cell in a defined brain region, the m-Torquer conjugate bound to the drug target protein can, upon the application of a rotating magnetic field, generate a magnetic stimulation pattern that selectively activates and/or inhibits the specific neuronal cell. This modulation can regulate the release and levels of neurotransmitters (e.g., glutamate, gamma-aminobutyric acid (GABA), dopamine) that mediate neuronal signaling to other brain regions.

Moreover, the m-Torquer conjugate-based drug of the present invention can modulate animal behavior through the application of a controlled rotating magnetic field in a predetermined pattern or treat physiological disorders or diseases through long-term, neuron-specific neuromodulation.

Additionally, the m-Torquer conjugate-based drug of the present invention or its metabolites can be designed to be cleared by glial cells (neuroglia) or eliminated via lymphatic drainage from the cerebrospinal fluid (CSF).

**[Demonstration of the Drug Modality and Mode of Action (MoA) of the m-Torquer Conjugate (Example 2)]**

Neuroscience has undergone a revolutionary transformation with the advent of cutting-edge technologies that enable cell-type specificity, real-time bidirectional control of neural activity, and circuit-level manipulations. However, despite the potential for long-range working distance, prior methods have failed to demonstrate neural circuit-based behavioral control via deep brain neuromodulation in freely moving animals. Therefore, it is crucial to precisely define and study specific cell types.

Furthermore, the use of magnetogenetics enables non-invasive, wireless neural stimulation in various behavioral paradigms. This approach offers a novel strategy that provides a long-lasting neural interface in brain regions, thereby facilitating long-term and repetitive neuromodulation for potential therapeutic applications in various diseases. Additionally, by eliminating the need for tethering or implanted stimulators, this method allows for the investigation of more complex behaviors (e.g., social behavior) in freely moving animals under more natural (less constrained) conditions. However, despite its potential, such nanotechnology-based magnetogenetic neuromodulation approaches have not yet been extensively reported.

The inventors designed a method for magneto-mechanically stimulating specific populations of excitatory or inhibitory neurons within deep brain regions in vivo by combining Cre-dependent expression of Piezo1 with cell-type-specific control of neural activity (Example 2).

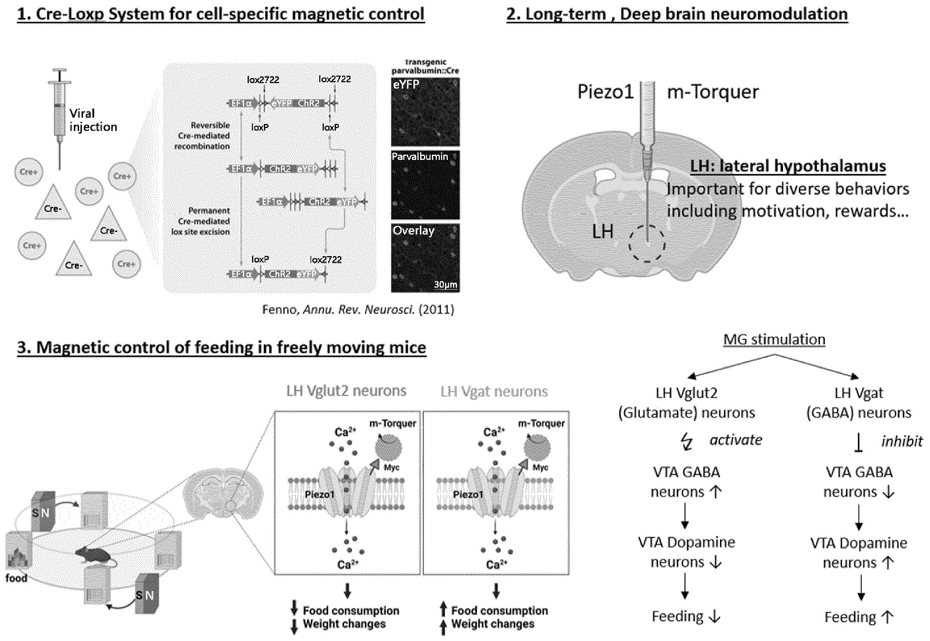

As illustrated in Fig. 18, the magnetogenetic tool designed using the m-Torquer conjugate-based drug of the present invention enables deep brain stimulation in freely moving mice without implantation or tethering. This is achieved through the rotational force generated by the m-Torquer, stereotactically injected into the target region, upon application of a controlled rotating magnetic field. Specifically, this system activated ectopically expressed, mechanically sensitive transient receptor Piezo1 in lateral hypothalamic (LH) neurons. Using a rotating circular magnet array (CMA) positioned above the animal's head-at a distance exceeding 70 cm, which is the bore size of a standard human magnetic resonance imaging (MRI) device-along with an m-Torquer serving as a nanoscale magnetic torque actuator, the system successfully activated Piezo1-expressing neurons in the LH of the mouse brain.

Example 2, which validates the drug modality of the m-Torquer conjugate that generates rotational force upon the application of a controlled rotating magnetic field, demonstrated that specific magneto-mechanical stimulation can bidirectionally control feeding behavior. This was achieved using Cre-loxP technology to selectively express magnetogenetic ion channels and by targeting lateral hypothalamic (LH) neurons expressing the synaptic vesicular transport proteins Vgat (Slc32a1) and Vglut2 (Slc17a6), which are involved in feeding regulation. Furthermore, the study confirmed the reversibility of feeding behavior modulation by demonstrating the transient control of feeding behavior. Additionally, long-term, repetitive activation of specific neurons in the LH induced a prolonged anti-feeding effect, leading to weight reduction in obese mice.

Moreover, using m-Torquer, which generates rotational force upon the application of a controlled rotating magnetic field, Piezo1-mediated stimulation of LH neurons was shown to regulate feeding behavior and reciprocal social behavior in mice. The activation of Vgat neuron-specific drug-target proteins in the LH facilitated feeding, whereas the activation of Vglut2 neuron-specific drug-target proteins in the LH induced food aversion. Specifically, by selectively activating glutamatergic and GABAergic neurons in the lateral hypothalamus (LH), feeding behavior in freely moving animals was successfully enhanced or suppressed, thereby verifying the role of LH neural circuits in feeding regulation. Furthermore, prolonged and repeated activation of Vglut2 neurons in the LH induced sustained anti-feeding effects, leading to significant weight loss and metabolic changes in obese mice. These findings confirm that the lateral hypothalamus (LH) plays a critical role in feeding-related behaviors. Finally, it was demonstrated that Vglut2-specific magnetogenetic neuromodulation in diet-induced obesity (DIO) mice effectively reduced food intake and alleviated obesity.

Furthermore, according to the present invention, the m-Torquer conjugate, which generates rotational force upon the application of a controlled rotating magnetic field, has been demonstrated to modulate other types of animal behaviors, such as social interactions, through magnetogenetic activation in the deep brain.

Therefore, according to the present invention, the m-Torquer conjugate, which generates rotational force upon the application of a controlled rotating magnetic field, presents a novel approach to neuromodulation, specifically enabling long-term, non-invasive control of feeding-specific neural circuits for applications such as obesity treatment.

In summary, the Magnetic Torquer (m-Torquer) conjugate-based drug platform technology, a novel drug modality according to the present invention, provides, for the first time, a method for selectively targeting specific types of brain cells located in deep brain tissues and remotely regulating their activation in a wireless manner.

In Example 2, by utilizing cell-type-specific gene delivery techniques, it was demonstrated that selective gene expression and functional modulation in specific types of neurons can be achieved. Furthermore, the m-Torquer conjugate-based drug of the present invention enables targeting deep brain regions that are otherwise difficult to access using optogenetic technology, while delivering long-term stimulation. Remarkably, as a result, it was demonstrated that magnetogenetic technology, characterized by its remote and spatiotemporally precise control of brain neurons, can be utilized to treat metabolic disorders such as obesity, which are difficult to address in the short term (Figs. 18 to 30). Thus, the m-Torquer conjugate-based drug of the present invention introduces a new drug mechanism of action and drug modality applicable to neuromodulation and metabolic disorder treatment.

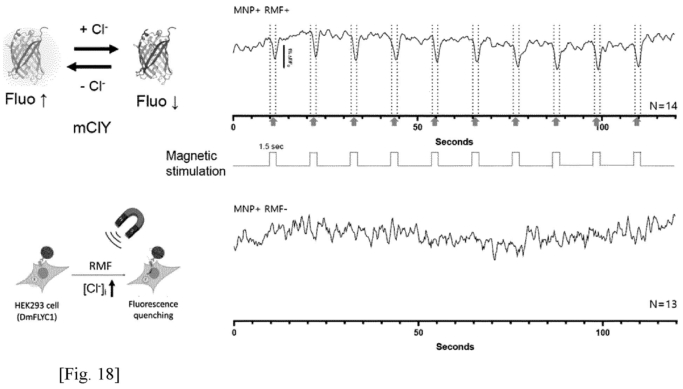

Example 2 utilized the Cre-LoxP system to selectively express mechanosensitive ion channels in specific types of brain cells. The novel drug modality of the present invention, the m-Torquer conjugate-based drug, was administered into deep brain regions, where it was molecularly targeted to mechanosensitive ion channels. The magnetic nanoparticle (m-Torquer) of the m-Torquer conjugate-based drug responded to the rotating magnetic field, generating a mechanical force (rotational torque) that activated the targeted ion channels, thereby enabling the selective modulation of specific neuronal activity (Figs. 17 to 30).

Notably, through the design of this novel drug modality, the m-Torquer conjugate-based drug was demonstrated in animal models to regulate neuronal activity in the lateral hypothalamus (LH), a central brain region for appetite control, thereby modulating feeding behavior and food intake. This study further established that the wireless, remote modulation of deep brain-specific cells was feasible and could deliver long-term stimulation (>10 days), outperforming existing deep brain modulation technologies such as optogenetics and chemogenetics.

In essence, the m-Torquer conjugate-based drug of the present invention enabled the long-term, selective control of specific neural circuits in the LH, leading to appetite suppression, reduced feeding behavior, and weight loss in an obese animal model. This breakthrough technology demonstrates the potential of a novel therapeutic approach for treating feeding-related disorders, such as overweight conditions, obesity, and anorexia, which pose significant public health challenges in modern human society.

**[rotating magnetic field generator]**

As used herein, the term "rotating magnetic field" may refer to a magnetic field in which magnetic field lines rotate around an imaginary axis of rotation in a direction perpendicular to that axis. It may also refer to a magnetic field that appears to rotate at a predetermined angular velocity over time. The angular velocity of the rotating magnetic field may, for example, be 0.01 Hz or higher and/or 10,000 Hz or lower, but it is not limited thereto. Additionally, a "reference plane" is defined as an imaginary plane in which the flux direction of the rotating magnetic field is horizontal, and the "magnetic field intensity" may refer to the value measured on this reference plane. Furthermore, the term "distance from the axis of rotation to the magnetic force-generating unit" refers to the arithmetic mean of the distances from the axis of rotation to multiple magnetic force-generating units.

The intensity of a magnetic field can be described by the magnetic flux density (B), which has the inherent property of being inversely proportional to the distance from its source. Due to this property, a significant challenge exists in applying a uniform-intensity magnetic field over long distances. Consequently, previous research on magnetic fields has been limited to implementing cellular activation only at short distances. Based on this understanding, the inventors of the present disclosure have discovered that the activity of target cells in vivo can be regulated using a rotating magnetic field that satisfies the equations below.

Based on this discovery, in the present invention, a rotating magnetic field generator that applies a controlled rotating magnetic field to induce rotational force in magnetic nanoparticles (m-Torquer) at a working distance of at least 20 cm, preferably at least 50 cm, and more specifically at approximately 70 cm may be designed to satisfy the following Equation 1 and Equation 2.

$$[\text{Equation 1}] \; |Mc| \geq 1 \; \text{mT}$$

$$[\text{Equation 2}] \; |M75\text{-}Mc|/D75 \leq 5.0 \; \text{T/m}$$

In Equations 1 and 2, Mc represents the magnetic field intensity at the position of the rotation axis, while D75 refers to the distance corresponding to 75% of the distance from the rotation axis to the magnetic force-generating unit, and M75 represents the magnetic field intensity at the D75 position.

Equation 1 pertains to the magnetic field intensity at the position of the rotation axis.

The rotating magnetic field generator used in the present invention applies a rotating magnetic field to induce torque in the magnetic nanoparticles (m-Torquer). As the direction of the magnetic flux rotates, the magnetic nanoparticles bound to the drug target protein transmit this torque to the drug target protein, thereby exerting mechanical force on it. In this process, the magnetic field intensity decreases as the distance from the magnetic force-generating unit increases. However, when Equation 1 is satisfied, the rotating magnetic field generator can still generate a sufficient level of torque even at its central region. Consequently, the m-Torquer conjugate bound to the drug target protein can modulate the activation or inactivation of the mechanosensitive drug target protein.

Equation 2 describes the relationship between the magnetic field intensity at the rotation axis position and the magnetic field intensity at a position corresponding to 75% of the distance from the rotation axis to the magnetic force-generating unit. Specifically, when Equation 2 is satisfied, the change rate of magnetic field intensity-i.e., the gradient of magnetic field intensity as a function of distance from the rotation axis-remains at 5.0 T/m or less. This means that when controlling the activation or inactivation (e.g., ion channel opening and closing) of mechanosensitive drug target proteins, the rotating magnetic field generator maintains a relatively stable magnetic field intensity variation. When Equation 2 is satisfied, the system can form a uniform rotating magnetic field over a specified distance from the center, thereby maximizing the internal space of the rotating magnetic field generator. This allows wider-area modulation of the activation or inactivation of drug target proteins (e.g., ion channel gating).

Additionally, the magnetic field generator in the present invention may further satisfy Equation 3 or Equation 4.

$$[\text{Equation 3}] \; |M50\text{-}Mc|/D50 \leq 1 \; \text{T/m}$$

In Equation 3, Mc represents the magnetic field intensity at the rotation axis position, D50 refers to the distance corresponding to 50% of the total distance from the rotation axis to the magnetic force-generating unit, and M50 represents the magnetic field intensity at the D50 position.

$$[\text{Equation 4}] \; |M75\text{-}M50|/(D75\text{-}D50) \leq 10 \; \text{T/m}$$

In Equation 4, D75 refers to the distance corresponding to 75% of the total distance from the rotation axis to the magnetic force-generating unit, while D50 refers to the distance corresponding to 50% of the total distance from the rotation axis to the magnetic force-generating unit. Additionally, M75 represents the magnetic field intensity at the D75 position, and M50 represents the magnetic field intensity at the D50 position.

Equation 3 relates to the magnetic field intensity at the rotation axis position and the magnetic field intensity at the midpoint between the rotation axis and the magnetic force-generating unit. A rotating magnetic field that satisfies Equation 3 can maintain a magnetic field gradient of 1.0 T/m or less as a function of distance from the rotation axis. When Equation 3 is satisfied, the central region around the rotation axis can maintain a nearly uniform magnetic field intensity. When modulating the activation/inactivation (e.g., ion channel opening and closing) of a drug target protein responsive to mechanical stimulation, a rotating magnetic field that satisfies Equation 3 can be applied, ensuring that within a specific spatial region, magnetic field variations remain minimal, thereby enabling precise activation/inactivation of drug target proteins (e.g., ion channels) over a broader area.

Equation 4 relates to the magnetic field intensity at the position corresponding to 75% of the total distance between the rotation axis and the magnetic force-generating unit, and the magnetic field intensity at the position corresponding to 50% of the total distance from the rotation axis to the magnetic force-generating unit. While Equations 2 and 3 describe the rate of change of magnetic field intensity in the central region of the rotating magnetic field generator, Equation 4 represents the rate of change of magnetic field intensity at the peripheral region of the rotating magnetic field generator. When Equation 3 is satisfied, the variation in magnetic field intensity at the peripheral region of the rotating magnetic field generator remains within a predefined range, thereby enabling more extensive and precise modulation of the activation/inactivation of drug target proteins (e.g., ion channels) over a larger spatial area.

Through the aforementioned rotating magnetic field generator, the m-Torquer conjugate-based drug of the present invention can provide sufficient rotational force to the m-Torquer-based drug, which serves as a physical stimulus bound to a drug target protein fixed in the biological lipid membrane, even at a working distance of 20 cm or more, preferably 50 cm or more, and up to 70 cm. Consequently, the rotating magnetic field generator or any of its components do not need to be implanted in the treatment subject, and a controlled rotating magnetic field can be applied without physically restraining the subject.

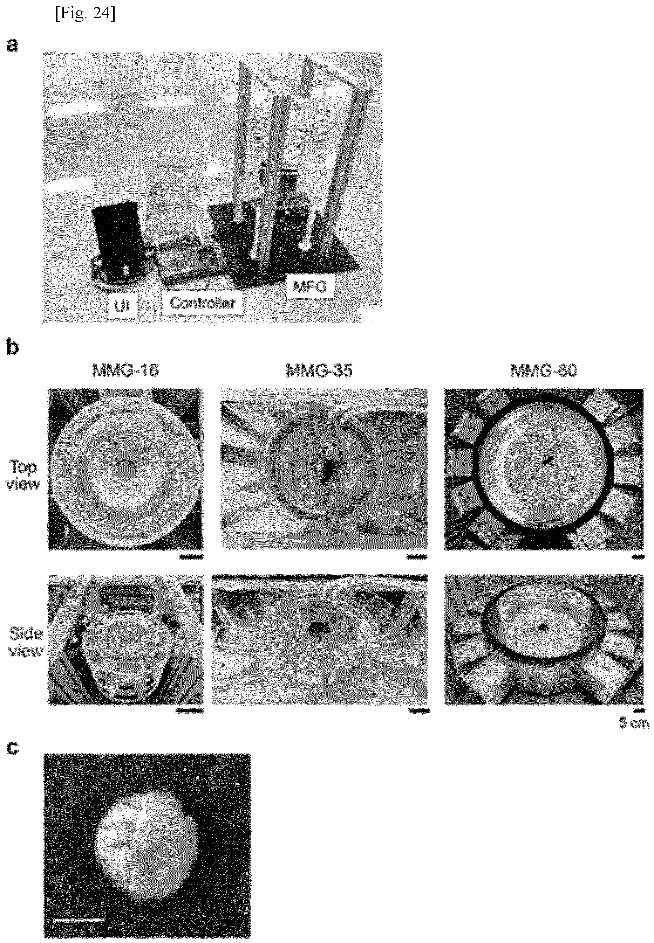

Magnetism has recently gained attention as a promising therapeutic modality due to its non-invasive nature and excellent tissue penetration capability. In Example 2, using the rotating magnetic stimulation generated by the rotating magnetic field generator of the present invention, a magnetogenetic technology was developed by conjugating m-Torquer with the mechanosensitive cation channel Piezo1, allowing for the precise perturbation of biological circuits (Fig. 4). The rotating magnetic field generator of the present invention (Fig. 24) enables the m-Torquer, which consists of an octahedral-shaped iron oxide nanoparticle coating, to move along the magnetic field lines, convert magnetic energy into mechanical force, and activate Piezo1, thereby depolarizing the excitable cells, even at a working distance of 20 cm or more.

**[(a) a magnetic nanoparticle that generates rotational force upon application of a controlled rotating magnetic field]**

Since magnetic fields can penetrate bones and tissues without loss of magnetic force, a magnetic field generator installed at least 20 cm away from the target-preferably at least 50 cm, and for example, approximately 70 cm- can still effectively deliver a magnetic field to drug target proteins present in vivo and the magnetic nanoparticles bound to them. Therefore, in the m-Torquer conjugate-based drug of the present invention, the magnetic nanoparticles that generate controlled rotational force upon the application of a rotating magnetic field from a rotating magnetic field generator positioned at a working distance of at least 1 cm, preferably at least 2 cm are illustrated in Figs. 3 and 4.

The m-Torquer conjugate-based drug of the present invention can locally stimulate diseased neurons or tissues, thereby minimizing side effects, and can be partially designed with a flexible and elastic material, ensuring functionality throughout various regions of the body.

The average diameter of the magnetic nanoparticles generating rotational force may range from 100 to 3,000 nm. If the size is too small, it may be difficult to generate the desired level of torque.

Fig. 4 is a schematic representation of the m-Torquer conjugate according to an embodiment of the present invention, illustrating the structure of the magnetic nanoparticles (m-Torquer) that generate rotational force upon application of a rotating magnetic field. The magnetic nanoparticles (m-Torquer) generating rotational force have a core-shell structure, where the shell contains octahedral unit magnetic nanoparticles, which may be randomly arranged on the core via 1,2,3-Triazole bonding.

**[(b) a binding moiety that binds to a drug target protein present in the biological lipid membrane]**

In the present invention, the "binding moiety for the drug target protein" refers to a binding region that interacts with the drug target protein and may be conjugated to the surface of the magnetic nanoparticle (m-Torquer) that generates rotational force. Accordingly, the m-Torquer conjugate of the present invention can function as a locally acting drug that selectively targets cells expressing the drug target protein and the tissues containing them, via the binding moiety (b) for the drug target protein.

The binding moiety for the drug target protein may include an antibody or its antigen-binding domain, a ligand or its receptor, a lipibody or an aptamer, or a molecule binding to a protein tag (e.g., a specific binding protein).

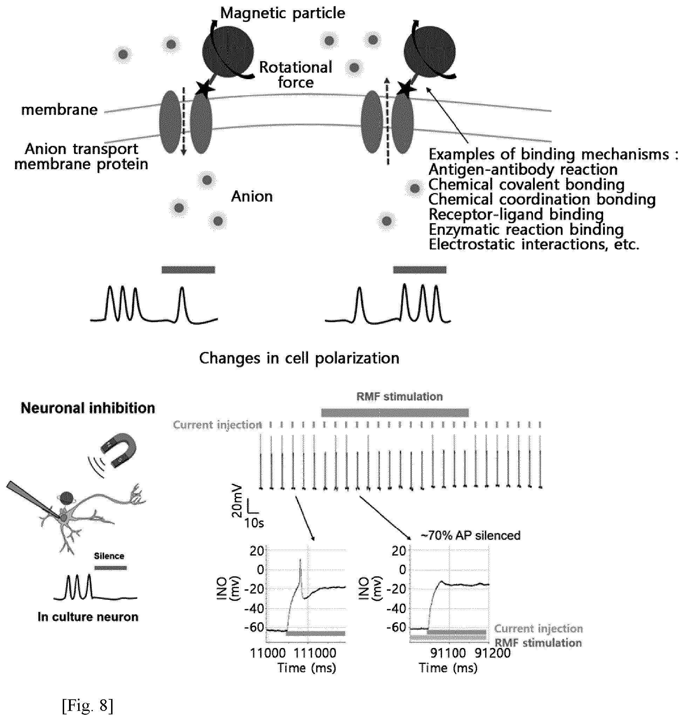

The binding interaction between the drug target protein and its binding moiety (b) may involve covalent bonding or protein-protein interactions. Non-limiting examples include antigen-antibody interactions, chemical covalent bonding, chemical coordination bonding, receptor-ligand binding, enzyme-substrate binding, and electrostatic interactions, as illustrated in Fig. 7.

The binding moiety (b) for the drug target protein can be optimized and designed in various ways based on a molecular-level understanding of the structure, function, and regulatory mechanisms of the targeted drug target protein.

For example, if the binding moiety for the drug target protein is an antibody, it can be immobilized onto the surface of the magnetic nanoparticle (a) that generates rotational force through covalent bonding using EDC/NHS chemistry. Alternatively, the antibody can be attached to the surface of the magnetic nanoparticle (a) via interactions with a pre-attached Protein A or Protein G.

**[drug target protein present in the biological lipid membrane]**

In the present invention, the drug target protein can be a protein present in the biological lipid membrane that undergoes conformational changes due to the rotational force of the magnetic nanoparticle in the bound m-Torquer conjugate. For example, the drug target protein present in the biological lipid membrane may be a receptor that undergoes conformational changes upon ligand binding, particularly an ion channel-linked receptor.

The conformational change of the drug target protein can lead to activation of intracellular signaling pathways and, in some cases, physiological responses. Such pathways may include gene expression, enzyme activity modulation, ion channel gating, or other cellular responses. The cellular responses can be highly diverse and may involve changes in cellular metabolism, cell division, muscle contraction, neural transmission, or other physiological processes.

A physiological response refers to biological changes an organism exhibits in reaction to its environment or stimuli, encompassing physical reactions and alterations in biological activity. Physiological responses may be triggered by emotions, stress, nutrition, or physical stimuli. Examples of physiological responses include changes in heart rate, respiratory rate, and blood pressure, among other bodily functions.

Accordingly, the m-Torquer conjugate of the present invention can serve as a drug that induces a physiological response through specific binding with a drug target protein (e.g., a receptor) capable of triggering a specific intracellular reaction or signaling pathway. In this context, the receptor specifically bound by the m-Torquer conjugate may be a drug target protein embedded in the biological lipid membrane. Additionally, in the m-Torquer conjugate, the binding moiety (b) that binds to the drug target protein can provide a targeting site for binding to the drug target protein through an affinity interaction, such as the binding relationship between an antibody and its antigen-binding domain or between a ligand and its receptor.

Thus, the m-Torquer conjugate-based drug of the present invention can be designed to act as a ligand such as a hormone, neurotransmitter, or other signaling molecules (e.g., cytokines, chemokines); an agonist, antagonist, enhancer, inhibitor, or blocker for the drug target protein; or a therapeutic antibody.

Furthermore, molecules or protein complexes capable of triggering a specific intracellular reaction or signaling pathway (e.g., lipids, glycans) are also within the scope of the drug target proteins of the present invention.

Moreover, as long as a controlled rotating magnetic field applied to the m-Torquer conjugate can trigger a specific intracellular reaction or signaling pathway, drug target proteins that are not embedded in the biological lipid membrane are also within the scope of the drug target proteins of the present invention.

Cells constitute the fundamental structural unit of living organisms and are organized into tissues, organs, and biological systems to perform various biological processes. Cells play an essential role in survival and reproduction, serving as the fundamental building blocks that contribute to the diversity and functionality of organisms. The size of a cell varies from approximately a few micrometers ($\mu$m) to several hundred micrometers, with human cells typically measuring around 100 $\mu$m.

In multicellular organisms, cells undergo morphological and functional differentiation, typically assembling into groups of similar cell types arranged to perform specific functions. Such organized cell groups are referred to as tissues. In animals, tissues are classified into epithelial tissue, connective tissue, cartilage tissue, bone tissue, blood and lymph, muscle tissue, and nervous tissue based on their morphology and function.

Epithelial tissue covers the surfaces of the body, the lumens of the digestive and respiratory tracts, and the peritoneal and pericardial cavities, forming a single-layered or multilayered sheet of tightly packed cells with minimal intercellular space. In some cases, epithelial tissue folds inward to form glandular tissues composed of secretory cells, while certain specialized epithelial structures, such as sensory epithelium in the visual, auditory, and vestibular systems, as well as structures like hair and nails, exhibit unique functional properties.

Connective tissue, cartilage tissue, bone tissue, blood, and lymph are collectively referred to as supporting tissues, which are characterized by an abundance of extracellular matrix. These tissues function to maintain the structural integrity of the body and organs. The extracellular matrix consists of fibers and a ground substance, with cells embedded within it. Blood and lymph are classified as supporting tissues because plasma and lymph fluid serve as the ground substance, while fibrin is considered the fibrous component of the extracellular matrix.

Muscle tissue consists of muscle cells specialized for contraction. These muscle cells, also known as muscle fibers, exhibit a long and slender fiber-like morphology due to their structural organization.

Nervous tissue comprises neurons (nerve cells) and glial cells (neuroglia) and functions as a wired communication system for transmitting biological information. In higher-order animals, the brain and spinal cord constitute the central nervous system (CNS), while the nerves branching from it form the peripheral nervous system (PNS). The CNS consists of nervous tissue combined with blood vessels and connective tissue, whereas the PNS is primarily composed of neurons, nerve fibers, and Schwann cells (a type of glial cell) that envelop the fibers.

Non-limiting examples of cells in which the drug target proteins of the m-Torquer conjugate may be naturally or artificially expressed include neurons, muscle cells, endocrine cells, and cancer cells.

In present invention, the biological lipid membrane where the drug target protein is located includes not only the plasma membrane but also the lipid bilayer of cellular organelles, such as mitochondria.

The cell membrane serves as a barrier, separating the interior and exterior of the cell. It is primarily composed of a lipid bilayer and protein molecules, enabling selective transport of substances across the membrane.

Eukaryotic cells, including human cells, possess an intracellular membrane system that compartmentalizes various organelles. The biological membranes constituting this system are composed of lipid bilayers, with proteins either embedded within or attached to the membrane surface.

Eukaryotic cells and mitochondria share interconnected membranes, with mitochondria-ER contact sites (MERCS) serving as dynamic interfaces involved in mitochondrial quality control, lipid and calcium homeostasis, protein homeostasis, and various intracellular signaling pathways. These MERCS act as structural platforms that optimize energy production and utilization in response to cellular environmental changes, thereby regulating cell survival, proliferation, and apoptosis.

The membranes of biological cells are typically phospholipid bilayers. The hydrophilic heads of phospholipids face outward, while the hydrophobic tails are oriented inward. Various functional proteins, including enzymes, are embedded within this bilayer, and additional molecules such as carbohydrates and lipids are arranged inside, outside, or within the membrane based on their physicochemical properties. The composition of ions differs across the biological membrane, leading to a difference in electrical potential between the interior and exterior of the cell. This electrochemical gradient arises due to differences in ion distribution and the selective permeability of the cell membrane. In most cells, the intracellular potential is approximately -50 to -100 mV relative to the extracellular environment. This membrane potential, known as the resting membrane potential (RMP) or transmembrane potential, must be continuously maintained to ensure cell survival and function.

**[ion channel as a drug target protein]**

As used herein, the terms "ion channel" and "ion pore" are used interchangeably.

The plasma membrane of eukaryotic cells consists of a lipid bilayer with embedded proteins or glycoproteins. Due to the hydrophobic nature of the membrane, hydrophilic molecules require specialized mechanisms to cross into the cell. The transport of small ions across the membrane occurs through regulated opening and closing of ion channels. Similarly, the lipid bilayer of intracellular organelles, such as mitochondria, also contains various ion pores. One of the essential functions of mitochondria, which play a critical role in cellular metabolism, is the regulation of the flow of signaling ions, such as $Ca^{2+}$.

In the m-Torquer conjugate-based drug of the present invention, the drug target protein that can be remotely and wirelessly modulated via the m-Torquer conjugate may include mechanosensitive ion channels or other membrane proteins. Accordingly, the m-Torquer conjugate-based drug of the present invention can induce changes in the electrical polarization of the cell membrane.

Ion channels are transmembrane transport proteins that facilitate the passage of specific ions across biological membranes. Their opening and closing are regulated by various intracellular and extracellular stimuli, and once activated, they enable the passive diffusion of ions following their electrochemical gradients.

Ion channel proteins are found in the biological membranes of all living organisms, with approximately 300 distinct ion channel proteins identified in humans.

Although ion pores exist in virtually all cellular membranes, they are predominantly concentrated in excitable tissues, including the central nervous system (CNS), autonomic ganglia, and neuromuscular junctions. Ionotropic receptor proteins typically consist of four to five subunits.

Ion channels are extremely narrow, allowing only small ions such as $Na^+$ and $K^+$ to pass through, while preventing the passage of larger particles. Ion channels selectively permit the passage of one or multiple types of ions, which are classified based on the ions they transport, such as $Na^+$, $K^+$, $Ca^{2+}$, or $Cl^-$ channels. These channels primarily regulate the resting membrane potential, the generation of electrical signals, the flow of $Ca^{2+}$ as a second messenger, cell volume regulation, and the overall ion flux in secretory cells.

Ion channels serve as a critical class of drug targets and are a subset of membrane transport proteins.

The drug-target proteins in the m-Torquer conjugate-based drug of the present invention include naturally occurring or genetically engineered recombinant membrane transport proteins.

As used herein, membrane transport proteins include ion channel proteins and active transport proteins, but are not limited thereto.

This definition encompasses both cation-transporting and anion-transporting membrane transport proteins.

The term cation-transporting proteins includes, but is not limited to, those that transport $Na^+$, $Ca^{2+}$, and $K^+$, as well as combinations thereof.

Similarly, anion-transporting proteins include, but are not limited to, those that transport $Cl^-$, as well as various combinations of anions.

Non-limiting examples of membrane transport proteins include the following:

$H^+$-transporting membrane proteins: Hv channels, proton pumps (H-type, V-type, F-type, and P-type)
$Na^+$-transporting membrane proteins: Nav channels, ASIC channels, EnaC channels
$Ca^{2+}$-transporting membrane proteins: Cav channels, $IP_3$ receptors, TPCN channels
$K^+$-transporting membrane proteins: Kv channels, Kca channels, Kna channels, $K_2p$ channels, KscA channels
Non-specific cation-transporting membrane proteins: TRP channels, Piezo channels, MscS, MscM, MscL channels
$Cl^-$-transporting membrane proteins: CLC channels, E-CLC channels, CLIC channels, GABA receptors
Non-specific anion-transporting membrane proteins: MscS channels, MscL channels, SWELL channels, ANO channels, Maxi anion channels, FLYC channels

Other ion pumps and transporters

Additionally, mutant variants of membrane transport proteins may be included. An example of such a mutant variant is a mutant form of DmFLYC1 protein, which may include single and/or multiple mutations at positions R599 (arginine 599), K606 (lysine 606), V656 (valine 656), and T659 (threonine 659). In one embodiment of the present invention, the mutant variants may include single mutants (e.g., R599K, K606R, V656L, and T659L), double mutants (e.g., V656L/T659L), triple mutants (e.g., R599K/V656L/T659L), quadruple mutants (e.g., R599K/K606R/V656L/T659L), or higher-order mutants. In Example 1, the DmFLYC1 mechanosensitive anion channel, which mediates chloride ion transport, is utilized. The ion transport membrane protein used in Example 1 is an anion channel that allows the influx of anions into the cell upon activation.

Notably, ligand-gated ion channels and voltage-gated ion channels are recognized as major drug targets.

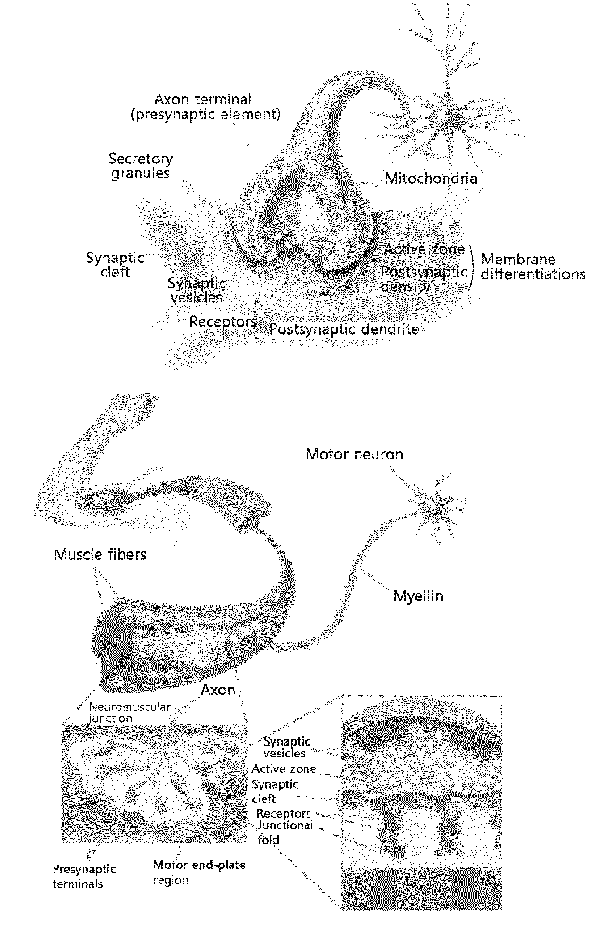

Ligand-gated ion channels are activated upon binding to an agonist, leading to the opening of the ion channel, thereby facilitating ion transport. This results in depolarization or hyperpolarization of the postsynaptic neuron or muscle cell membrane, allowing extracellular signals to be transduced into intracellular responses (Fig. 32).

Ligand-gated ion channels, also known as ion channel-linked receptors or transmitter-gated ion channels, are membrane proteins that mediate ion flux across the cell membrane in response to the binding of specific chemical messengers, such as neurotransmitters. These ion channels play a critical role in neuronal communication and synaptic transmission.

The basic structure of transmitter-gated ion channels consists of multiple subunits that assemble to form a functional channel complex. While the number and arrangement of subunits can vary depending on the channel type, the general organization follows a common structural pattern. The key components of transmitter-gated ion channels include the following:

Subunit Composition: Transmitter-gated ion channels are typically composed of multiple subunits that come together to form a pore through which ions can pass. The subunits are generally arranged symmetrically around a central ion-conducting pore. The number and type of subunits vary depending on the specific channel's properties and function.

Extracellular Domain: The extracellular domain of the ion channel contains binding sites for neurotransmitters. Each subunit usually has a ligand-binding domain that recognizes and binds to a specific neurotransmitter. When the neurotransmitter binds to this region, it induces a conformational change, leading to the opening or closing of the ion channel.

Transmembrane Domain: The transmembrane domain spans the lipid bilayer of the cell membrane and forms the ion-conducting pore. This region consists of multiple transmembrane segments (typically four or five) that create a hydrophilic pathway for ion passage. The precise arrangement and topology of these transmembrane segments vary based on the ion channel type.

Intracellular Domain: The intracellular domain of transmitter-gated ion channels interacts with intracellular signaling molecules and contributes to channel modulation and regulation. This region may contain phosphorylation sites, protein interaction domains, and regulatory motifs that influence channel activity.

When a neurotransmitter binds to the ligand-binding site in the extracellular domain, it induces a conformational change within the ion channel complex. This structural rearrangement leads to the opening of the ion channel pore, allowing specific ions to selectively pass across the membrane. The movement of these ions generates electrical currents, which influence the electrical activity and signaling properties of the neuron.

Different transmitter-gated ion channels exhibit selectivity for particular ions, such as sodium ($Na^+$), potassium ($K^+$), calcium ($Ca^{2+}$), or chloride ($Cl^-$) ions. This ion selectivity is determined by the properties of the transmembrane segments surrounding the ion channel pore, including the presence of specific amino acid residues that interact with ions in a size- and charge-dependent manner.

The fundamental structure of transmitter-gated ion channels enables rapid and precise signal transmission between neurons in response to neurotransmitters. The complex interactions among neurotransmitters, receptor subunits, and ion permeability contribute to the fine-tuning of neuronal communication and the regulation of synaptic activity.

In voltage-gated ion channels, changes in membrane potential trigger conformational changes in the ion channel protein, leading to the opening of the $Na^+$ ion channel gate and the subsequent influx of sodium ions. This influx causes the membrane potential to become more positive. $Na^+$ ions continue to enter the cell until the local membrane potential reaches a positive value. Once the membrane potential reverses from a negative to a positive value, the $Na^+$ ion channels close, and $K^+$ ion channels open, allowing $K^+$ ions to exit the cell. The outward flow of $K^+$ ions restores the membrane potential to its resting state. Additionally, channel gating can be influenced by intracellular second messengers, proteins, phosphorylation events, and mechanical stimuli.

Accordingly, the m-Torquer conjugate-based drug of the present invention is designed to bind to ligand-gated ion channels or voltage-gated ion channels through its binding moiety (b). Upon the application of a controlled rotating magnetic field, the resulting mechanical torque exerted by the magnetic nanoparticles induces a precise, temporally controlled conformational change in these ion channels. This enables fine-tuned regulation of ion channel activation, including the degree and duration of activation, gating frequency, and ion selectivity.

In higher-order animals, ion channels play essential roles in electrical signal generation and transmission, muscle contraction, hormone and neurotransmitter secretion, as well as intracellular and extracellular ion homeostasis. They also directly or indirectly regulate various cellular biological processes, including cell growth, differentiation, and intracellular signaling pathways. The physiological significance of these ion channels is exemplified by various ion channelopathies caused by genetic mutations. Representative examples include long QT syndrome, which results from mutations in cardiac $Na^+$ or $K^+$ channels, and cystic fibrosis, which is caused by mutations in the $Cl^-$ channels of airway epithelial cells.

Accordingly, the m-Torquer conjugate-based drug of the present invention enables the regulation of specific ion channel activity by leveraging the rotational force generated by magnetic nanoparticles under a controlled rotating magnetic field. By modulating the conformational changes of target ion channels, this approach allows for the precise control of cellular and physiological processes in which these ion channels are involved (Examples 1 and 2). Notably, by artificially enhancing or suppressing the activity of these ion channels, pathological conditions can be alleviated, thereby achieving therapeutic and pharmaceutical benefits.

Currently marketed blockbuster drugs include the antihypertensive drug amlodipine, which acts as an antagonist of voltage-gated $Ca^{2+}$ channels; the insomnia treatment drug zolpidem, which functions as an agonist of the $GABA_A$ receptor, a central nervous system $Cl^-$ channel; and the antiepileptic drug lamotrigine, which serves as a blocker of voltage-gated $Na^+$ channels.

**[Ion channel-linked receptors]**

The m-Torquer conjugate-based drug of the present invention targets ion-transport membrane proteins, either naturally or artificially expressed, and modulates the electrical polarization of the cell membrane by activating and/or inhibiting these ion-transport membrane proteins upon application of a controlled rotating magnetic field.

In particular, the m-Torquer conjugate-based drug is designed such that its binding moiety (b) interacts with ion channel-linked receptors, preferably by binding to their extracellular ligand-binding domain. Upon application of a controlled rotating magnetic field, the rotational force generated by the magnetic nanoparticles induces precisely timed conformational changes in the ion channel. This allows for the fine-tuned control of ion channel activation, including its degree and duration of activation, opening and closing frequency, and ion selectivity, thereby regulating neural signaling and transmembrane electrical signals.

Ion channel-linked receptors, also known as ionotropic receptors, represent a class of cell membrane receptors that are directly coupled to ion channels. These receptors play a fundamental role in neural signaling and transmembrane electrical signal transmission. Ion channels are broadly distributed across various tissues and are involved in synaptic transmission, sensory perception, and muscle contraction.

The fundamental structure of an ion channel-linked receptor consists of two primary components, an extracellular ligand-binding domain and a transmembrane ion channel domain. These structural elements are typically part of the same protein or exist as closely associated subunits.

The extracellular ligand-binding domain is located outside the cell and contains binding sites for specific chemical messengers, such as neurotransmitters or hormones. When these ligands bind to the receptor, the receptor protein undergoes a conformational change, leading to the activation of the associated ion channel.

The transmembrane ion channel domain spans the cell membrane and forms the ion-conducting pore through which ions can flow. This domain consists of multiple transmembrane segments that create a hydrophilic pathway for ion passage. The opening and closing of the ion channel are controlled by ligand-induced conformational changes in the extracellular domain.

When a ligand binds to the extracellular domain, the receptor undergoes a structural transformation, leading to the opening of the ion channel. This allows specific ions, such as sodium ($Na^+$), potassium ($K^+$), calcium ($Ca^{2+}$), or chloride ($Cl^-$), to flow across the cell membrane. The movement of these ions generates electrical currents that alter membrane potential and trigger downstream signaling events (Examples 1 and 2).

The activation of ion channel-linked receptors is typically rapid and transient, enabling fast signal transmission and electrical excitability in the nervous system. The duration of ion channel opening is generally brief, ranging from a few milliseconds to several seconds, depending on the specific receptor and its regulatory mechanisms.

Examples of ion channel-linked receptors include the nicotinic acetylcholine receptor, which is involved in neuromuscular transmission, and the NMDA (N-methyl-D-aspartate) receptor, which plays a crucial role in synaptic plasticity and learning in the brain.

Ion channel-linked receptors enable the direct coupling of ligand binding to ion flow, allowing for the efficient and precise transmission of signals across the cell membrane. The activation of these receptors and the subsequent ion conductance are critical for physiological processes and serve as therapeutic targets for various diseases and disorders.

Accordingly, the m-Torquer conjugate of the present invention is designed to bind to ion channel-linked receptors, preferably at the extracellular ligand-binding domain, such as the binding site for neurotransmitters or hormones. Furthermore, the m-Torquer conjugate can be engineered to respond to a precisely controlled rotating magnetic field with desired temporal resolution, spatial specificity, and frequency modulation, enabling targeted neuromodulation.

**[Regulation of Ligand-Receptor Interactions and Intracellular Signaling by m-Torquer Conjugate-Based Drug]**

The term "ligand-receptor interaction" refers to the specific binding and communication between a molecule (ligand) and a cellular structure (receptor). This interaction is a fundamental biological process that governs various physiological and biochemical responses in living systems.

A ligand is an ion or molecule that binds to the central region of a receptor (ion channel) to form a complex.

Ligands are typically highly specific to their receptors and can include hormones, neurotransmitters, signaling ions such as $Ca^{2+}$, drugs, or other signaling molecules such as cytokines and chemokines. In general, ligands are small molecules or ions with chemical properties that enable interaction with their respective receptors.

A receptor is a protein or molecular structure located on the cell surface or within the cell. Receptors contain specific binding sites that recognize and bind to ligands based on complementary shape and chemical properties. Upon ligand binding, the receptor triggers intracellular signaling pathways or specific cellular responses.

Accordingly, the m-Torquer conjugate-based drug of the present invention can be designed to modulate ligand-receptor interactions and intracellular signaling by acting as a ligand-such as a hormone, neurotransmitter, signaling ion (e.g., $Ca^{2+}$), drug, or other signaling molecules (e.g., cytokines, chemokines)-or as an agonist, antagonist, enhancer, inhibitor, or blocker for the receptor (i.e., the drug target protein) of such ligands, upon application of a controlled rotating magnetic field.

The ligand-receptor interaction process can be divided into several stages:

(1) Recognition: The ligand and receptor must possess compatible shapes and charges to interact. This interaction is highly specific, similar to how a key fits into a specific lock. In this regard, in the m-Torquer conjugate-based drug of the present invention, the binding moiety (b) for the drug target protein can be designed to be recognizable by the receptor.
(2) Binding: The ligand physically attaches to the receptor. This binding is generally reversible, allowing the ligand to dissociate from the receptor over time. Accordingly, in the m-Torquer conjugate-based drug of the present invention, the binding moiety (b) for the drug target protein can be designed to bind either reversibly or irreversibly to the receptor.
(3) Activation: Upon binding, the receptor undergoes a conformational change, which may lead to the activation of intracellular signaling pathways. These pathways may involve gene expression, enzymatic activity, ion channel opening, or other cellular responses. In this context, the m-Torquer conjugate-based drug of the present invention can be designed to precisely regulate the intensity and pattern of receptor conformational changes in real-time via the rotational force generated by the application of a controlled rotating magnetic field.

(4) Signal Transduction: The activated receptor initiates a series of intracellular events, commonly referred to as signal transduction, wherein signals from the receptor are transmitted to intracellular machinery that executes the appropriate response. In this regard, the m-Torquer conjugate-based drug of the present invention enables precise real-time control of ligand-receptor interactions and intracellular signaling via receptor conformational changes induced by the rotational force generated upon the application of a controlled rotating magnetic field.

(5) Response: The cellular response to ligand-receptor interaction can vary widely, including changes in metabolism, cell division, muscle contraction, neuronal signaling, and other physiological processes. In this context, the m-Torquer conjugate-based drug of the present invention is designed to precisely regulate cellular responses, including metabolism, cell division, muscle contraction, and neuronal signal transmission, in real time by controlling receptor conformational changes via the rotational force induced by the application of a controlled rotating magnetic field.

Non-limiting examples of ligand-receptor interactions include:

Hormones and Their Receptors: Hormones such as insulin, estrogen, and adrenaline bind to specific receptors on target cells, triggering various physiological responses.

Neurotransmitters and Synaptic Receptors: Neurotransmitters released at the synapse bind to receptors on adjacent neurons, facilitating neuronal signal transmission.

Drug-Receptor Interactions: Many drugs function by binding to specific receptors, either enhancing or inhibiting the receptor's normal function.

Understanding ligand-receptor interactions is critical for drug development, cellular process research, and elucidating the mechanisms underlying physiological functions and diseases.

The m-Torquer conjugate-based drug of the present invention enables real-time precision control of ligand-receptor interactions, including hormones and their receptors, neurotransmitters and synaptic receptors, and drug-receptor interactions, by inducing controlled conformational changes in receptors through the rotational force generated upon application of a controlled rotating magnetic field.

The term "cellular machinery" refers to the complex network of molecular components and processes that operate within a cell to perform various functions essential for cell survival, growth, and reproduction. These components include proteins, enzymes, organelles, and other molecules that work together to execute vital cellular functions. Overall, the cellular machinery consists of an intricate system of molecular interactions and processes that are tightly regulated and coordinated to ensure proper cellular function within the broader context of an organism.

The m-Torquer conjugate-based drug of the present invention enables real-time signaling to the cellular machinery by inducing controlled conformational changes in receptors through the rotational force generated upon application of a controlled rotating magnetic field.

The m-Torquer conjugate-based drug functions by binding to receptor proteins embedded in the biological lipid membrane of target cells, similar to a ligand or other signaling molecule. Upon application of a controlled rotating magnetic field, the rotational force of the magnetic nanoparticles induces conformational changes in the receptor, thereby activating a cascade of changes in intracellular signaling pathways that trigger specific cellular responses.

The m-Torquer conjugate-based drug of the present invention, upon application of a controlled rotating magnetic field, induces rotational force in magnetic nanoparticles, thereby facilitating signal transduction within the cellular machinery. While not limited thereto, the cellular machinery activated by the m-Torquer conjugate-based drug may perform the following activities:

(1) Gene Expression: The cellular machinery activated by the m-Torquer conjugate-based drug is responsible for transcribing DNA into RNA and translating RNA into proteins. This involves various enzymes, transcription factors, ribosomes, and other molecular components.

(2) Protein Synthesis and Folding: Ribosomes and associated molecules assemble amino acids into proteins. Chaperones assist in the proper folding of newly synthesized proteins.

(3) Energy Production: Mitochondria, often referred to as the "powerhouse of the cell," are involved in energy production through processes such as oxidative phosphorylation.

(4) Metabolism: Intracellular enzymes catalyze chemical reactions involved in various metabolic pathways, including nutrient breakdown and molecular synthesis.

(5) Cell Division: The cellular machinery activated by the m-Torquer conjugate-based drug coordinates processes such as DNA replication, chromosome segregation, and cytokinesis during cell division.

(6) Signal Transduction: Proteins and molecules within the cell transmit and amplify signals received from receptors, facilitating intracellular communication.

(7) Membrane Transport: The cellular machinery activated by the m-Torquer conjugate-based drug regulates the movement of molecules into and out of cells and organelles via processes such as endocytosis, exocytosis, and membrane pumps.

(8) Cytoskeletal Dynamics: The cytoskeleton, composed of protein filaments, provides structural support and facilitates cellular movement, shape changes, and intracellular transport.

(9) Cell-to-Cell Communication: The cellular machinery activated by the m-Torquer conjugate-based drug plays a role in cell signaling, allowing cells to communicate and respond to external stimuli.

Intercellular signaling-where sending cells transmit signals to receiving or target cells-is a fundamental mechanism for coordinating cellular activities. In most cases, signaling molecules bind to receptor proteins embedded in the biological lipid membrane of the target cell, thereby initiating a cascade of intracellular signal transduction events. Once a signaling molecule binds to its specific receptor, a series of relay proteins are activated, which in turn activate transcription factors. These activated transcription factors bind to DNA, triggering the transcription of specific genes. The mRNA message is then translated into new proteins, modifying the structure or function of the receiving cell. Since extracellular signaling molecules regulate this cascade, even a small signal can be amplified within the cell.

Accordingly, the m-Torquer conjugate-based drug of the present invention may be designed to function as a signaling molecule that transmits signals to receiving or target cells upon application of a controlled rotating magnetic field.

(10) DNA Repair and Maintenance: Various mechanisms and enzymes are responsible for preserving the integrity of genetic material and repairing damaged DNA.

Cells primarily interpret environmental changes through the binding of signal molecules to receptor proteins. The interaction between ligands and receptors induces changes in the structure, oligomeric state, and local environment of the receptor. These changes transduce the ligand-binding signal into intracellular pathways, leading to alterations in cellular function.

Although some signal transduction pathways differ in complexity, many share common characteristics.

Ligand binding induces structural changes or clustering of receptors.

When a ligand binds to a receptor, it triggers physicochemical modifications in the receptor itself or associated molecules. Many growth factor receptors undergo dimerization upon ligand binding, and dimerized receptor molecules subsequently undergo cross-phosphorylation of intracellular residues. For example, upon binding to an antigen ligand, the B lymphocyte receptor (BCR) undergoes a conformational change in its antigen-unbound region. This leads to BCR clustering, which subsequently forms multivalent complexes and migrates to specific regions within the cell membrane.

Some growth factors induce receptor dimerization, leading to cross-phosphorylation of receptor-associated tyrosine residues. The cytoplasmic domains of many growth factor receptors exhibit tyrosine kinase activity. Upon ligand binding, receptor dimerization occurs, initiating cross-phosphorylation at multiple sites, which triggers downstream signaling cascades.

Receptor clustering alters receptor localization. Ligand-induced receptor clustering results in a rearrangement of receptor positioning within the cell membrane.

Ligand-induced clustering of BCRs or TCRs slows their diffusion within the membrane, promoting their recruitment into lipid rafts-specialized microdomains within the plasma membrane enriched in cholesterol and sphingolipids. These lipid rafts serve as platforms where numerous key signal transduction molecules are concentrated, playing a critical role in receptor-mediated signaling.

Cytokines are small signaling molecules involved in intercellular communication among immune cells, while chemokines, a subclass of cytokines, specifically mediate chemotactic attraction or repulsion of cells.

The interaction between a cognate receptor and its ligand occurs through highly specific non-covalent binding. The extracellular domain of a membrane receptor recognizes and binds to its ligand.

Each lymphocyte expresses a single type of antigen receptor, but it also possesses diverse receptors for other signaling molecules, including cytokines and chemokines. At the moment of receptor-ligand binding, all recruited receptors integrate the received signals to generate a coordinated cellular response.

The binding of receptors to their ligands relies on non-covalent interactions, meaning that the interaction between the receptor molecule's surface and its corresponding ligand occurs through non-covalent forces.

Biologically meaningful receptor-ligand interactions require a combination of multiple forces, such as hydrogen bonding, ionic interactions, hydrophobic interactions, and Van der Waals forces. Because non-covalent interactions are inherently weak in aqueous environments, the structural complementarity between the receptor and ligand must be highly precise.

Signal transduction refers to the molecular pathway through which ligand-receptor interactions are converted into intracellular biochemical changes. The transmission of information by a ligand is initiated by the structural complementarity between the ligand and its receptor. The binding event must be sufficiently strong and sustained for a period long enough to induce biochemical modifications in the receptor and its associated signaling molecules.

Accordingly, in the m-Torquer conjugate-based drug of the present invention, the binding moiety (b) that interacts with the drug target protein can be engineered with an optimized affinity.

Furthermore, recognizing that signal transduction requires receptor-ligand binding to be sufficiently strong and long-lasting for efficient signaling, the present invention enables precise control over the rotating magnetic field strength and duration of exposure applied to the m-Torquer conjugate-based drug.

## [Nervous System, Neurons, and Neural Circuits]

The nervous system is an organ system responsible for transmitting stimuli and coordinating responses to various environmental changes.

The nervous system consists of the central nervous system (CNS), which includes the brain and spinal cord and plays a critical role in sensing overall metabolic status and regulating metabolism by releasing endocrine neuro-signals in response to sensory inputs, and the peripheral nervous system (PNS), which branches out from the CNS and extends throughout the body, transmits these released neural signals to target organs.

Accordingly, for neuromodulation, the m-Torquer conjugate-based drug of the present invention can be designed to magnetically stimulate targeted neurons by selectively binding to drug target proteins through its binding moiety (b) in either (i) CNS neurons, which regulate metabolism by releasing endocrine neuro-signals, or (ii) PNS neurons, which relay neural signals from the CNS to target organs. The magnetic stimulation can be precisely modulated using controlled patterned rotational magnetic field application.

The peripheral nervous system (PNS) is further divided into the autonomic nervous system (ANS), which controls involuntary functions of smooth muscle and glands, and the somatic nervous system (SNS), which governs voluntary muscular movements within the body.

The autonomic nervous system (ANS) innervates the heart, blood vessels, glands, and digestive organs, autonomously regulating internal bodily functions independently of conscious control. It plays a critical role in maintaining homeostasis in response to environmental changes. The ANS is further subdivided into the sympathetic nervous system (SNS) and the parasympathetic nervous system (PNS).

The central nervous system (CNS) consists of neuronal clusters in the brain and spinal cord, which are protected by the skull and vertebral column. The peripheral nervous system (PNS) comprises neuronal clusters distributed outside the brain and spinal cord, including those in the skin, muscles, and visceral organs.

In the central nervous system (CNS), which consists of the brain and spinal cord, a neural nucleus refers to a cluster of thousands to tens of millions of neuronal cell bodies densely packed within a structure ranging in size from a few millimeters to several centimeters.

In the peripheral nervous system, which consists of the 12 cranial nerves and spinal cord nerves, a collection of neurons is called a ganglia. As many as 100,000 neurons can be packed into a volume of 1 mm$^3$, and a single neuronal axon can extend tens of centimeters. The neuronal cell body (soma) is roughly 20 micrometers in size, but axons that are 1,000 times longer than the soma branch off from the cell body.

The key distinction between the CNS and PNS lies in the type of glial cells responsible for electrical insulation of axons. In the CNS, oligodendrocytes provide myelination, with a single oligodendrocyte capable of wrapping around multiple neurons. In contrast, in the PNS, Schwann cells myelinate axons, but each Schwann cell only insulates a single neuron. Consequently, oligodendrocyte dysfunction in the CNS can impair multiple neurons simultaneously, whereas damage to a single Schwann cell in the PNS only affects one neuron, allowing axonal regeneration and remyelination by newly formed Schwann cells.

A group of nerve cells in the central nervous system is called a nucleus, and a nerve cell cluster or a group of nerve cell bodies in the peripheral nervous system is called a ganglion. A ganglion is the site of synapse formation.

In vertebrates, nerves, blood vessels, and muscles are segmentally organized along spinal cord segments, forming metameric structures. Spinal nerves extend into muscle tissues, while skeletal muscles attach to structural components such as the limbs, vertebrae, and spinal column. The nerves that control the forelimbs and hindlimbs extend from the spinal cord, and at the caudal end of the spinal cord, nerve fibers densely converge in a bundle resembling a "horse's tail"-known as the cauda equina.

Brain function arises from neural circuits, which consist of interconnected neurons within the brain. For example, axons of thalamic neurons project to the cerebral cortex, while reciprocally, axons of cortical neurons form synapses with neurons in the thalamic nuclei.

For neuromodulation, the m-Torquer conjugate of the present invention can be designed to target specific neurons within the central nervous system (CNS), including the brain and/or spinal cord, and to apply a controlled rotating magnetic field in a specific pattern, thereby inducing stimulation to form desired neural circuit loops for brain function regulation.

A neural circuit loop refers to recurring patterns of connectivity and activity within neural circuits. These loops are essential for information processing and transmission, playing critical roles in learning, memory, motor control, and various other brain functions. Neural circuit loops arise from interactions between different brain regions, and they function by transmitting information via synaptic connections and processing electrochemical signals.

Therefore, by employing the m-Torquer conjugate-based drug of the present invention to target specific neurons in the CNS, it is possible to study and manipulate neural circuit loops, thereby advancing our understanding of brain function, addressing diseases or neurological disorders, and facilitating the development of therapeutic strategies for brain diseases and dysfunctions.

When the drug target protein is expressed on the neuronal cell membrane, the m-Torquer conjugate-based drug of the present invention, upon binding to the drug target protein, can modulate neural circuits and behavior in freely moving animals within the space of the rotating magnetic field.

The m-Torquer conjugate-based drug of the present invention can target drug target proteins that are naturally or artificially expressed on postsynaptic neurons and, through the application of a controlled rotating magnetic field in a specific pattern, can magnetically stimulate the drug target protein, thereby inducing specific action potential firing patterns in postsynaptic neurons according to a desired design.

Additionally, the m-Torquer conjugate-based drug of the present invention can selectively stimulate specific neurons in particular brain regions, thereby modulating neural circuits that transmit chemical signals to other brain regions. Moreover, this approach can optionally be used to induce or suppress behaviors associated with the targeted neural circuits.

Furthermore, by applying a controlled rotating magnetic field in a specific pattern, the rotational force of the magnetic nanoparticles generated under this condition, combined with repeated magnetic stimulation over an extended period of at least two days, can induce the formation of neural circuits and train animal behavior.

Non-limiting examples of generated or modulated neural circuits include memory circuits, emotional circuits, and reward circuits associated with dopamine secretion.

The behaviors regulated through the generated or controlled neural circuits include feeding behavior, social interactions, addiction, habitual behavior, and cognitive behavior.

Accordingly, the m-Torquer conjugate-based drug of the present invention can generate or regulate neural circuits, thereby inducing or controlling movements or behaviors (such as sequenced and coordinated movements) and can be used to prevent or treat various disorders, including cognitive impairments and metabolic diseases.

The present invention further provides that, when the drug target protein is expressed on the cell membrane of specific neurons in particular brain regions, the m-Torquer conjugate, upon binding to the drug target protein, allows the controlled rotating magnetic field to generate a specific magnetic stimulation pattern. This stimulus selectively activates or inhibits targeted neurons, thereby modulating neurotransmitter release (e.g., glutamate, gamma-aminobutyric acid (GABA), dopamine, etc.), and controlling both the secretion and levels of neurotransmitters that mediate signal transmission from one brain region to another.

In neuroscience, human behavior is generally understood to be driven by two primary motivations: one is necessities-essential for survival-such as water, food, sleep, and the avoidance of pain, and the other is rewards (Deborah Halber, 2018).

The brain contains a reward system, which consists of a set of interconnected brain structures that are activated by stimuli associated with reward or reinforcement. A central component of this system is the reward pathway.

The reward pathway functions like a highway for dopamine, a neurotransmitter that conveys information about reward stimuli. Dopamine is released from the reward centers located in the primitive brain and primarily transmits reward signals via two key pathways: the mesolimbic reward pathway (midbrain-limbic circuit) and the mesocortical reward pathway (midbrain-cortical circuit). The specific circuitry and levels of dopamine release determine the impact on behavior. In general, dopamine is most commonly associated with pleasure and reward, which is primarily linked to the function of the mesolimbic reward pathway.

The fundamental principles governing dopamine reward circuit function can be summarized as follows:

① A novel experience stimulates specific neurons in the brain, transmitting chemical signals to other brain regions.
② Repeated experiences establish multiple dopamine pathways, which become associated with past memories.
③ These interconnected pathways create a strong link between ongoing activities and past experiences, driving behavioral motivation.
④ Pleasurable or highly rewarding experiences tend to be repeated, whereas negative or aversive experiences are avoided.

The key brain regions that constitute the reward system communicate using the neurotransmitter dopamine. When the brain is exposed to rewarding stimuli, such as consuming delicious food, experiencing affectionate touch, engaging in pleasurable social interactions, eating chocolate, gambling, using social media, or online shopping, the reward circuit is activated, inducing a pleasurable sensation (euphoria). Remarkably, even merely imagining such enjoyable experiences can trigger dopamine release. When the reward circuit is activated, the brain interprets it as a signal of importance, stores it in memory, and craves repetition. In other words, repeated bursts of dopamine strengthen neural circuits, reinforcing the desire to repeat previously pleasurable behaviors. This dopaminergic reinforcement is the fundamental mechanism underlying addiction and motivational learning.

Higher dopamine levels are associated with increased interest in novelty and greater motivation. In controlled studies, artificial modulation of dopamine levels in experimental rodents showed that when dopamine levels were high, the animals opted for high-effort, high-reward tasks, whereas low dopamine levels led them to choose low-effort, low-reward tasks. This suggests a strong correlation between dopamine signaling and motivation intensity.

Beyond its role in pleasure and reward, dopamine is essential for attention, memory, and executive function. Moderate increases in dopamine levels are associated with enhanced satisfaction, creativity, motivation, curiosity, and perseverance.

While dopamine release generally elevates mood, improves concentration, and boosts motivation, excessive or insufficient dopamine levels can lead to various health issues. Additionally, habitual use of dopamine-enhancing substances (e.g., narcotics) or frequent engagement in highly stimulating activities (e.g., gaming) can lead to addiction. Chronic exposure to intense stimuli may result in excessive dopamine release, potentially rewiring the brain to become dependent on such stimuli while simultaneously losing interest in all other experiences.

The functioning of the brain arises from the interconnections among the spinal cord, brainstem, cerebellum, hypothalamus, thalamus, hippocampus, amygdala, cingulate cortex, and cerebral cortex. The hippocampus is involved in temporary memory storage, while the cerebral cortex is responsible for long-term memory storage. The thalamus functions as a sensory relay center, plays a role in neural oscillation synchronization, and generates sleep spindles. The brainstem acts as a switch regulating sleep-wake states. The cerebral cortex is highly interconnected via long-range neural fibers, and its primary function is sensory input processing, which is fundamental to perception formation.

At the center of the cerebrum lies the ventricular system, which is formed during development as cells in the ventricular zone proliferate to shape the central nervous system. The brain is enveloped by the pia mater, and the central nervous system is further protected by the pia mater, arachnoid mater, and dura mater, with cerebrospinal fluid (CSF) circulating in the subarachnoid space.

Although the human brain weighs approximately 1,400 g, it is suspended in cerebrospinal fluid, which provides buoyancy, thereby reducing its effective weight and serving as a shock absorber.

The choroid plexus, a network of capillaries intertwined within the ventricles, produces approximately 500 mL of cerebrospinal fluid per day. CSF flows sequentially through the lateral ventricles, third ventricle, cerebral aqueduct, and fourth ventricle.

At the cellular level, key neural structures include Purkinje cells, granule cells, mossy fibers, climbing fibers, and parallel fibers.

The human brain consists of approximately 86 billion to 100 billion neurons, interconnected through 100 trillion synapses, forming an extensive neural network. Neural communication within the brain occurs through neurons and synapses. When the presynaptic neuron releases neurotransmitters, the postsynaptic neuron detects these signals through receptor-mediated transmission.

A neuron communicates at synapses or junctions with other neurons or target cells, such as muscles, to either initiate or inhibit cellular activity.

The cell membrane is a fluid, dynamic structure, allowing continuous morphological changes. Notably, the plasma membrane of neurons extends into numerous projections and branches.

Each neuron forms approximately 10,000 synapses. The number of synapses can increase in response to sensory stimulation and decrease during sleep. The neuronal protrusions that form synapses are referred to as spines. A single neuron can extend 10,000 spines, which interact with adjacent neurons through voltage pulses and neurotransmitter signaling, thereby facilitating neural communication. Neurons are specialized cells capable of converting electrochemical signals into neurotransmitter-mediated action potentials. Neurons generate voltage pulses and transmit them to other neurons, forming neural circuits.

When neurons receive stimulation and establish functional connections with other cells, they receive an adequate supply of oxygen and glucose, which is essential for their survival. If neurons fail to form such connections, they cannot sustain life. Neural circuits emerge from neuronal interconnections, and fundamental brain functions such as memory and language arise from the operation of these neural networks.

The central nervous system (CNS) comprises nuclei, which are clusters of neurons, and axon bundles, which serve as pathways formed by very long axons extending from the nerve cell's plasma membrane for neural signal transmission. Axon bundles function as communication highways, relaying voltage pulses across neural circuits.

Dendrites act as the antennas of neurons, receiving synaptic inputs. Their spine structures undergo dynamic modulation depending on the type and intensity of synaptic activity.

Synapses can be either chemical or electrical.

The majority of synapses are chemical. This means that electrical stimulation or action potentials trigger the release of chemical messengers, known as neurotransmitters. The presynaptic neuron refers to the neuron that sends the signal, while the postsynaptic neuron is the one that receives it.

The presynaptic neuron generates action potentials, which propagate along its axon. At the axon terminal, vesicles filled with neurotransmitters are present. When the action potential reaches the axon terminal membrane, it activates voltage-gated calcium ion channels, allowing $Ca^{2+}$ influx due to its higher extracellular concentration. This $Ca^{2+}$ influx triggers vesicle fusion with the terminal membrane, leading to neurotransmitter release into the synaptic cleft.

Accordingly, the m-Torquer conjugate-based drug of the present invention is designed to target voltage-gated calcium ion channels expressed in presynaptic neurons. By applying a controlled rotational magnetic field, the m-Torquer conjugate can magnetically stimulate these channels, thereby regulating neurotransmitter release from the presynaptic neuron to the postsynaptic neuron or target cell. This allows for precise modulation of neural activity, either activating or inhibiting the postsynaptic neuron or target cell as needed (see Fig. 18).

The synaptic cleft refers to the extracellular space between the presynaptic and postsynaptic cells. Once released from the presynaptic neuron, neurotransmitters rapidly diffuse across the synaptic cleft and bind to their respective receptors on the postsynaptic neuron. This ligand-receptor interaction triggers chemical changes, such as the opening or closing of ion channels in the postsynaptic membrane, leading to alterations in membrane potential. Consequently, this process either increases or decreases the likelihood of an action potential firing in the postsynaptic neuron.

To terminate neurotransmission, synaptic neurotransmitters are degraded by enzymes, reabsorbed by the presynaptic neuron, diffused away from the synaptic cleft, or cleared by glial cells (astrocytes and other supporting cells in the nervous system).

The type of neurotransmitter released and its interaction with target neurons play a crucial role in determining its physiological function in the nervous system.

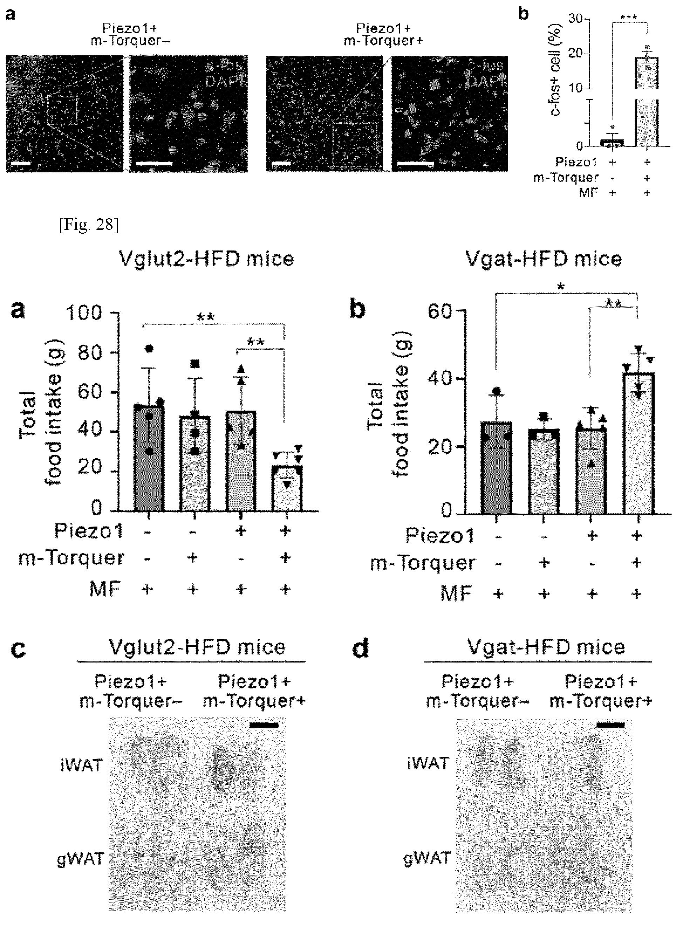

As illustrated in Figs. 28a and 28b, the m-Torquer conjugate-based drug of the present invention is designed to bind to drug target protein (neurotransmitter receptors) that are either naturally or artificially expressed in postsynaptic neurons. By applying a controlled rotational magnetic field, the m-Torquer conjugate induces precise conformational changes in the neurotransmitter receptor via the torque generated by the magnetic nanoparticles.

Furthermore, the efficacy of the m-Torquer conjugate-based drug, which is used in place of a ligand, can be regulated by applying a controlled rotational magnetic field in a specific pattern to stimulate the target protein. As a result, there is no need for ligand (e.g., neurotransmitter) concentration modulation or removal.

Electrical synapses exist in both invertebrate and vertebrate nervous systems. Unlike chemical synapses, which have a synaptic cleft approximately 20-50 nm wide, electrical synapses have narrower gap junctions (1-2 nm in diameter) that enable the direct transmission of ions between neurons, allowing for rapid signal propagation. However, unlike chemical synapses, electrical synapses cannot amplify or transform presynaptic signals. Electrical synapses play a crucial role in neuronal synchronization, making them particularly suited for transmitting rapid, invariant signals, such as those required for escape reflexes in squid.

The m-Torquer conjugate-based drug of the present invention is designed to bind to ion channels that are naturally or artificially expressed in postsynaptic cells of electrical synapses. Upon the application of a controlled rotational magnetic field, the resulting magnetic torque generated by the magnetic nanoparticles can precisely modulate the conformational changes of ion channels.

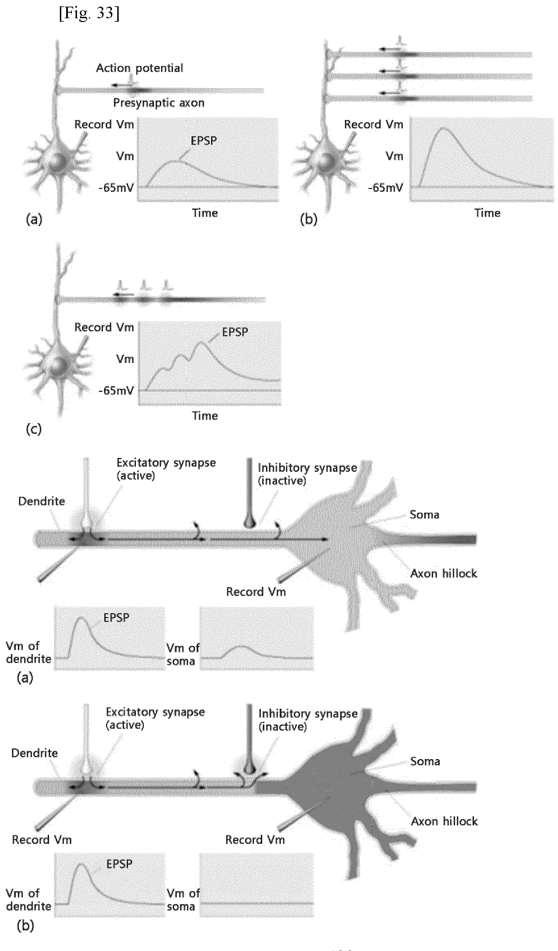

Neurons communicate with multiple other neurons, and the integration of multiple stimuli received by a postsynaptic cell determines the firing pattern of action potentials in that cell (Fig. 33).

The m-Torquer conjugate-based drug of the present invention is designed to target ion transport membrane proteins, either naturally or artificially expressed. By applying a controlled rotational magnetic field, the activation or inhibition of ion transport membrane proteins can modulate the electrical polarization of the cell membrane.

Furthermore, the m-Torquer conjugate-based drug can target excitatory and/or inhibitory neurotransmitter receptors that are naturally or artificially expressed in postsynaptic neurons. Through precise modulation of these receptors via a controlled rotational magnetic field, the drug can activate or inhibit excitatory and/or inhibitory neurotransmitter receptors.

Thus, by appropriately specifying the drug target protein expressed naturally or artificially in postsynaptic cells, the m-Torquer conjugate-based drug and the application of a controlled rotational magnetic field in a specific pattern can precisely stimulate the target protein, thereby enabling the precise control of postsynaptic neuronal firing patterns as desired (Fig. 17).

Brain function arises from the complex interactions between neurons. Brain waves emerge from the synchronized signaling of large populations of neurons and serve as a critical indicator of brain activity and functional state.

The brain generates and propagates electrical signals through neuronal interactions, and these electrical activities can be measured as brain waves. The formation and modulation of brain waves are primarily influenced by the overlapping network of excitatory and inhibitory neurons, where excitatory neurons respond to stimuli, while inhibitory neurons regulate cognitive processes. Analyzing brain waves provides valuable insights into brain function and state.

Meanwhile, the body contains numerous peripheral nerves, among which the vagus nerve plays the most significant role in regulating metabolism within the autonomic nervous system. The vagus nerve establishes distinct connections with individual organs, and organ-specific control is mediated by spike-patterned action potential activity. The pattern of action potentials determines the release of neurotransmitters, thereby influencing intracellular signaling cascades and modulating the activity of target cells or neighboring cells.

The m-Torquer conjugate-based drug of the present invention is designed to target transmitter-gated ion channels that are naturally or artificially expressed in postsynaptic cells. By applying a controlled rotational magnetic field, the drug can stimulate transmitter-gated ion channels, thereby regulating the opening and closing of these ion channels in postsynaptic neurons, effectively substituting for neurotransmitter release from presynaptic neurons (Figs. 28a and 28b).

**[Neurotransmitters and Their Receptors]**

A neurotransmitter is a chemical messenger secreted by a neuron to affect another cell across a synapse.

Depending on the type of neurotransmitter secreted by the presynaptic neuron, neurons can be classified as follows:

Cholinergic neurons - Acetylcholine
GABAergic neurons - Gamma-aminobutyric acid (GABA)
Glutamatergic neurons - Glutamate
Dopaminergic neurons - Dopamine

For example, the m-Torquer conjugate-based drug of the present invention can selectively activate and/or inhibit glutamatergic neurons, GABAergic neurons, cholinergic neurons, and/or dopaminergic neurons through the application of a controlled rotational magnetic field in a specific pattern (Example 2).

In response to the neurotransmitter secreted by the presynaptic neuron, cell-surface receptor proteins expressed on the postsynaptic cell include:

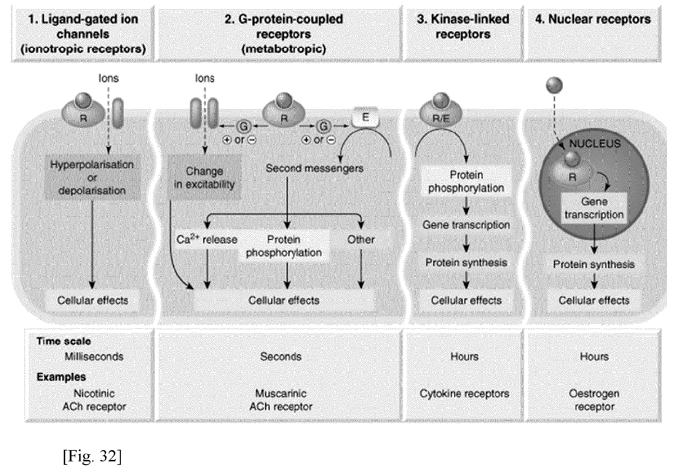

(1) Ion-channel-linked receptors (Transmitter-gated ion channels) (e.g., acetylcholine receptor, glutamate receptors, $GABA_A$ receptor)
(2) G-protein-linked receptors (e.g., $GABA_B$ receptor, muscarinic acetylcholine receptor (mACh), metabotropic glutamate receptor)
(3) Enzyme-linked receptors (Fig. 31).

Neurotransmitters regulate cognition, memory, learning, behaviors, emotion, mood, movement, pain, mental disorders, heart rate, hormone secretion, and respiration.

Glutamatergic neurons produce glutamate, the principal excitatory neurotransmitter in the mammalian central nervous system (CNS). Glutamate plays a critical role in fundamental brain functions, including cognition, learning, memory, sensation, and perception.

As the most abundant excitatory amino acid neurotransmitter in the CNS, the proper timing and location of glutamate release are crucial. Prolonged neuronal stimulation by excitatory amino acids can lead to neuronal death or injury, and excessive excitatory effects may contribute to Alzheimer's disease.

Accordingly, the m-Torquer conjugate-based drug of the present invention can be designed to target excitatory neurotransmitter receptors naturally or artificially expressed in postsynaptic neurons and inhibit the stimulation of excitatory neurotransmitter receptors through the application of a controlled rotational magnetic field in a specific pattern, thereby preventing neuronal death or injury in postsynaptic neurons.

The classification of a synapse as excitatory or inhibitory depends on the type of receptor present on the postsynaptic membrane.

An excitatory postsynaptic potential (EPSP) occurs when ion channels open, allowing cations to flow into the postsynaptic neuron, leading to a transient depolarization of the postsynaptic membrane potential. The primary function of neurotransmitters is to open ion channels in the postsynaptic neuron's membrane. The characteristics of the resulting membrane potential change depend on the specific ion channels that open. A postsynaptic potential may be either an EPSP or an inhibitory postsynaptic potential (IPSP), depending on the nature of the ion channel modulation. For instance, neurotransmitters that decrease $K^+$ conductance or open $Na^+$ channels typically generate an EPSP. The number of ions exchanged across the membrane is influenced by the number of ion channels opened by a given neurotransmitter, the duration for which these channels remain open, and the availability of neurotransmitter molecules in the synaptic cleft. Additionally, factors such as the level of depolarization in the presynaptic neuron and the amount of $Ca^{2+}$ influx at the axon terminal impact the available quantity of neurotransmitter molecules. The action of a neurotransmitter ceases when it is removed from the synaptic receptor site and the synaptic cleft. This removal occurs through either enzymatic degradation or reuptake into the presynaptic axon terminal. In most synapses, both mechanisms occur simultaneously, thereby limiting the duration of postsynaptic potentials and preventing interference among the numerous neural circuits connected to the synapse. Furthermore, the reuptake mechanism allows the presynaptic neuron to recycle neurotransmitters, sustaining their activity.

A notable example of this process is acetylcholine (ACh). Upon its release into the synapse, ACh induces an EPSP while simultaneously undergoing enzymatic degradation by acetylcholinesterase (AChE). This degradation prevents the continuous firing of action potentials by breaking ACh down into its metabolites, which are then taken back up into the presynaptic neuron (sleeping neuron). However, if this process fails, it can lead to severe consequences-one of the most well-known examples being sarin gas.

An inhibitory postsynaptic potential (IPSP) is a postsynaptic potential that reduces the likelihood of an action potential occurring in the postsynaptic neuron or an alpha motor neuron.

A significant proportion of synapses are inhibitory rather than excitatory. An inhibitory synapse generates a hyperpolarizing postsynaptic potential, meaning that the action of a neurotransmitter opens ion channels that selectively allow the movement of $K^+$, $Cl^-$, or both. The voltage fluctuation recorded at synapses exhibiting such hyperpolarization is referred to as an inhibitory postsynaptic potential (IPSP). This potential modifies the membrane potential of the postsynaptic neuron, increasing the threshold required for action potential generation. For example, if an inhibitory neurotransmitter selectively opens $K^+$ channels, $K^+$ ions will flow out of the cell, leading to a decrease in the membrane potential to approximately -75 mV. This process continues until the $K^+$ channels close, thereby reducing the electrochemical gradient of $K^+$ ions. Such hyperpolarization effectively raises the threshold for action potential initiation. The relationship between the membrane potential induced by postsynaptic potentials and the threshold for action potential initiation is critical, with $Cl^-$ ions playing a key role in this process.

**[hypothalamus and neurohormones]**

The hypothalamus is located beneath the two thalami, positioned just above the brainstem. Anatomically, it is part of the diencephalon and is situated at the ventral center beneath both thalami. The hypothalamus is a structure found in the brains of all vertebrates, and in humans, it is approximately the size of an almond.

One of the primary functions of the hypothalamus is to bridge the nervous system and the endocrine system via the pituitary gland.

The hypothalamus regulates metabolic processes and controls the autonomic nervous system (ANS). Additionally, it synthesizes and secretes neurohormones, which, in turn, stimulate or inhibit the release of pituitary hormones. As a result, the hypothalamus plays a crucial role in regulating body temperature, hunger, thirst, fatigue, sleep, and circadian rhythms.

Neurohormones are a specialized class of hormones produced by neurons (nerve cells) and released into the bloodstream or extracellular fluid. Unlike conventional hormones, which are typically produced by endocrine glands, neurohormones are synthesized within specialized neurons and are released in response to specific neural signals.

The term "neurohormone" encompasses a broad range of signaling molecules that exhibit hormone-like functions and are involved in the regulation of various physiological processes. Similar to classical endocrine hormones, neurohormones can act on distant target cells or organs, eliciting specific physiological responses. Neurohormones are generally classified into two major types: releasing hormones and inhibiting hormones.

Releasing hormones are neurohormones that stimulate the secretion of other hormones from endocrine glands. For instance, the hypothalamus produces and releases various releasing hormones that regulate hormone secretion from the pituitary gland. These releasing hormones travel through the bloodstream to the pituitary gland, where they stimulate the release of specific hormones such as growth hormone (GH), thyroid-stimulating hormone (TSH), or adrenocorticotropic hormone (ACTH).

Inhibiting hormones, on the other hand, function to suppress the secretion of specific hormones. The hypothalamus also produces inhibiting hormones that regulate hormone secretion from the pituitary gland. These hormones act on the pituitary to reduce the secretion of specific hormones, thereby providing negative feedback regulation.

Neurohormones can also play a crucial role in modulating behavior, emotions, and other aspects of neural function. For example, the neuropeptide oxytocin functions as a neurohormone, released from specific neurons in the hypothalamus. Oxytocin is involved in social bonding, maternal behavior, and the regulation of reproductive functions.

The release and regulation of neurohormones are tightly controlled and can be influenced by neural signals, feedback mechanisms, and external stimuli. Neurohormones often exhibit pulsatile or rhythmic patterns of secretion, allowing for precise and coordinated responses in the body.

As essential signaling molecules, neurohormones bridge the gap between the nervous and endocrine systems. They play a critical role in regulating physiological processes, maintaining homeostasis, and coordinating various bodily functions.

The lateral hypothalamus (LH) is a region of the hypothalamus, which is centrally located in the brain and plays a critical role in regulating various physiological functions and maintaining homeostasis.

The lateral hypothalamus is primarily involved in the regulation of appetite and energy balance. Activation of this region induces hunger and the drive to eat, promoting food intake and meal consumption. Conversely, damage to this area can lead to reduced appetite, weight loss, and anorexia-related symptoms.

The lateral hypothalamus (LH), also referred to as the lateral hypothalamic area (LHA), contains primary orexinergic nuclei, which are widely projected throughout the nervous system. This neural network mediates various cognitive and physiological processes, including feeding behavior, arousal promotion, pain perception reduction, thermoregulation, digestive function, and blood pressure control. Clinically significant disorders associated with dysfunctions of the orexinergic projection system include narcolepsy, movement disorders, functional gastrointestinal disorders related to visceral hypersensitivity (e.g., irritable bowel syndrome), and feeding disorders. The key neurotransmitters and neuromodulators involved in the activity of orexin neurons include glutamate, endocannabinoids (e.g., anandamide), and the orexin neuropeptides-orexin A and orexin B. Pathway-specific neurochemical mediators include GABA, melanin-concentrating hormone (MCH), nociceptin, glucose, dynorphin peptides, and the appetite-regulating peptide hormones leptin and ghrelin. Notably, cannabinoid receptor 1 (CB1) is co-localized with orexinergic projection neurons in the lateral hypothalamus and many output structures, indicating that CB1 and orexin receptor 1 (OX1) are co-expressed. The body forms CB1-OX1 receptor heterodimers.

**[Cell-Specific Gene Delivery Technology for Drug Target Proteins]**

One embodiment of the present invention provides a kit comprising (i) the m-Torquer conjugate-based drug described above and (ii) a carrier capable of delivering the gene encoding a drug target protein, which can bind to the m-Torquer conjugate, to a specific population of cells within a defined region in vivo.

In this embodiment, the carrier enables the expression of the drug target protein in the biological lipid membrane of specific cells within a targeted region in vivo. When the m-Torquer conjugate-based drug is administered, the m-Torquer conjugate binds to the drug target protein, and upon the application of a controlled rotating magnetic field, the mechanical torque generated by the magnetic nanostructure induces conformational changes in the drug target protein, thereby locally delivering a magnetic stimulus to the targeted protein.

The targeted region for gene expression facilitated by the carrier may include the brain, particularly the lateral hypothalamus (LH), and the targeted cells may include both general cell types and neurons.

Nanomagnetic magnetogenetics, which utilizes genetically encoded mechanosensitive ion channels activated by magnetic nanotransducers, enables the wireless and remote activation of neurons in freely moving animals with rapid precision. However, magnetogenetics has yet to achieve cell-type-specific expression of magnetogenetic molecules, which is critical for precise temporal control of targeted neural activity.

Magnetogenetics-based cell-specific gene delivery technology enables selective activation and inhibition of various neuronal subtypes across different brain regions, facilitating an understanding of how specific cell types contribute to brain circuit function.

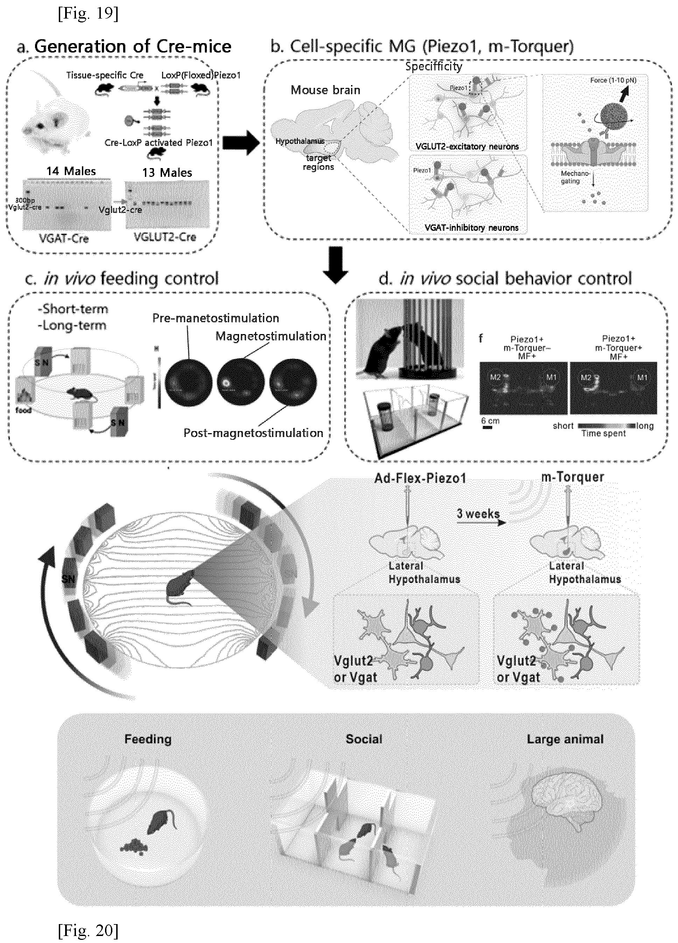

In light of this, Example 2 presents, for the first time, a method to magneto-mechanically stimulate distinct populations of excitatory or inhibitory neurons within deep brain regions in vivo using the m-Torquer conjugate-based drug of the present invention, by integrating Cre-dependent expression of Piezo1 with cell-type-specific control of neuronal activity (Figs. 18 and 19). Using the m-Torquer conjugate-based drug, glutamatergic and GABAergic neurons in the lateral hypothalamus (LH) were selectively activated, successfully facilitating or suppressing feeding behavior in freely moving animals, thereby validating the role of LH neural circuits in feeding regulation. Furthermore, prolonged and repeated activation of Vglut2 neurons in the LH using the m-Torquer conjugate-based drug resulted in sustained anorexigenic effects, significant weight loss, and metabolic alterations in obese mice. Thus, the m-Torquer conjugate-based drug of the present invention introduces a novel approach and pharmacological modality for long-term, noninvasive control of feeding-specific neural circuits for obesity treatment.

Additionally, in Example 2, the inventors demonstrated for the first time that freely moving animals' neurons could be rapidly and remotely activated wirelessly using nano-magnetogenetics-an approach built upon the m-Torquer conjugate-based drug as a magnetic nanotransducer for the mechanical activation of genetically encoded mechanoresponsive ion channels. Therefore, the m-Torquer conjugate-based drug is applicable to a wide range of neuroscientific studies utilizing magnetogenetics, particularly for selective interrogation of brain cell populations and neural circuits, tethered optogenetic control, investigating complex behavioral paradigms that may be disrupted by less naturalistic environments, and studying neurological disorders requiring long-term stimulation and response monitoring.

Furthermore, using the m-Torquer conjugate-based drug of the present invention, it was confirmed for the first time that magnetogenetics can be tailored for diverse neuroscience research, particularly for the selective interrogation of the LH-VTA circuit in social interactions within less restrictive settings. Therefore, remote and minimally invasive animal behavior control technology utilizing the m-Torquer conjugate-based drug establishes a crucial tool for nanotechnology-based magnetogenetic neuromodulation and provides a promising therapeutic approach for obesity by regulating feeding behavior and body weight.

As used herein, a cell-specific gene refers to a gene that includes a promoter enabling spatially regulated expression in a target cell. The term cell is not limited to a specific cell type.

Representative examples include the Cre-LoxP and Flp-FRT systems, but are not limited thereto.

Additionally, for temporal regulation, the Cre-LoxP system may be coupled with an inducible system that can be activated at a specific time. The induction mechanism may be physical, chemical, or biological. Representative examples include the tetracycline-inducible system, but are not limited thereto.

The m-Torquer conjugate-based drug of the present invention may be used in combination with a viral vector designed to introduce the genetic information of a drug target protein (e.g., an ion transport membrane protein) into a target cell, thereby enabling the expression of the drug target protein.

In this case, for example, an ion transport membrane protein, expressed in a target cell at a desired time, may be activated using the rotational force of magnetic nanoparticles generated upon application of a controlled rotating magnetic field, thereby altering the electrical polarization of the cell membrane.

To enable the m-Torquer conjugate-based drug to bind to the drug target protein (e.g., an ion channel), the drug target protein may be genetically engineered to include a protein tag on its extracellularly exposed surface, allowing its expression in the target cell.

A protein tag may be co-expressed with the drug target protein by cloning the tag along with the target gene.

The protein tag functions as a specific binding site for the binding moiety (b) of the m-Torquer conjugate-based drug, thereby facilitating the connection between the m-Torquer conjugate-based drug and the drug target protein. The protein tag is not particularly limited, as long as it interacts with the binding moiety (b) of the m-Torquer conjugate-based drug, but preferred examples include MYC-tag, HA-tag, FLAG-tag, His-tag, and GST-tag. According to a preferred embodiment of the present invention, the binding moiety (b) of the m-Torquer conjugate-based drug and the protein tag may be linked via a streptavidin-biotin interaction.

For example, an ion channel may be expressed on the cell surface through the introduction of a recombinant genetic sequence. According to one embodiment of the present invention, the genetic sequence of the ion channel to be expressed may be recombined by inserting the genetic sequence of a protein tag, followed by the introduction of this recombinant gene into the cell. In the introduced cell, the recombinant gene enables the formation of an ion channel conjugated with a protein tag on the cell membrane.

Vectors used for the intracellular delivery of the recombinant gene may include various types of vectors and viral vectors, such as plasmids and recombinant viruses, including but not limited to recombinant adeno-associated virus (rAAV), recombinant retrovirus, recombinant lentivirus, recombinant adenovirus, recombinant herpes simplex virus, and other viruses known in the art.

The expression of the recombinant gene encoding the ion transport membrane protein in Example 1 is driven by constitutive promoters, such as the CMV promoter, Ef1-alpha promoter, or LTR. In another embodiment, the promoter may be an inducible or cell-type-specific promoter. A cell-type-specific promoter that enables the expression of the recombinant ion transport membrane protein in specific subpopulations of cells, particularly in neurons, may be preferred. These cells may include neurons within the nervous system, but are not limited thereto. Cell-type-specific promoters are well known in the art. Particularly preferred cell-type-specific promoters include hSyn, vGat, vGlut, and TH, but are not limited to these. Methods for the intracellular delivery of the recombinant genetic sequence may employ conventional techniques. However, it is preferable to introduce the gene into the cell using adeno-associated virus (AAV) or lentivirus.

In the recombinant genetic sequence of the present invention, the position of the genetic sequence corresponding to the protein tag may be adjusted so that the tag protein is expressed on the surface of the drug target protein protruding outside the cell.

Meanwhile, the success of gene therapy depends on the safe delivery of the therapeutic gene to the target cells. One of the most promising vectors for gene therapy is the adeno-associated virus (AAV).

A gene therapy drug consists of a "therapeutic gene" for disease treatment and a "vector" for gene delivery. Vectors are classified into "non-viral vectors", such as plasmids and liposomes, and "viral vectors", such as adeno-associated virus (AAV), adenovirus, retrovirus, and lentivirus. When genetic material is directly delivered in vivo, viral vectors exhibit significantly superior efficiency.

Wild-type AAV, which exists in nature, encapsulates DNA necessary for viral replication within a protein complex called the capsid. This DNA contains genetic information essential for genome replication and the biosynthesis of capsid proteins that enclose the viral genome.

In pharmaceutical applications, recombinant AAV vectors are used. A recombinant AAV vector replaces the original viral DNA genome with a therapeutic gene (drug target protein) intended for disease treatment. The capsid protein of the recombinant AAV binds to the cell membrane receptors of the target tissue and facilitates the efficient intracellular delivery of the therapeutic gene via the endocytosis pathway. Once inside the cell, the therapeutic gene is transported to the nucleus, where it enables the expression of the drug target protein, ultimately leading to disease treatment.

AAV is a historically validated non-pathogenic virus, as no diseases have been reported despite 30-40% of the global population having been exposed to AAV infections. One of its most significant advantages is its low immunogenicity.

The human body typically mounts an immune response to eliminate non-self molecules introduced from external sources. However, in the case of recombinant AAV used as a pharmaceutical agent, the original viral genome has been almost entirely replaced with a therapeutic gene, thereby minimizing immune response-related adverse effects.

Moreover, therapeutic genes delivered into target cells via recombinant AAV remain as an episomal structure, independent of the host cell genome. Consequently, the risk of genotoxicity associated with genomic DNA integration is significantly reduced. Additionally, because AAV cannot replicate or propagate independently without a helper virus, there is no risk of viral amplification in vivo following administration, further eliminating concerns about virus-induced adverse effects.

## [Magnetic-Field-Based Stimulation via Magnetic Torquer (m-Torquer) Conjugate-Based Drugs]

Magnetic-field-based stimulation holds significant potential for providing rapid, tetherless, and implant-free deep brain stimulation. Compared to optical, electrical, or other stimulation methods, magnetic fields do not suffer from attenuation or low focal capacity when penetrating brain tissue. Magnetic control enables less invasive targeting of deeper brain regions and specific neuronal populations.

Furthermore, magnetogenetics, an approach that integrates magnetic fields with magnetic actuators, presents a novel tool for controlling biological functions.

Accordingly, the inventors have developed the m-Torquer system, which includes the m-Torquer conjugate-based drug developed in the present invention, as a new magnetic toolset for tetherless neuromodulation via magnetically induced torque-driven mechanotransduction. The m-Torquer system of the present invention utilizes Piezo1, a genetically encoded mechanosensitive ion channel, in a magnetogenetic approach, demonstrating that rotating magnetic field-based physical stimulation (rotating CMA) can be precisely delivered to the target region in a non-contact manner with spatial accuracy. Moreover, despite the long working distance, the m-Torquer system provides the first evidence that deep brain neuromodulation can be achieved in freely moving animals to modulate behavior by controlling neural circuits.

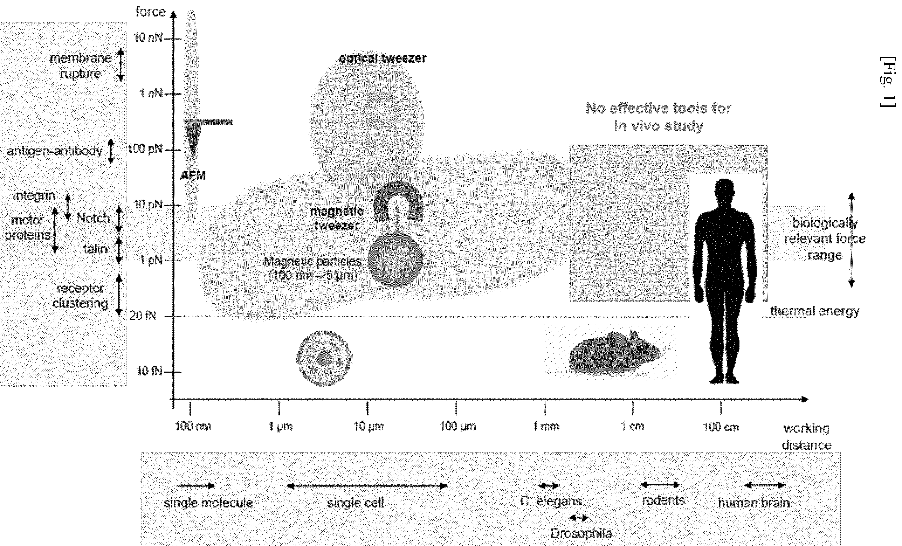

In the m-Torquer conjugate-based drug of the present invention, when the m-Torquer conjugate binds to the drug target protein, the rotational force of magnetic nanoparticles can be precisely tuned via controlled application of a rotational magnetic field. This force is sufficiently small to avoid tissue damage, yet strong enough to activate mechanically responsive drug target proteins (Fig. 1).

To achieve this, the present invention enables the transmission of the rotational force of magnetic nanoparticles to the drug target protein by:

(1) Structurally designing the binding moiety (b) and linker (c) in the m-Torquer conjugate to functionally integrate with the drug target protein; and/or

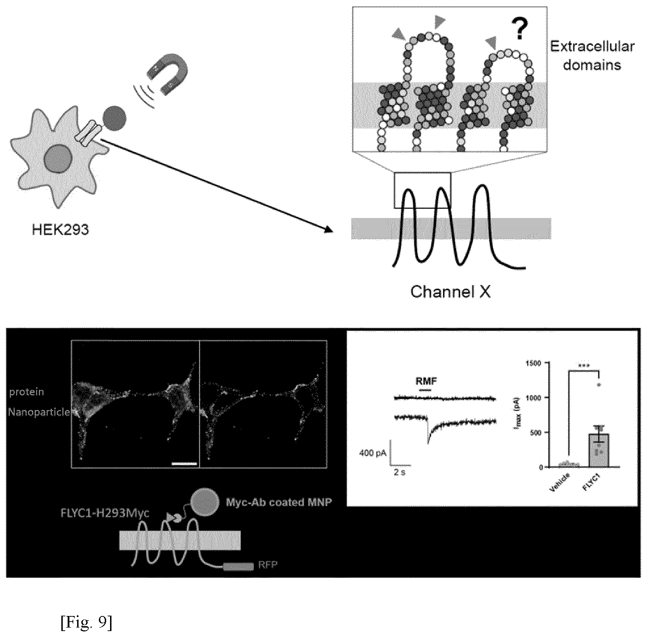

(2) Optimizing the binding position of the m-Torquer conjugate on the drug target protein, such as a specific site on the extracellular loop domain of an ion transport membrane protein, through structural design of the binding moiety and/or amino acid modifications at the binding site of the drug target protein (Fig. 8).

Additionally, the m-Torquer conjugate-based drug of the present invention enables wireless and remote-controlled modulation of drug target protein activity with precise temporal accuracy and resolution via the m-Torquer conjugate. In addition to enabling sub-second, high-resolution temporal control over stimulation, the m-Torquer conjugate allows for long-term magnetic stimulation lasting more than 10 days.

To regulate the efficacy of the m-Torquer conjugate-based drug, a controlled rotational magnetic field can be applied in a specific pattern to stimulate the drug target protein. When necessary, to apply various controlled rotational magnetic field patterns, a uniform magnetic field can be used to maintain a constant field strength, and/or a gradient magnetic field can be applied to modulate the field intensity accordingly.

## [Regulation of Anion Transport Membrane Protein Gating and Neuronal Inhibition via Magnetic Torque-Induced Rotation of Magnetic Particles (Example 1)]

Various techniques for inhibiting neural activity have been previously explored. The suppression of neural activity through pharmacological agents, electrical stimulation, or magnetic field-based stimulation alone has been extensively studied and is currently employed as a therapeutic approach. However, these three conventional methods share a critical limitation-they lack cellular specificity in targeting the desired neuronal population.

In contrast, by integrating the m-Torquer conjugate-based drug of the present invention with a magnetogenetic approach, we have demonstrated that targeted inhibition of specific neurons can be achieved via the expression of anion transport membrane proteins in designated neuronal populations (Example 1).

Silencing neuronal activity is also a crucial tool in neuronal computation for investigating the causal relationships of specific neurons. In studies involving large-scale neural networks or intact brains, temporary silencing can yield significantly more informative insights than mere excitation. Achieving non-invasive neuronal silencing enables unprecedented in vivo experimental paradigms in basic neuroscience while also expanding the clinical applicability of precision neuromodulation. Thus, the discovery and application of mechanically-gated inhibitory channels has long been a central objective in magnetogenetics.

Neuronal activity can be modulated bidirectionally, either through activation or inhibition.

Ion transport membrane proteins are known to undergo mechanically gated opening and closing, depending on their intrinsic properties, and anion transport membrane proteins, in particular, play a key role in neuronal inhibition.

Ion transport membrane proteins include both channel proteins and pump proteins, but are not limited thereto. Specific examples include: chloride ion ($Cl^-$) transport membrane proteins, such as FLYC channels, CLC channels, E-CLC channels, CLIC channels, and GABA receptors; non-selective anion transport membrane proteins, such as MscS channels, MscL channels, SWELL channels, ANO channels, and Maxi anion channels; and other ion pumps involved in anion transport. The transported anions primarily include chloride ions ($Cl^-$), but are not limited to $Cl^-$ alone and may involve various combinations of anions.

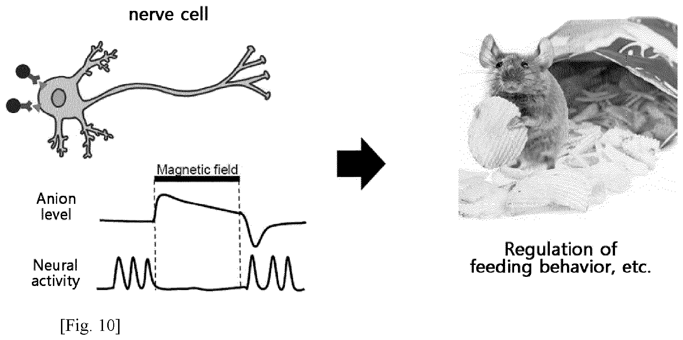

Considering the general ionic balance in the nervous system, the use of mechanosensitive potassium or anion channels can silence neural activity. Mechanosensitive K2P potassium channels have been extensively studied and have the capacity to induce hyperpolarization in neuronal cells. However, a major drawback of these channels is the occurrence of leak currents in response to various stimuli, and it remains challenging to maintain their sensitivity to mechanical stimulation while reducing their responsiveness to other stimuli. Anion channels also have the potential to hyperpolarize or shunt the membrane and have been successfully employed for silencing neuronal activity using light, chemicals, or temperature. However, the use of mechanosensitive anion channels for neuromodulation has not yet been achieved due to the lack of identified mechanosensitive anion channels. The present inventors have identified several MscS-like mechanosensitive channels that are selectively conductive to $Cl^-$ ions. These channel proteins share homology with the bacterial MscS protein, one of the most well-studied mechanosensitive ion channels. By leveraging the molecular, functional, and structural knowledge of MscS and MscS-like channels, it is possible to design mechanosensitive channels optimized for magnetic-mechanical manipulation. Thus, an ideal approach for magneto-mechanical silencing of neurons would be the combination of nanotechnology with genetically engineered mechanosensitive anion channels (Fig. 7 and Fig. 9).

Example 1 demonstrates a neural activity suppression technique that integrates the m-Torquer conjugate-based drug of the present invention and a rotating magnetic field generator into a magnetogenetic approach. Instead of cation-transporting membrane proteins, this example employs anion-transporting membrane proteins. As a result, it was confirmed that the m-Torquer conjugate-based drug of the present invention, using the magnetogenetic approach, enables the regulation of anion transport membrane proteins and thereby suppresses neural activity (Example 1).

In Example 1, the ion channel DmFLYC1 was used as the drug target protein, with MYC-tag serving as the protein tag. The binding moiety (b) of the m-Torquer conjugate-based drug was designed as a MYC antibody that binds specifically to the MYC-tag. Upon application of a rotating magnetic field, the magnetic nanoparticles within the administered m-Torquer conjugate-based drug rotate, exerting mechanical force on the DmFLYC1 ion channel expressed on the cell membrane. In response to this mechanical stimulus, the DmFLYC1 ion channel opens, allowing $Cl^-$ ions to influx into the cell, leading to hyperpolarization of the membrane potential. This result is consistent with the graph shown in Fig. 17. Thus, the m-Torquer conjugate-based drug of the present invention demonstrates the ability to regulate cellular activity through the rotational force of magnetic nanoparticles. When the target cells are neurons, this process enables the suppression of neuronal activity.

According to one embodiment of the present invention, the method for inducing intracellular anion influx into target cells using the m-Torquer conjugate-based drug and a magnetogenetic approach, or the method for regulating the opening and closing of anion transport membrane proteins and suppressing neural activity through the rotational force of magnetic nanoparticles, comprises:

(1) a step of designing an anion transport membrane protein that is capable of binding to magnetic nanoparticles and whose opening and closing can be controlled via a magnetogenetic approach;
(2) a step of producing an m-Torquer conjugate-based drug comprising a binding moiety that interacts with the anion transport membrane protein and magnetic nanoparticles that generate rotational force when subjected to a rotating magnetic field;
(3) a step of expressing the designed anion transport membrane protein in target cells and administering the prepared m-Torquer conjugate-based drug to allow for binding to the target protein.
(4) a step of applying a rotating magnetic field to the magnetic nanoparticles of the m-Torquer conjugate-based drug to deliver rotational force stimulation to the anion transport membrane protein expressed on the cell membrane, thereby activating the target protein.

The present invention further includes a step of suppressing neural activity by targeting neurons as the target cells, with applications both in vitro and in vivo.

In step (1), a nucleotide sequence encoding an antigenic polypeptide (e.g., a polypeptide corresponding to the arch-like binding region of the ion channel shown in Fig. 8) is synthesized within the extracellular loop domain of the anion transport membrane protein. This antigenic sequence mediates binding to the antibody moiety of the magnetic nanoparticles.

Additionally, in step (1), the anion transport membrane protein is assessed to determine whether it is functionally responsive to the rotational force of the magnetic nanoparticles. If necessary, the nucleotide sequence is modified and synthesized to enhance its responsiveness to mechanical stimulation, ensuring that the protein can undergo functional regulation upon exposure to mechanical force (Fig. 8).

Step (2) is identical to the description of the m-Torquer conjugate-based drug provided throughout this specification, except that the drug target protein is specifically an anion transport membrane protein. Therefore, the detailed explanation is omitted.

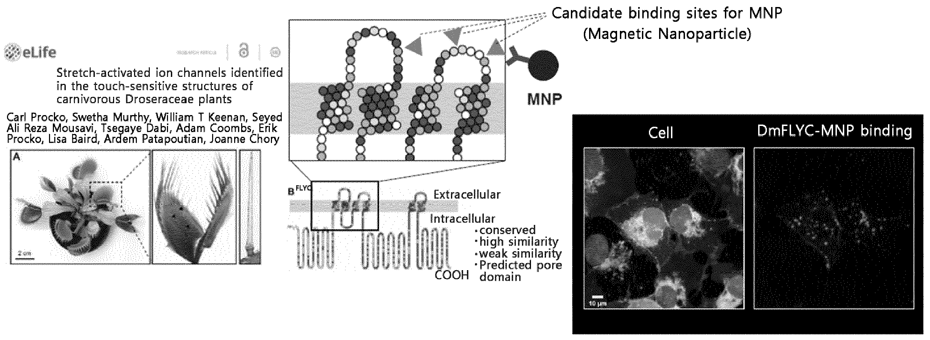

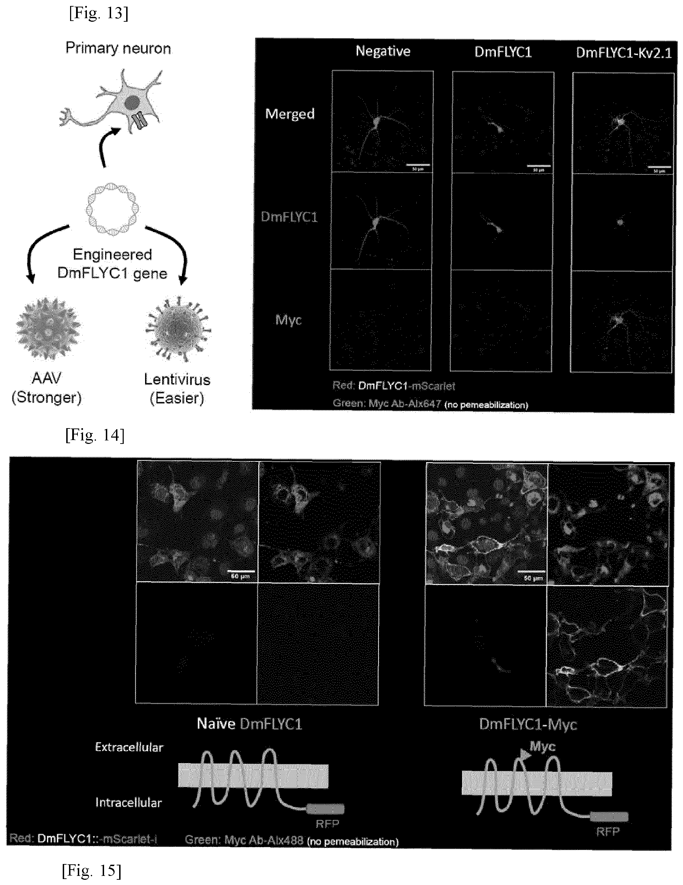

In step (3), the genetic information encoding the anion transport membrane protein is introduced into the target cells in a suitable form to enable its expression (Fig. 12). This step includes the use of viral vectors, which involves constructing a viral vector containing the gene for the anion transport membrane protein and injecting this virus into the target cells or animal tissues (Fig. 13).

Following the gene introduction, the anion transport membrane protein is expressed within the target cells. If a viral delivery system is used, gene expression typically requires a period of two to three weeks. Once the anion transport membrane protein is expressed in the target cells, the next step is to induce antigen-antibody binding between the anion transport membrane protein and the m-Torquer conjugate-based drug of the present invention. This step involves administering the m-Torquer conjugate-based drug into the environment where the target cells are present. The binding between the anion transport membrane protein and the m-Torquer conjugate-based drug occurs over a period ranging from one hour to one day.

In step (4), a rotating magnetic field generator is used to generate a rotating magnetic field in the environment where the target cells are present. This rotating magnetic field consists of a concentric, uniform magnetic field (ranging from 25 mT to 100 mT) that rotates in either a clockwise or counterclockwise direction. The rotating magnetic field induces the rotation of magnetic nanoparticles within the magnetic field region, generating rotational force. This rotational force is transmitted to the anion transport membrane protein to which the magnetic nanoparticles of the m-Torquer conjugate-based drug are bound, ultimately facilitating anion influx into the target cells and leading to the suppression of neural activity in the target neurons (Fig. 9).

Accordingly, the present invention enables the modulation of the electrical polarization of the cell membrane using the rotational force of magnetic nanoparticles, specifically inducing the suppression of neuronal activity. Importantly, the target cells are not limited to a specific cell type.

Furthermore, the present invention allows for the inhibition of neuronal activity for both fundamental scientific research and clinical applications. Specific exemplary applications are as follows, though the scope of the invention is not limited to these examples:

(1) Investigation and characterization of specific neuronal properties through the inhibition of neuronal activity.
(2) Study of neural circuit connectivity by suppressing neuronal activity.
(3) Identification and analysis of drug mechanisms of action on neurons via neuronal activity suppression.
(4) Discovery and research of behavioral mechanisms in animals through in vivo inhibition of neuronal activity.
(5) Suppression of excessive neuronal activity in epileptic brains as a therapeutic approach.
(6) Reduction of hyperactivity caused by excessive neuronal activity in ADHD patients.
(7) Suppression of excessive pain perception in chronic pain disorders.

The fundamental scientific applications outlined in points 1-4 have been extensively studied using optogenetics and chemogenetics, which have become essential tools for understanding animal nervous systems.

As described in the comparison with prior technologies, magnetogenetic inhibition of neuronal activity offers unique advantages over conventional techniques. Therefore, the present invention is expected to have market potential and anticipated benefits in areas that could not be realized with existing technologies. In particular, the ability of the magnetic field generated from a device positioned tens of centimeters away to penetrate biological tissue freely and non-invasively reach target cells in vivo makes the present invention applicable to medium- and large-sized animals, including humans, thereby enhancing its market potential and applicability. The clinical applications outlined in points 5-7 are expected to be highly effective in establishing therapeutic methodologies using experimental animal models for disease treatment research. Moreover, the potential for clinical use in therapeutic applications suggests a significant market opportunity.

**[Pharmacological Mechanism of Action (MoA) of m-Torquer Conjugate-Based Drugs]**

The m-Torquer conjugate-based drug of the present invention can induce beneficial modifications in pathological processes through conformational changes in the drug target protein upon application of a rotating magnetic field. For instance, it can function as an agonist, antagonist, enhancer, inhibitor, or blocker for drug target proteins embedded in the biological lipid membrane.

Accordingly, the m-Torquer conjugate-based drug of the present invention, upon exposure to a patterned and controlled rotating magnetic field, can modulate the conformational state of the drug target protein to act as an agonist, antagonist, enhancer, inhibitor, or blocker of the drug target protein present in the biological lipid membrane, thereby preventing or treating diseases associated with the drug target protein.

The m-Torquer conjugate-based drug of the present invention targets drug target proteins that are naturally or artificially expressed into postsynaptic cells. By applying a controlled rotating magnetic field in a specific pattern, it can stimulate the drug target protein, thereby mimicking or inhibiting the biological effects induced when neurotransmitters released from presynaptic cells bind to their respective receptors (i.e., the drug target protein), leading to receptor activation or inhibition.

For example, if the drug target protein is an ion channel or a receptor, the m-Torquer conjugate-based drug can be utilized as a therapeutic or preventive agent for specific diseases. When exposed to a rotating magnetic field, the m-Torquer conjugate bound to the drug target ion channel can induce biological activity (e.g., cellular depolarization or hyperpolarization) within seconds or therapeutic effects. The ion channels capable of exerting biological activity or therapeutic effects within seconds include ligand-gated ion channels or G-protein-coupled receptors.

The m-Torquer conjugate-based drug of the present invention can exert its pharmacological effects through the gating mechanism of the drug target ion channel to which the m-Torquer conjugate is bound. To achieve this, the m-Torquer conjugate-based drug can be designed to maintain the ion selectivity or narrow selectivity filter function of the drug target ion channel, taking into account pore and filter selectivity, specifically $Na^+ > Li^+ >> K^+$, **$Ca^{2+}$, $Mg^{2+}$**.

Additionally, cardiotoxicity can be controlled through localized administration.

The m-Torquer conjugate-based drug of the present invention can be used for the treatment of epilepsy, ADHD, chronic pain disorders, or for the inhibition of pain perception.

In the present invention, the subject to be treated may be a mammal, including humans, primates, mice, rats, cattle, pigs, horses, sheep, dogs, and cats.

For regions such as the brain and nervous system, where functions differ by area, precise stimulation is critical. First, accurate mapping of the functions of nerves and tissues within the body must be established. Furthermore, clinical effects of stimulation intensity and frequency upon application of a controlled rotating magnetic field should be evaluated through data collection on biological signals, enabling the implementation of a biofeedback-based closed-loop system (a system in which the output signal directly influences the control operation).

Given the sensitivity of stimulating the internal nervous system of the human body, the m-Torquer conjugate-based drug's impact on patients can be accurately measured by integrating a closed-loop control system and software capable of real-time collection and analysis of biological signals, allowing immediate adaptive responses. Since the therapeutic effects of the m-Torquer conjugate-based drug's mechanism of action may vary among patients, a clear understanding of its mechanism is essential to achieve effective therapeutic outcomes. This allows for the personalized prescription of physical stimulation parameters, including intensity, frequency, and duration, tailored to individual patients. Additionally, the m-Torquer conjugate-based drug's physical stimulation may be co-administered with conventional chemical drugs as part of a combination therapy.

The present invention enables the structural transformation (conformational change) of a drug target protein bound to an m-Torquer conjugate upon application of a rotating magnetic field, thereby preferably and reversibly controlling its activation (e.g., the opening and closing of ion channels), allowing for the regulation of neural information transmission, such as synaptic transmission, sensory perception, and muscle contraction.

According to the present invention, an m-Torquer conjugate bound to a drug target protein that interacts with a neurotransmitter or hormone can function as various agonists/antagonists, precisely regulating the cellular effects of the neurotransmitter or hormone upon application of a controlled rotating magnetic field by facilitating, modulating, and/or inhibiting such effects-optionally even in the absence of the neurotransmitter or hormone.

Furthermore, an m-Torquer conjugate bound to a drug target protein that interacts with neurotransmitters or hormones can, upon application of a controlled rotating magnetic field, either activate or inhibit the activity of target cells such as neurons or muscle cells.

Additionally, the m-Torquer conjugate-based drug of the present invention can be designed for targeted biodistribution, reaching specific neural cells via cerebrospinal fluid (CSF).

In accordance with the present invention, the drug target protein to which the m-Torquer conjugate binds can stimulate neural circuits to regulate metabolic functions.

Moreover, an m-Torquer conjugate bound to a drug target protein that interacts with neurotransmitters or hormones can, upon application of a controlled rotating magnetic field, facilitate neuromodulation-such as deep brain stimulation (DBS) and/or artificial stimulation of the peripheral nervous system-to induce physiological responses.

According to the present invention, an m-Torquer conjugate bound to a drug target protein can alter the electrical properties of the membrane upon application of a controlled rotating magnetic field, wherein the target cell may be an excitable cell such as a neuron or a muscle cell.

Further, in accordance with the present invention, an m-Torquer conjugate bound to a drug target protein can precisely regulate neural activity for a specific duration (on the order of seconds with relatively fine temporal resolution) upon application of a controlled rotating magnetic field.

## [Neuroregulatory Drug Targeting the Central and Peripheral Nervous Systems]

The m-Torquer conjugate-based drug of the present invention can be designed to specifically target neurons through its binding moiety (b), which interacts with drug target proteins embedded in the biological lipid membrane.

For instance, the m-Torquer conjugate-based drug of the present invention can be designed such that the gating of ion transport membrane proteins is controlled via structural changes induced by the rotational force of magnetic nanoparticles generated under a controlled rotating magnetic field. This may involve:

(1) Magnetic nanoparticles designed to respond to an external rotating magnetic field, generating a rotational force that is sufficiently small to avoid tissue damage while still being strong enough to modulate mechanosensitive protein activity; and
(2) A binding moiety specifically designed to interact with ion transport membrane proteins.

Furthermore, the binding moiety of the m-Torquer conjugate in the present invention may selectively target specific types of brain cells located in deep brain structures. In such cases, the drug target protein selectively targeted by the m-Torquer conjugate may be either naturally expressed or artificially introduced in certain brain cell types. For example, the m-Torquer conjugate-based drug can regulate neurons in the lateral hypothalamus (LH), a key brain region regulating appetite, to control feeding behavior and dietary intake in animals.

Additionally, the m-Torquer conjugate-based drug enables wireless remote control of drug target protein activation-specifically, conformational changes-at high temporal precision and resolution. This allows for neural activation and/or inhibition with relatively fine time resolution on the order of seconds.

Accordingly, the m-Torquer conjugate-based drug of the present invention can be employed for neuromodulation by selectively targeting either (i) the central nervous system (CNS), which regulates metabolism via the release of neuroendocrine signals, or (ii) the peripheral nervous system (PNS), which transmits neural signals from the CNS to various organs.

Thus, the m-Torquer conjugate-based drug of the present invention can serve as a physiologically functional regulatory drug, behavioral modulation drug, or anticancer agent that provides mechanical magnetic stimulation to target cells-including neurons, secretory cells, muscle cells, or cancer cells-through rotational magnetic force in a non-invasive manner.

Moreover, the m-Torquer conjugate-based drug can be used as a pain modulation or behavioral regulation drug that non-invasively delivers mechanical rotational force stimulation to spinal cord neurons, a key component of the CNS, upon application of a rotating magnetic field.

To regulate physiological functions, various mechanisms can be employed, including modulating the permeability of the cell membrane to ions, facilitating the release of neurotransmitters at the presynaptic nerve fiber terminals, regulating intracellular $Ca^{2+}$ availability for muscle contraction, and promoting the secretion of catecholamines from the adrenal medulla.

Additionally, the m-Torquer conjugate-based drug of the present invention can modulate animal behavior or treat physiological disorders and diseases through long-term, neuron-specific neuromodulation by applying a controlled rotating magnetic field in a patterned manner.

Furthermore, when the m-Torquer conjugate binds to a drug target protein, the rotational force of the magnetic nanoparticles generated under a controlled rotating magnetic field is transmitted to the drug target protein. The stimulation delivered by the m-Torquer conjugate-based drug of the present invention can promote recovery and regeneration following nerve injury and restore lost functions. Specifically, stimulation using the m-Torquer conjugate-based drug can enhance axonal regeneration of damaged peripheral nerves, improve functional connectivity, and activate spinal cord circuits.

The neural control method using the m-Torquer conjugate-based drug of the present invention can regulate neurotransmitter activation by artificially modulating neuronal action potentials. Through this mechanism, it can restore disrupted homeostasis in metabolic functions. In addition to the vagus nerve, this approach can also be applied to the sacral nerve (sacral nerve stimulation) for the treatment of fecal incontinence and gastrointestinal disorders.

Rheumatoid arthritis is an autoimmune disease that affects the joints, characterized by an inflammatory response in the synovial membrane. Synovitis softens and erodes joint surface tissues, ultimately impairing joint function. Most patients with arthritis rely on medications to alleviate pain; however, severe side effects associated with these drugs remain a significant concern.

The vagus nerve plays a crucial role in detecting and regulating inflammation. The mechanism of neural control of the vagus nerve using the m-Torquer conjugate-based drug of the present invention operates on a principle similar to that of drug administration for inhibiting the production of cytokines, which are inflammatory mediators.

By targeting the drug target protein present in the vagus nerve, which detects and regulates inflammation, or in the postsynaptic target cells synapsing with the vagus nerve, the m-Torquer conjugate enables non-invasive control through the application of a controlled rotating magnetic field. When the drug target protein is magnetically stimulated, acetylcholine, a neurotransmitter, is produced. The generated acetylcholine then acts on cytokine-secreting cells, thereby suppressing cytokine secretion and alleviating arthritis and its associated pain.

Diabetes is a chronic metabolic disorder characterized by elevated blood glucose levels. Type 1 and Type 2 diabetes are, respectively, an autoimmune disease in which pancreatic β-cells are destroyed, leading to limited insulin secretion, and a disorder caused by insulin resistance, both of which result in chronic hyperglycemia.

Glucose-sensing cells in the oral cavity, intestines, and hepatoportal vein relay glucose-related information to the brainstem and hypothalamus. To maintain glucose homeostasis, these brain regions regulate hepatic glucose production and hormone secretion in the endocrine pancreas via the vagus nerve. For instance, vagal terminals connected to the pancreas release acetylcholine, which stimulates pancreatic β-cells to secrete insulin. The vagus nerve is known to regulate not only insulin secretion but also β-cell proliferation and insulin sensitivity.

Therefore, when the m-Torquer conjugate-based drug targets the vagus nerve connected to the pancreas or drug target proteins in postsynaptic target cells synapsing with the vagus nerve, it enables non-invasive magnetic stimulation via a rotating magnetic field. Through the induced conformational changes in the drug target protein bound to the m-Torquer conjugate, insulin secretion, pancreatic β-cell proliferation, and/or insulin sensitivity can be modulated without the need for exogenous insulin administration.

Obesity is strongly associated with a high level of comorbidities, including cancer, cardiovascular diseases, and diabetes. While electrical stimulation of the abdominal nerves alone can reduce food intake and body weight, stimulating the vagus nerve may activate the emetic pathway, causing confusion similar to that experienced during food consumption. Additionally, vagus nerve stimulation is known to induce responses that regulate metabolism and digestive processes, including the secretion of insulin, glucagon, and gastric acid.

Accordingly, if the m-Torquer conjugate of the present invention targets a drug target protein present in the vagus nerve that regulates metabolism and digestive processes, including insulin, glucagon, and gastric acid secretion, non-invasive application of a rotating magnetic field may induce conformational changes in the m-Torquer conjugate-bound drug target protein, thereby enabling obesity treatment.

The Claudin protein family consists of four-pass transmembrane proteins present in tight junctions of epithelial and endothelial cells. Their primary function is to maintain tight junctions that regulate intercellular molecular exchange, and at least 27 members have been identified. In normal tissues, Claudin 18.2 (expressed in HEK293 cells) is exclusively expressed in differentiated epithelial cells of the gastric mucosa. However, in various malignant tumor tissues, such as gastric cancer, pancreatic cancer, cholangiocarcinoma, ovarian cancer, and lung cancer, its expression is significantly upregulated. In addition to monoclonal antibodies, drug modalities targeting Claudin 18.2 include bispecific antibodies, antibody-drug conjugates (ADCs), and CART therapies.

Therefore, if the m-Torquer conjugate of the present invention targets overexpressed multi-pass transmembrane proteins as drug target proteins-one of the key tools mediating intracellular and extracellular signaling while being restricted by the surface area of the cell membrane-the activity of these overexpressed multi-pass transmembrane proteins can be inhibited.

## ADVANTAGEOUS EFFECTS

The m-Torquer conjugate-based drug of the present invention utilizes magnetic nanoparticles (m-Torquer) that generate rotational force at a working distance of at least 20 cm, preferably at least 50 cm, and, for example, up to 70 cm from a rotating magnetic field generator. This system can induce beneficial modifications in biological functions or pathological processes of the body, enabling the treatment of various diseases, including intractable disorders. The m-Torquer conjugate-based drug can selectively treat localized areas of the body by non-invasively applying a rotating magnetic field, mechanically stimulating target neural cells, secretory cells, muscle cells, or cancer cells via rotational magnetic force. This system can achieve therapeutic effects equivalent to conventional pharmaceuticals, and it may be particularly effective for conditions that are difficult to treat with conventional drugs or surgical interventions.

The m-Torquer conjugate-based drug of the present invention can non-invasively regulate neural signals through the application of a rotating magnetic field, thereby treating or alleviating metabolic disorders caused by disruptions in neural circuits.

The m-Torquer conjugate-based drug of the present invention can detect real-time changes in a patient's symptoms and adjust therapeutic stimulation accordingly, enabling the development of personalized treatments. Additionally, it allows for the remote monitoring of symptom-related data. Its applications can be expanded to the treatment of various conditions, ranging from neuropsychiatric disorders to obesity, autoimmune diseases such as rheumatoid arthritis, and intractable diseases including dementia, cancer, and Parkinson's disease.

The physical stimulation sites for symptom relief extend from spinal nerves to deep brain structures and the vagus nerve, encompassing various neural targets within the body. By identifying disease-related neural circuits and precisely mapping the anatomical regions, the m-Torquer conjugate-based drug of the present invention can be designed after detecting existing electrical signals or action potentials. The m-Torquer conjugate-based drug can be wirelessly controlled and provides localized and appropriately tuned physical stimulation to minimize side effects.

Through the application of the m-Torquer conjugate-based drug, magnetogenetics can be utilized for diverse neuroscience research applications. This includes the selective interrogation of brain cell populations and neural circuits, tethered optogenetic control, studies on complex behavioral paradigms that are otherwise disrupted in unnatural experimental environments, and research on neurological disorders that require long-term stimulation and response monitoring.

**BRIEF DESCRIPTION OF THE DRAWINGS/FIGURES**

**Fig. 1** is a conceptual diagram illustrating current force actuators (probes) for mechanical stimulation.

**Fig. 2** compares the working distance and rotational force generated upon applying a rotating magnetic field between a conventional T-tweezer and the magnetic nanoparticles (also referred to as m-Torquer) of the present invention.

**Fig. 3** is a conceptual diagram depicting the rotational force of magnetic nanoparticles and the arrangement of magnets.

**Fig. 4** presents a schematic diagram (top) showing the structure of the m-Torquer (a magnetic nanoparticle that generates rotational force upon application of a rotating magnetic field) used in Examples 1 and 2, along with a conceptual diagram (bottom) illustrating the magnetic modulation of target neurons using the m-Torquer conjugate-based drug in Example 2.

The magnetic nanoparticle (m-Torquer) has a particle size ranging from approximately 300 to 700 nm. In one embodiment of the present invention, the core-shell structured magnetic nanoparticle (m-Torquer), which generates rotational force upon application of a rotating magnetic field, exhibits a magnetic moment that is over 470 times higher compared to an octahedral unit magnetic nanoparticle.

**Fig. 5** is a conceptual diagram illustrating the bidirectionality of neuromodulation and comparing the operational principles of optogenetics and magnetogenetics.

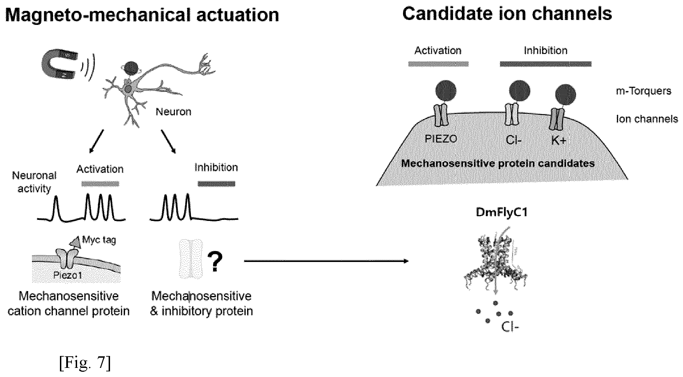

**Fig. 6** depicts a method for identifying candidate ion channels for magnetogenetic neuronal inhibition and provides a schematic comparison of excitatory and inhibitory ion channels. When ion channels are opened in response to stimulation, unlike excitatory ion channels, inhibitory ion channels allow anions to enter the cell, leading to membrane hyperpolarization and suppression of neuronal activity.

In this process, if the transported ion is a cation and its transport direction is inward, membrane depolarization occurs upon channel opening, thereby inducing neuronal activity. Conversely, if the transported ion is a cation but its transport direction is outward, membrane hyperpolarization occurs upon channel opening, leading to neuronal inhibition. Similarly, when the transported ion is an anion, its inward transport through an open channel results in membrane hyperpolarization, thereby suppressing neuronal activity.

**Fig. 7** is a conceptual diagram illustrating the regulation of anion transport membrane protein gating via the rotational force of the magnetic nanoparticle (m-Torquer) in the m-Torquer conjugate-based drug (top) and the potential for neuronal inhibition in an ex vivo system (bottom).

**Fig. 8** presents the m-Torquer tagging strategy for channel X and the regulation of candidate protein function using magnetogenetic approaches.

**Fig. 9** is a conceptual diagram depicting neuronal inhibition using candidate mechanosensitive anion channels, modulation of brain function, and behavioral regulation in animal experiments.

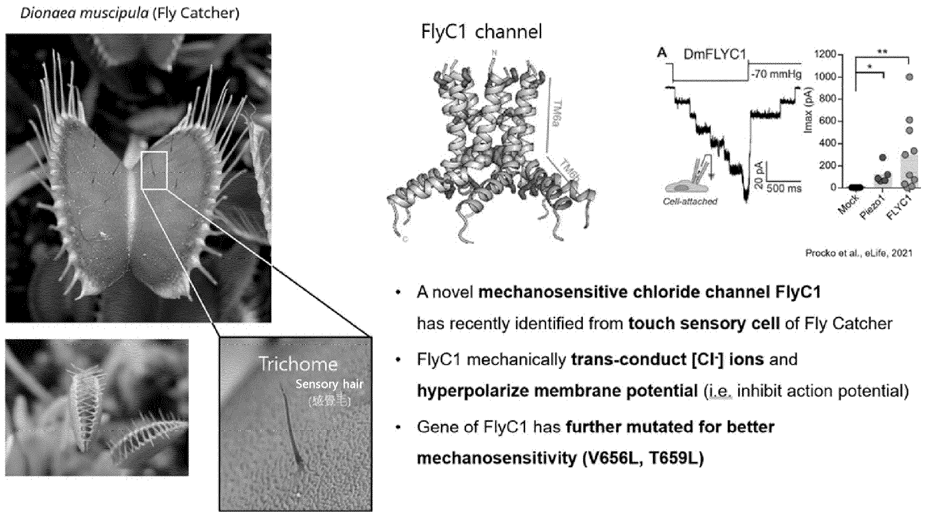

**Fig. 10** provides an overview of the selection of DmFLYC1, a plant-derived novel mechanosensitive chloride channel protein, as a candidate anion channel protein and its genetic engineering.

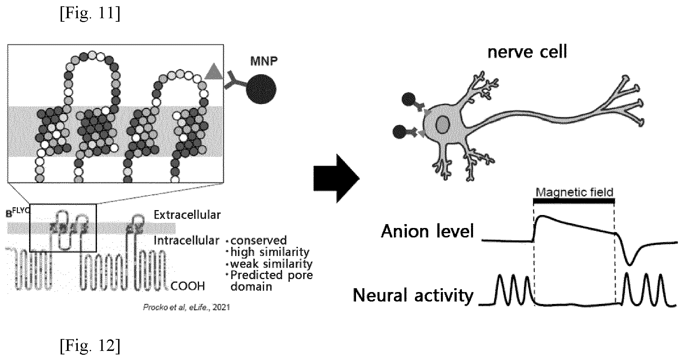

**Fig. 11** is an overview of the experimental design of Example 1.

**Fig. 12** shows the design of the binding site of the m-Torquer conjugate to the drug target protein, ensuring that the binding site is positioned at a specific location within the extracellular loop domain of the ion transport membrane protein, along with modifications incorporating tagging proteins at the binding site.

**Fig. 13** presents a schematic representation of the formation of the DmFLYC1-Myc ion channel according to Example 1, along with verification images.

**Fig. 14** demonstrates the expression of DmFLYC1 and the targeting of its extracellular epitope (Expression and extracellular epitope targeting of DmFLYC1).

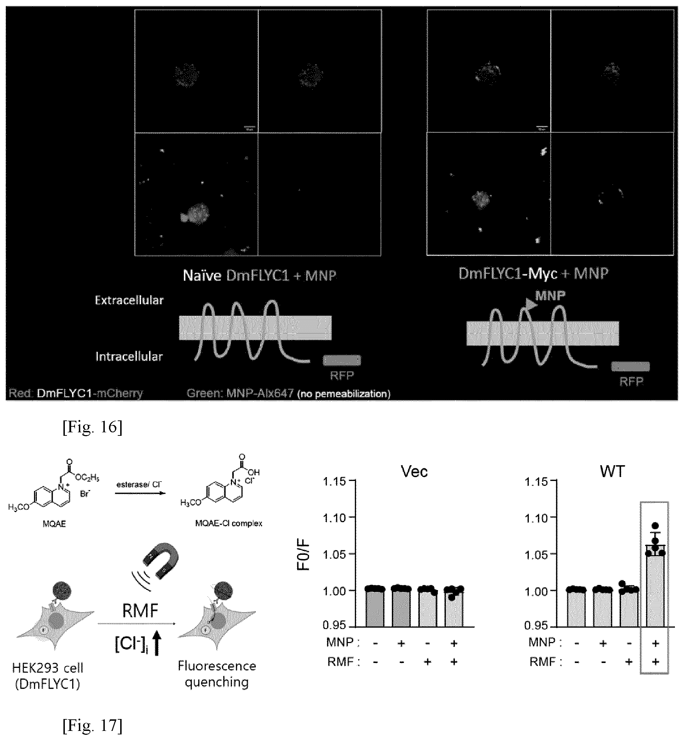

**Fig. 15** shows images illustrating the targeting of DmFLYC1 with magnetic nanoparticles.

Fig. 16 outlines the methodology for verifying chloride ion (Cl⁻) influx using the MQAE quenching technique.

**Fig. 17** presents the results of measuring the membrane potential of cells expressing DmFLYC1 ion channels after treatment with magnetic nanoparticles and subsequent exposure to a magnetic field over a defined time period, as performed in Example 1.

**Fig. 18** illustrates the objectives of Example 2 and provides an overview of cell-type-specific neuromodulation using magneto-mechanical genetics (MMG).

**Fig. 19** is a conceptual diagram illustrating the research strategy and methodology employed in Example 2.

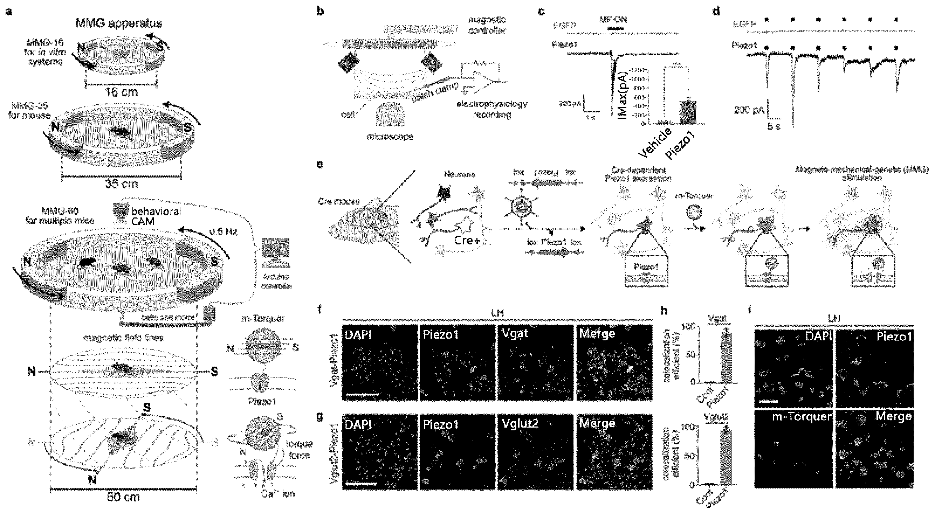

**Fig. 20** illustrates the cell-type-specific neuromodulation using magneto-mechanical genetics (MMG) as applied in Example 2. (a) MMG apparatus. The MMG apparatus with various sizes (16, 35, and 60 cm) is constructed for both *in vitro* and *in vivo* studies. For *in vivo* experiments of untethered, freely moving animals, the apparatus with 60 cm diameter and 20 cm height is used. As a magnetic field rotates with 0.5 Hz, m-Torquer rotates at the same frequency as the magnetic field, generating forces to activate Piezo1 channels. **(b)** Schematic illustration showing a patch-clamp set-up for electrophysiological recording during MMG stimulation. **(c)** Representative whole-cell voltage-clamp trace from a Piezo1-expressing (black) and a EGFP-transfected (gray) HEK293 cell evoked by a single pulse (1.0 s, 0.5 Hz, 180°) MMG stimulation. (right inset) Quantification of peak current amplitudes. Data is the mean ± s.d. of n = 9 cells. *** p < 0.001; two-tailed Student's t test. **(d)** Representative current traces recorded from a Piezo1- and EGFP-expressing HEK cell in response to a train of 6 repetitive magnetic pulses. Each black bar represents a single magnetic pulse with a 1-sec duration and 0.5 Hz frequency. **(e)** Experimental procedure of Cre-loxP based cell-type specific MMG control of deep brain neural circuit. The Cre-loxP technique is employed to enable cell type-specific expression of Piezo1 mechanosensitive ion channels. m-Torquers are specifically labeled to Piezo1-expressing neurons. Upon a rotating magnetic field, torque forces generated by m-Torquer mechanically open the bound Piezo1 channels and activate specific neurons. **(f, g)** Representative fluorescence histology images of the lateral hypothalamus (LH) region showing co-localization of Piezo1 with Vgat in Vgat-Piezo1 mouse (f) or with Vglut2 in Vglut2-Piezo1 mouse (g) Scale bar, 50 μm. **(h)** Statistical analysis of the fraction of Piezo1-expressing cells that are Vgat-positive or Vglut2-positive in respective Vgat-Piezo1 or Vglut2-Piezo1 mice. n = 3 animals. **(i)** Representative histology images showing co-localization of m-Torquers and Piezo1 expression in the LH region. Scale bar, 10 μm.

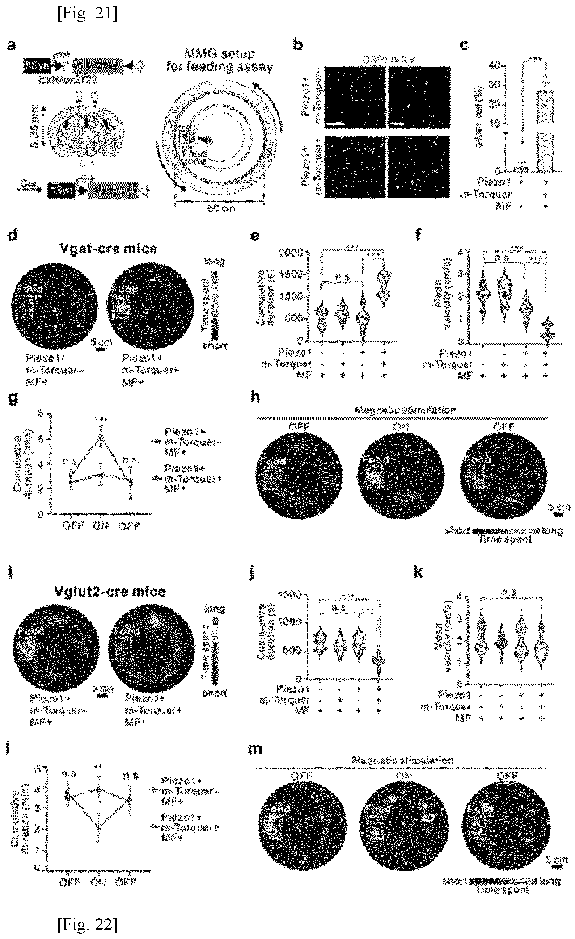

**Fig. 21.** presents the Vgat- and Vglut2 specific MMG of the lateral hypothalamus (LH) for the bidirectional regulation of real-time animal feeding. (a) Scheme for viral targeting of Ad-hSyn-FLEX-Piezo1 to the LH of Cre mice and real-time feeding assay. **(b)** Fluorescence histology images of Vgat-Piezo1 brain slices for c-Fos expression (red) at the LH in the absence (upper) and in the presence (lower) of m-Torquers upon MMG stimulation. Scale bar, 50 μm. Right: magnified images of the boxed region. Scale bar, 20 μm. **(c)** Quantification of c-fos-positive cells in the LH of Vgat-Piezo1 mice upon MMG stimulation. Data is the mean ± s.d. of n = 3 animals. *** p < 0.001; two-tailed Student's t test. **(d)** Representative spatial heat map for MMG stimulation of Vgat neurons in the LH of Vgat-Piezo1 mice during real-time feeding assay. **(e, f)** Statistical analysis of feeding duration (e) and locomotion (f) of Vgat-Piezo1 mice during MMG stimulation. **(g)** Statistical analysis of feeding duration and (h) representative spatial heap map of Vgat-Piezo1 mice during 30-min home cage feeding task with three 10-min epochs (pre-stimulation, during stimulation, and post-stimulation). **(i)** Representative spatial heat map for magnetogenetic stimulation of Vglut2 neurons in the LH of Vglut2-Piezo1 mice during real-time feeding assay. **(j, k)** Statistical analysis of feeding duration (i) and locomotion (j) of Vglut2-Piezo1 mice during MMG stimulation. **(l)** Statistical analysis of feeding duration and **(m)** representative spatial heap map of Vglut2-Piezo1 mice during 30-min home cage feeding task with three 10-min epochs (pre-stimulation, during stimulation, and post-stimulation). **(e, f, g, j, k, l)** All data are presented as mean ± s.d.; ** p<0.01; *** p < 0.001; ns, non-significant; one-way ANOVAfollowed by Tukey's multiple comparison tests; n = 5-8 animals per group.

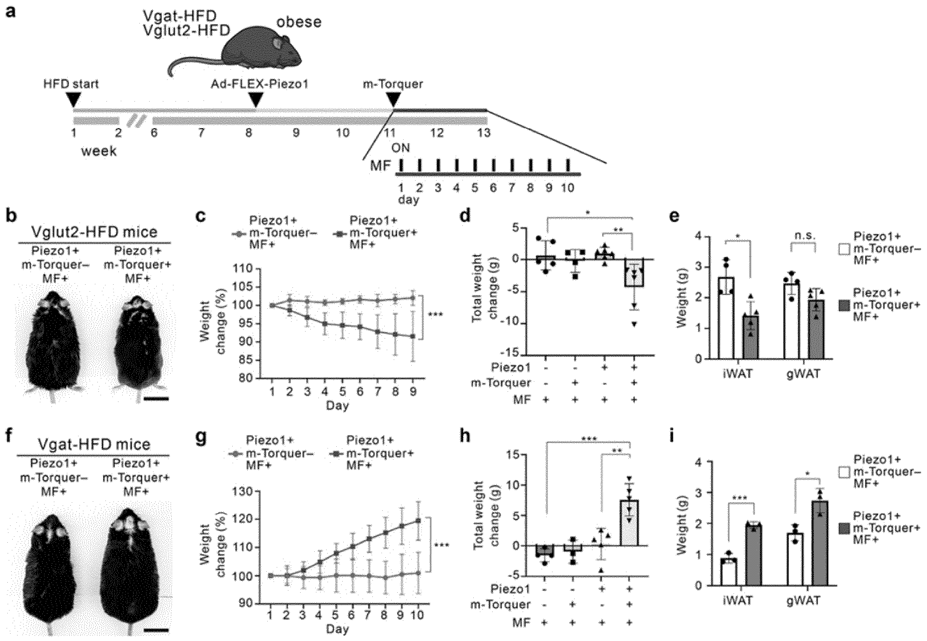

**Fig. 22** demonstrates a Long-term cell-type specific neuromodulation for controlling dietary habit and body-weight change in mice. (a) *In vivo* experimental scheme for long-term neuromodulation of Vglut2- or Vgat-Piezo1 HFD obese mice. Diet-induced obesity was induced by the 8-week high-fat diet regime. After Cre-dependent Piezo1 expression and m-Torquer delivery into the LH, mice were subjected to 2-week MMG stimulation (1-hr/day, 0.5 Hz). **(b)** Representative photograph showing Vglut2-Piezo1 HFD mice in the absence of m-Torquers (left) and in the presence of m-Torquers (right) after long-term stimulation. **(c)** Body-weight changes of Vglut2-Piezo1 HFD mice over 10 days of MMG stimulation (1-hr/day, 0.5 Hz). Body weights were measured daily. n = 6 (Piezo1+ m-Torquer-) and 6 (Piezo1+m-Torquer+) animals per group. **(d)** Statistical analysis of total weight changes after long-term MMG stimulation in Vglut2-Piezo1 HFD mice. **(e)** Statistical analysis of mass (gram) of white adipose tissues acquired from Vglut2-Piezo1 HFD mice after 2-week MMG stimulation. **(f)** Representative photograph showing Vgat-Piezo1 HFD mice in the absence of m-Torquers (left) and in the presence of m-Torquers (right) after long-term stimulation. **(g)** Body-weight changes of Vgat-Piezo1 HFD mice over 10 days of MMG stimulation (1-hr/day, 0.5 Hz). Body weights are measured daily. n = 5 (Piezo1+ m-Torquer-) and 5 (Piezo1+m-Torquer+) animals per group. **(h)** Statistical analysis of total weight changes after long-term MMG stimulation in Vgat-Piezo1 HFD mice. **(i)** Statistical analysis of mass (gram) of white adipose tissues acquired from Vgat-Piezo1 HFD mice. **(b, f)** scale bar = 2 cm, **(c, g)** All data are presented as mean $\pm$ s.d.; Two-tailed unpaired student's t test, *** p<0.001. **(d, e, h, i)** All data are presented as mean $\pm$ s.d.; One-way ANOVA with multiple comparison test, * p<0.05, *** p<0.01, n.s., non-significant. n = 5 (Piezo1-m-Torquer-), 4 (Piezo1-m-Torquer+), 6 (Piezo1+m-Torquer-) and 6 (Piezo1+m-Torquer+) animals per group. **(e, i)** iWAT and gWAT stand for inguinal white adipose tissue and gonadal white adipose tissue, respectively.

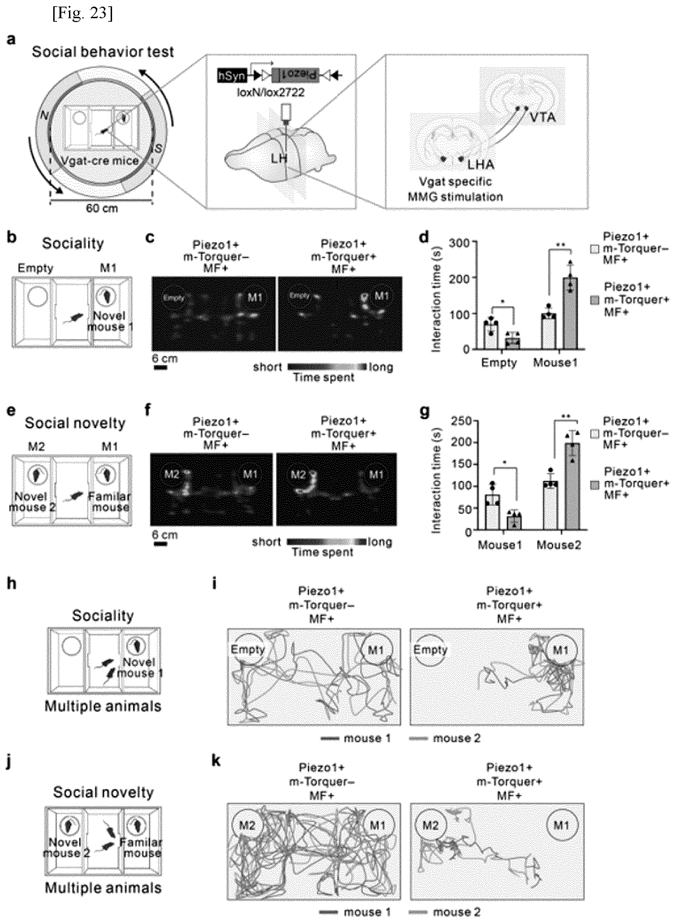

**Fig. 23** shows the MMG stimulation of GABAergic neurons in the LH for promoting sociality and social novelty. (a) (left) MMG setup for the three-chamber sociality assay. (center) Viral delivery for Cre-dependent Piezo1 expression in Vgat neurons in the LH$_A$. (right) Schematics of LH$_A$-VTA projection regulating social behaviors. **(b)** Schematic of the three-chamber sociality test. **(c)** Representative heat map encoding the spatial location of a Vgat-Piezo1 mouse without (left) and with (right) m-Torquers in a three-chamber sociality test during MMG stimulation. **(d)** Quantification of time spent with an empty or an M1 chamber during 5-min stimulation. **(e)** Schematics of the three-chamber social novelty test. **(f)** Representative heat map encoding the spatial location of a Vgat-Piezo1 mouse without (left) and with (right) m-Torquers in the social novelty test during MMG stimulation. **(g)** Quantification of time spent with a familiar mouse (M1) or a second novel mouse (M2) during 5-min stimulation. Activity heat maps and total time spent in the social interaction zone in the three-chamber test are increased in Vgat-specific mice delivered with m-Torquers upon MMG stimulation. **(h)** Schematics of three-chamber sociality test with multiple (two) mice using MMG. **(i)** Representative spatial map tracking the movement of two Vgat-Piezo1 mice without (left) and with (right) m-Torquers in sociality assays upon MMG stimulation. **(j)** Schematics of three-chamber social novelty test with multiple (two) mice using MMG. **(k)** Representative spatial map tracking the movement of two Vgat-Piezo1 mice without (left) and with (right) m-Torquers in social novelty assays upon MMG stimulation. **(d, g)** All data are presented as mean $\pm$ s.d.; * p<0.05, ** p<0.01; two-tailed unpaired Student's *t* test; n = 4 for Vgat-Piezo1 without m-Torquers; n = 4 for Vgat-Piezo1 with m-Torquers.

**Fig. 24** depicts the experimental setup of MMG apparatus for wireless neuromodulation *in vivo.* (a) General components of MMG apparatus. The system consists of a rotational magnetic force generator (MFG), an Arduino controller, and a user interface system. **(b)** Representative photographs showing top and side views of MMG apparatus of different sizes. The 16-cm and 35-cm MMG apparatus are designed for cell-based experiments and *in vivo* studies of a single mouse, respectively. The 60-cm MMG apparatus is constituted of 10 NdFeB magnets with an arena with 60-cm in diameter, which can house multiple animals and is suitable for larger animals including primates. Scale bar, 5 cm. **(c)** A representative SEM image of a single m-Torquer (right). The 200-nm m-Torquer system is composed of assembled monolayer octahedral nanoparticles on spherical support via click chemistry. Scale bar, 100 nm.

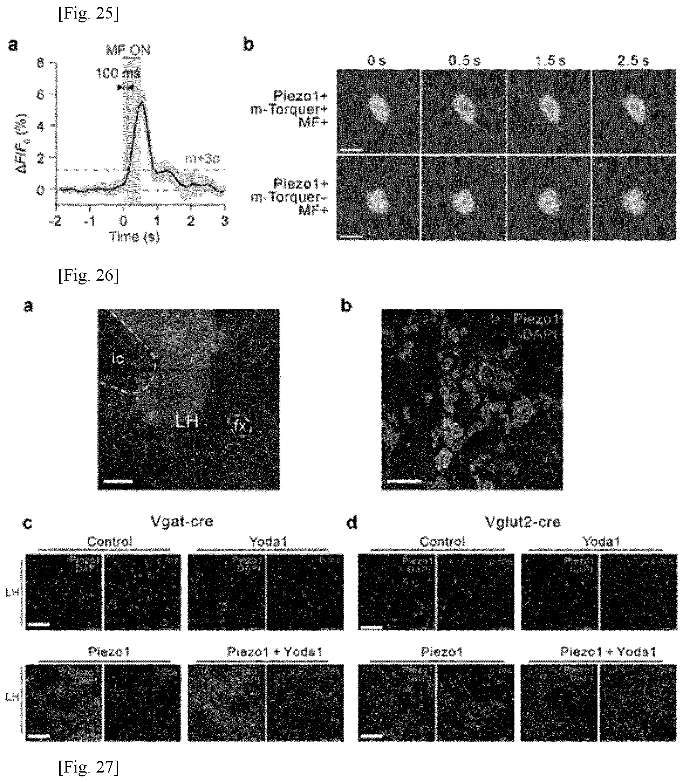

**Fig. 25** illustrates in situ calcium influx imaging of primary neurons using X-Rhod-1 in response to MMT stimulation. (a) Quantification of in situ Ca$^{2+}$ influx corresponding to MMG stimulation Piezo1-expressing and m-Torquer bound neurons. Neurons were stained with X-Rhod-1 Ca$^{2+}$ indicator dye. A single pulse has a 0.5 s duration (0.5 Hz, 90°). Data is the mean $\pm$ s.e.m of n = 8 neurons. **(b)** In situ calcium influx images (false-colour coded fluorescence) of a Piezo1-expressing neuron with (upper) and without (lower) m-Torquer upon MMG stimulation. Rapid and reversible calcium influx was observed only for the m-Torquer-bound neurons.

**Fig. 26** shows the expression and activity of Myc897-Piezo1 in the LH. (a) Representative histology images of brain slices showing Piezo1 expression in the LH region of Vglut2-Piezo1 mouse. Scale bar, 200 $\mu$m. **(b)** Membrane expression of Piezo1 in the LH region of Vgat-Piezo1 mouse. Blue; DAPI (nucleus), Green; Piezo (membrane). Scale bar, 20 $\mu$m. **(c, d)** Fluorescence histology images of brain slices showing Piezo1 (green) and c-Fos expression (red) at the lateral hypothalamus of Vgat- or Vglut2-Piezo1 mouse upon Yoda1 treatment. The expression of c-Fos was detected only in the Piezo1-expressing mice with Yoda1 treatment, confirming Piezo1 ion gating triggers neuronal activation. Scale bar, 50 $\mu$m.

**Fig. 27** presents neural activity in Vglut2 neurons in the LH by MMG stimulation. (a) Fluorescence histology images of Vglut2-Piezo1 brain slices showing c-Fos expression (red) at the lateral hypothalamus in the absence (left) and in the presence (right) of m-Torquers upon magnetogenetic stimulation. Scale bar, 50 $\mu$m (left), Scale bar, 20 $\mu$m (right). **(b)** Quantification of c-fos-positive cells in the LH of Vglut2-Piezo1 mice upon MMG stimulation. *** p < 0.001; two-tailed Student's t test; Data shown is mean $\pm$ s.d. from n = 3 animals.

**Fig. 28** demonstrates long-term, repeatable cell-type specific neuromodulation for modulating intake and fat mass in HFD obese mice. (a) Statistical analysis of total food intake after long-term MMG stimulation in Vglut2 HFD mice. Food intakes are measured daily. Data is mean $\pm$ s.d. from n = 5 (Piezo1-m-Torquer-), 4 (Piezo1-m-Torquer+), 5 (Piezo1+m-Torquer-) and 6 (Piezo1+m-Torquer+) animals. **(b)** Statistical analysis of total food intake after long-term MMG stimulation in Vgat HFD mice. Food intakes are measured daily. Data is mean $\pm$ s.d. from n = 3 (Piezo1-m-Torquer-), 3 (Piezo1-m-Torquer+), 5 (Piezo1+m-Torquer-) and 5 (Piezo1+m-Torquer+) animals. **(c)** Representative photograph of white adipose tissues acquired from Vglut2 HFD mice. **(d)** Representative photograph of white adipose tissues acquired from Vgat HFD mice. **(a, b)** One-way ANOVA test with multiple comparisons; * p<0.05, *** p<0.001, Scale bar for **(c, d)** 1 cm.

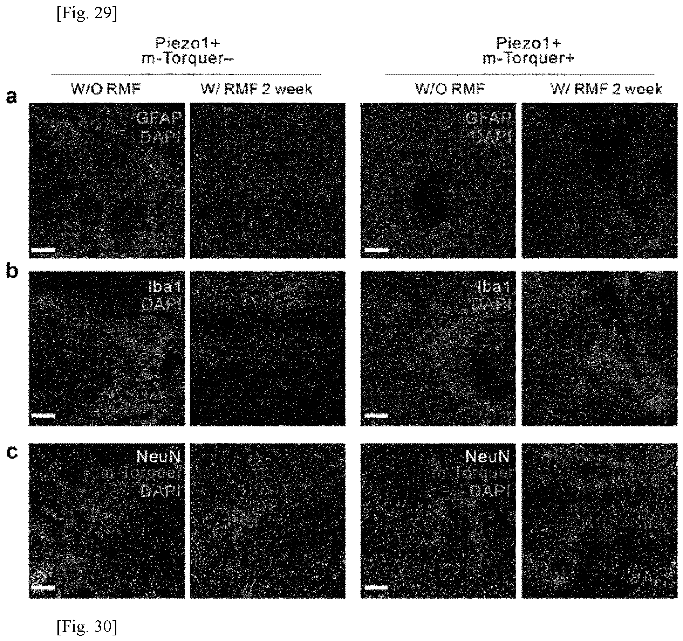

**Fig. 29** provides an assessment of chronic biocompatibility of m-Torquer injection. **(a-c)** Fluorescence histology images of brain slices showing **(a)** astrocytes (GFAP) **(b)** activated microglia (Iba1), and **(c)** neurons (NeuN) at the lateral hypothalamus of Vglut2-HFD mice after long-term MMG stimulation. Scale bar, 100 $\mu$m, Scale bar, 200 $\mu$m,

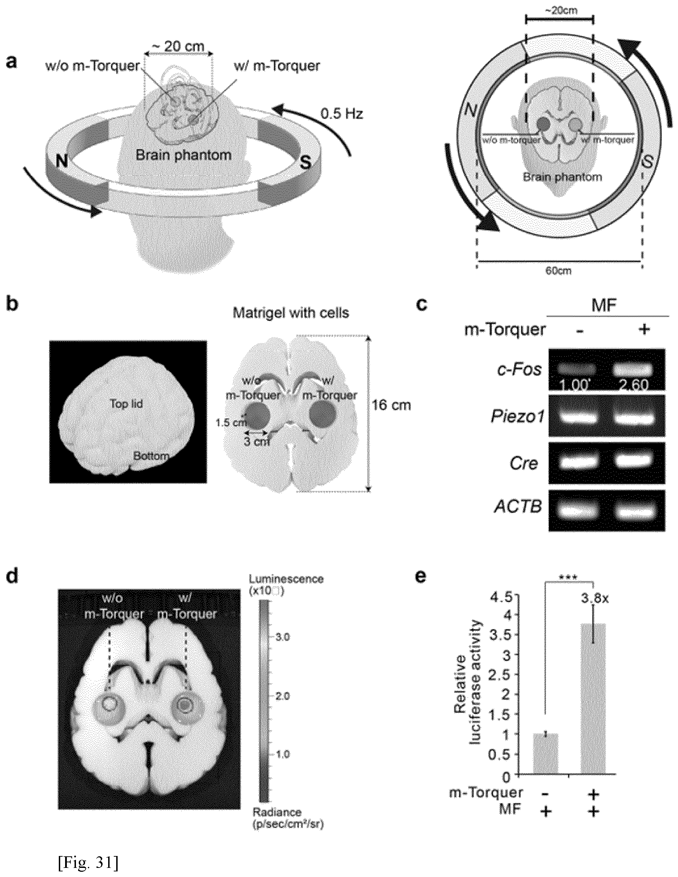

**Fig. 30** illustrates Cre-dependent Piezo1 activation with MMG stimulation in the pseudo primate brain. **(a)** Phantom model of a primate brain for long-range stimulation at the magnetically targeted position. **(b)** 3D printed primate brain phantom. Matrigel-embedded Piezo1 cells are cultured in the 3D large animal brain phantom to make *in vivo*-comparable conditions. **(c)** RT-PCR analysis of HEK293 cells co-transfected Cre and FLEX-Piezo1 confirming increased *c-Fos* expression in response to MMG stimulation. **(d)** Representative bioluminescence imaging (BLI) showing a higher luciferase expression in response to Cre-dependent magnetomechanical activation of Piezo1-based transcription in 3D primate brain phantom model. **(e)** MMG stimulation induces transcription of the intracellular $Ca^{2+}$-dependent luciferase reporter. All data are presented as mean $\pm$ s.d.; *** p < 0.001; two-tailed Student's t test; n = 3 biological replicates.

**Fig. 31** illustrates a conceptual diagram of receptor classification in vivo and their mechanisms of action.

**Figs. 32a** and **32b** respectively depict the mechanisms by which neurotransmitters are released from the presynaptic neuron to either the postsynaptic neuron **(Fig. 32a)** or the target cell **(Fig. 32b),** thereby triggering or inhibiting the activity of the postsynaptic neuron or target cell.

**Fig. 33** presents a schematic diagram illustrating the principles of synaptic integration.

## Mode for carrying out the invention

The following examples are provided to facilitate understanding of the invention. These examples are presented solely for illustrative purposes and do not limit the scope of the invention.

### Example 1: Regulation of Anion Transport Membrane Protein Gating and Inhibition of Neural Activity via Rotational Force of Magnetic Nanoparticles

The present example describes a method for inducing anion influx into specific target cells using a magnetogenetic approach. The method involves the use of anion transport membrane proteins, magnetic nanoparticles (m-Torquer), a magnetic field generator, and a rotating magnetic field.

Following the methodology outlined in Nature Materials (2022) (Nat Mater. 2021 Jul;20(7):1029-1036), the following components were prepared (1) m-Torquer, (2) m-Torquer conjugated with fluorescent particles and Myc antibodies, (3) Magnetic simulation, and (4) CMA.

The detailed methodology of the present example is described in Korean Patent Application No. 10-2022-0100260, filed on August 10, 2022, and the contents of that application are incorporated herein by reference.

The m-Torquer magnetic nanoparticles used in the present example have a core-shell structure, with the shell containing octahedron-shaped unit magnetic nanoparticles that are randomly arranged via 1,2,3-Triazole bonds to the core (Fig. 4). SEM imaging confirmed that these nanoparticles have a diameter of approximately 300 to 700 nm. Compared to individual octahedral magnetic nanoparticles, the m-Torquer nanoparticles exhibit a magnetic moment that is over 470 times higher.

The present example provides a method for inducing anion influx into target cells using a magnetogenetic technique, which includes the following steps:

(1) Generation of an anion transport membrane protein capable of binding to m-Torquer magnetic nanoparticle.

(2) Production of an anion transport membrane protein whose gating can be regulated using the magnetogenetic technique.

(3) Expression of the anion transport membrane protein in target cells, followed by treatment with magnetic nanoparticles to enable binding.

(4) Application of a rotating magnetic field to the magnetic nanoparticles, delivering rotational force stimulation to the anion transport membrane protein and thereby activating it.

In Step (1), a nucleotide sequence encoding an antigenic motif (i.e., a polypeptide) was synthesized and introduced into the extracellular loop domain of the anion transport membrane protein to facilitate binding to the antibody moiety of the magnetic nanoparticles (see Fig. 12 for the arch-shaped binding site of the ion channel).

As a specific example, the present study utilized the mechanosensitive anion channel DmFLYC1, which mediates chloride ion transport (Figs. 9 and 10).

The amino acid sequence of the recombinant anion transport membrane protein, expressed based on the corresponding genetic sequence, is designated as Sequence ID No. 1, whereas the amino acid sequence of the protein tag is designated as Sequence ID No. 2.

Accordingly, the anion transport membrane protein expressed in cells based on Sequence ID No. 1 contains an extracellular domain displaying a protein tag composed of the amino acid sequence denoted as Sequence ID No. 2.

Accordingly, the anion transport membrane protein, expressed in cells based on Sequence ID No. 1, includes an extracellular domain that presents a protein tag comprising the amino acid sequence designated as Sequence ID No. 2.

[table 1]

| Sequence ID No. | Name | Amino Acid Sequence |
|---|---|---|
| Sequence ID No. 1. | Amino Acid Sequence of DmFLYC1 Anion Transport Membrane Protein | MGSYLHEPPGDEPSMRIEQPKTADRAPEQVAIH ICEPSKVVTESFPFSETAEPEAKSKNCPCPEIARI GPCPNKPPKIPINRGLSRISTNKSRPKSRFGEPS WPVESSLDLTSQSPVSPYREEAFSVENCGTAGS RRGSFARGTTSRAASSSRKDETKEGPDEKEVY QRVTAQLSARNQKRMTVKLMIELSVFLCLLGC LVCSLTVDGFKRYTVIGLDIWKWFLLLLVIFSG MLITHWIVHVAVFFVEWKFLMRKNVLYFTHG LKTSVEVFIWITVVLATWVMLIKPDVNQPHEQ KLISEEDLQTRKILEFVTWTIVTVLIGAFLWLVK TTLLKILASSFHLNRFFDRIQESVFHHSVLQTLA GRPVVELAQGISRTESQDGAGQVSFMEHTKTQ NKKVVDVGKLHQMKQEKVPAWTMQLLVDVV SNSGLSTMSGMLDEDMVEGGVELDDDEITNEE QAIATAVRIFDNIVQDKVDQSYIDRVDLHRFLI WEEVDHLFPLFEVNEKGQISLKAFAKWVVKVY NDQAALKHALNDNKTAVKQLNKLVTAILIVM MIVIWLIVTGIATTKLIVLLSSQLVVAAFIFGNT CKTIFEAIIFVFVMHPFDVGDRCVIDGNKMLVE EMNILTTVFLKWDKEKVYYPNSILCTKAIGNFF RSPDQGDVLEFSVDFTTPVLKIGDLKDRIKMYL EQNLNFWHPQHNMVVKEIENVNKIKMALFVN HTINFQDFAEKNRRRSELVLELKKIFEELDIKYN LLPQEISIRNM |
| Sequence ID No. 2. | Myc Tag Sequence | EQKLISEEDL |

In Step (2), the responsiveness of the anion transport membrane protein to the rotational force of the magnetic nanoparticles was analyzed to assess its capability for functional modulation. If necessary, the nucleotide sequence was synthesized and modified to enhance its sensitivity to mechanical stimulation, ensuring adequate functional control (Fig. 15).

In Step (3), the genetic information of the anion transport membrane protein was introduced in a form suitable for the target cells to facilitate its expression (Fig. 16). This step includes the use of a viral vector as a method for gene delivery, wherein a viral vector carrying the gene encoding the anion transport membrane protein was constructed and subsequently introduced into the target cells or the tissue of an animal (Fig. 13).

After gene delivery, a step was performed to induce expression of the anion transport membrane protein in the target cells. When using viral vectors for gene delivery, this process took approximately 2 to 3 weeks. After expression of the anion transport membrane protein in the target cells, a subsequent step was conducted to induce antigen-antibody binding between the anion transport membrane protein and the magnetic nanoparticles by introducing the magnetic nanoparticles into the environment where the target cells were present. The binding of the anion transport membrane protein to the magnetic nanoparticles took approximately 1 hour to 1 day.

In step (4), a rotating magnetic field generator was used to generate a rotating magnetic field in the environment containing the target cells. The rotating magnetic field was a uniform concentric magnetic field (25 mT to 100 mT) rotating in either a clockwise or counterclockwise direction. This rotating magnetic field induced the rotation of the magnetic nanoparticles within the magnetic field region, thereby generating a rotational force. This rotational force was transmitted to the anion transport membrane protein bound to the magnetic nanoparticles, ultimately facilitating the influx of anions into the target cells, thereby suppressing the activity of the target neurons (Fig. 17).

[Discussion]

The left panel of Fig. 13 illustrates a schematic diagram of the formation of the DmFLYC1-Myc ion channel according to Example 1. The ion channel of the present invention can be expressed in cells by introducing a genetically modified sequence, which may be delivered into the cell via an adeno-associated virus (AAV) or lentivirus. In this case, the genetically modified sequence may include a DmFLYC1-Myc expression cassette.

The right panel of Fig. 13 presents an image confirming the formation of the FLYC ion channel and MYC protein tag according to Example 1. As shown in the right panel of Fig. 13, the protein tag in cells transfected with the modified gene sequence is expressed at the same location as the ion channel, verifying its co-localization and successful expression.

Fig. 14 presents an image of cultured HEK293 cells expressing the anion transport membrane protein (DmFLYC1) with an added antigenic nucleotide sequence designed to facilitate binding to the antibody moiety of magnetic nanoparticles. The cells were treated with a fluorescent antigen to visualize antigen-antibody binding. In Fig. 14, the FLYC ion channel (blue, corresponding to Fig. 13) is observed alongside the fluorescent protein RFP (red, Fig. 14). When the MYC protein tag (green, Fig. 14) was co-expressed with FLYC, MYC was detected at the same location as FLYC but showed no colocalization with intracellular RFP. This result confirms that the MYC tag is conjugated to the extracellular domain of FLYC, verifying its proper surface expression.

Fig. 15, which demonstrates the targeting of DmFLYC1 with magnetic nanoparticles (MNPs), shows that the MNPs (green, Fig. 15) are bound to the extracellularly exposed domain of the ion channel (blue, Fig. 15).

Fig. 16 illustrates the method for detecting $Cl^-$ influx using the MQAE quenching technique. It demonstrates that when DmFLYC1 is activated via magnetogenetics (magnetogenetic activation), intracellular $[Cl^-]_i$ accumulates. The left panel of Fig. 16 depicts the MQAE quenching technique used to confirm $Cl^-$ influx. The top portion of the left panel represents the reaction mechanism of MQAE binding to $Cl^-$, while the bottom portion conceptualizes the fluorescence quenching of MQAE when $Cl^-$ ions enter HEK293 cells expressing FLYC1 ion channels due to the rotation of magnetic nanoparticles. The right panel of Fig. 16 compares fluorescence intensity between HEK293 cells injected with MQAE, containing (1) ion channels without a protein tag (left graph) and (2) ion channels with a protein tag (right graph). The left graph indicates that in the absence of a protein tag, fluorescence intensity remains unchanged regardless of the presence of magnetic nanoparticles (MNPs) or rotating magnetic fields (RMFs). However, in the right graph, when MNPs (m-Torquer) and RMFs are present, the Fo/F ratio increases, confirming fluorescence quenching. This result indicates that the ion channel is opened, allowing $Cl^-$ influx into the cell.

Fig. 17 presents the results of membrane potential measurements in cells expressing DmFLYC1 ion channels after treatment with magnetic nanoparticles (m-Torquer) and exposure to a magnetic field over a specific period. Referring to the upper graph in Fig. 17, a decrease in membrane potential is observed upon the application of a magnetic field (indicated by the red arrow), suggesting that $Cl^-$ ions entered the cell upon ion channel opening. This result implies that the opening of the ion channel was induced by the rotation of the magnetic nanoparticles (m-Torquer) in response to the applied magnetic field. Conversely, in the lower graph of Fig. 17, where no magnetic field was applied, the periodic reduction in membrane potential is absent. This confirms that the decrease in membrane potential observed in the upper graph was specifically due to the magnetic field application.

In summary, when the ion channel is DmFLYC1 and the antigenic moiety facilitating binding to the antibody moiety of the magnetic nanoparticles is MYC-tag, the antibody moiety of the magnetic nanoparticle binds to the MYC-tag. Upon the application of a rotating magnetic field, the injected magnetic nanoparticles (m-Torquer) rotate, resulting in the opening of the DmFLYC1 ion channel. This channel opening allows $Cl^-$ ions to flow into the cell, leading to membrane hyperpolarization, which is consistent with the results presented in Fig. 17. Thus, this study demonstrates that cell activity can be modulated using the rotational force of m-Torquer conjugates.

**Example 2: Wireless cell-type specific magnetic control of neural activities in live animals**

**[Methods]**

Following the methodology outlined in Nature Materials (2022) (Nat Mater. 2021 Jul;20(7):1029-1036), the following components were prepared (1) m-Torquer, (2) m-Torquer conjugated with fluorescent particles and Myc antibodies,(3) Magnetic simulation, and (4) CMA (Controlled Magnetic Actuation).

## 2-1. Synthesis of m-Torquer

The m-Torquer was synthesized by conjugating azide-functionalized MNPs onto DBCO-functionalized supports as reported previously with slight modifications[17]. To synthesize the azide-functionalized magnetic nanoparticles (MNPs), MNPs were coated with silica as reported previously with little modification to ensure biocompatibility[46]. A mixture was prepared with 1 nmol MNPs, 78 g Igepal CO-520 (Sigma-Aldrich), 21 ml ammonium hydroxide (Sigma-Aldrich), and 200 $\mu$l tetraethylorthosilicate (Sigma-Aldrich) in 2500 ml cyclohexane. After 2 days, 2 $\mu$l aminopropyltrimethoxysilane (Sigma-Aldrich) was added and the solution was shaken at 200 r.p.m. for 2 hr at room temperature. Amine-functionalized MNPs were washed via centrifugation and redispersed in DMSO for further surface modification with tetrafluorophenyl (TFP) ester. To prepare azide-functionalized MNPs, 50 $\mu$mol azido-dPEG12-TFP ester (Quanta Biodesign) was reacted with 1 nmol amine-functionalized MNPs in dimethyl sulfoxide (DMSO) overnight. The azide-functionalized MNPs were purified via centrifugation and redispersed in DMSO.

To prepare DBCO-functionalized supports, 2.5 mmol DBCO-PEG5-TFP ester (Click Chemistry Tools) was reacted with 1 nmol amine-functionalized polystyrene microspheres (0.2 $\mu$m, Polyscience) overnight by copper-free click chemistry[47]. The DBCO-functionalized supports were purified via centrifugation and redispersed in DMSO. Then, to synthesize the m-Torquer, azide-functionalized MNPs, and DBCO-functionalized microspheres were mixed at a molar ratio of 2000:1. After 8 h, the m-Torquer was purified by centrifugation and redispersed in DMSO. For further functionalization, carboxylate groups were introduced by adding DBCO-PEG5-COOH (Click Chemistry Tools) and succinic anhydride (Sigma Aldrich) to the solution each at a final concentration of 1 mg·ml$^{-1}$. Carboxylated m-Torquer was purified via centrifugation and redispersed in 10 mM phosphate buffer (pH 7.2). The synthesized m-Torquer was characterized with an SEM using JSM-7900F with PC-SEM software.

## 2-2. Synthesis of m-Torquer conjugated with Myc antibodies

For Myc antibody surface functionalization, 100 $\mu$g protein A (Sigma Aldrich) was first conjugated onto 1 mg carboxylated m-Torquer via EDC/NHS coupling. After purification by centrifugation, 40 ug Myc antibody (Sigma Aldrich) was added to protein A conjugated m-Torquer. Myc antibody conjugated m-Torquer was purified by centrifugation and redispersed in 10 mM phosphate buffer (pH 7.2) at a final concentration of 50 mg·ml$^{-1}$.

## 2-3. Magnetic setup for *in vivo* MMG stimulation

For *in vivo* MMG stimulation of freely moving mice in a minimally restrained setup, the 60 cm (diameter) in-house-built magnetic arena was used. To build the 60 cm magnetic setup, ten 1T NdFeB magnets (12.5 $\times$ 15 $\times$ 12.5 cm) were assembled in a stainless stress cage. The 60 cm magnetic setup was mounted on an in-house-built motorized rotation stage and controlled by Arduino. A specialized arena designed for various animal behavioral assays, including feeding, social interactions, and motor function, was assembled and placed into the center of the magnetic arena. The magnetic field strength of the various configurations of magnetic setup was simulated by finite element analysis using COMSOL Multiphysics.

## 2-4. Plasmid and viral vector construction

For *in vivo* and *in vitro* magneto-mechanical-genetics (MMG) experiments, Ad-hSyn-FLEX-Myc897-Piezo1. Mouse Piezo1 with myc tag inserted at residue 897 (Myc897-Piezo1) was cloned in an antisense direction to create Ad-pEnt-hSyn-FLEX-Myc897-Piezo1. Myc897-Piezo1 was flanked by a pair of canonical loxP sites and a pair of adenoviral constructs were then sent to KOMABIOTECH Inc., for adenovirus packaging with serotype 5. Lenti viral particles of serotype 5 were produced by the Viral Core Facility of KOMABIOTECH Inc. Lentiviral particles were produced in-home using HEK293T cells and 3$^{rd}$ generation lentiviral packaging system. For transduction, in LH with Ad-hSyn-FLEX-Myc897-Piezo1 virus, it has been reported that the synapsin (Syn) promotor is neuron-specific with little glial expression.

## 2-5. Expression of Piezo1 in primary cortical neurons

Cortical tissues were dissected from C57BL/6 mice (Nara Biotech) on embryonic day 14 and placed in prechilled Hank's buffered salt solution (HBSS, Gibco). Then, the tissues were trypsinized for 15 min and physically dissociated into single neurons. The dissociated neurons ($10^5$ cells·cm$^2$) were plated on glass coverslips or confocal dishes coated with poly-D-lysine (Sigma-Aldrich) and laminin (Sigma-Aldrich), and incubated in neurobasal media (Gibco) containing 2% B27 supplement (Gibco), 0.5 mM L-glutamine (Gibco), 25 $\mu$M L-glutamate (Sigma-Aldrich) and 50 units per ml penicillin/streptomycin (Gibco) in 5% $CO_2$ at 37 °C. This experiment was carried out in accordance with the recommendations in the Guide for the Care and Use of Laboratory Animals of the Yonsei University Institutional Animal Care and Use Committee. The protocol was approved by the Committee on the Ethics of Animal Experiments of Yonsei University (approval number IACUC-A-201707-294-02). The recombinant adenovirus used for Myc897-Piezo1 expression was applied to cultured neurons on 4 DIV (days in vitro). The protocol for viral infection was approved by the Institutional Biosafety Committee of Yonsei University (approval number IBC-A-201706-146-01). After day five (9 DIV), 2 $\mu$l of m-Torquer (10 mg·ml$^{-1}$) was applied to the neurons.

### 2-6. HEK293 cell recording with the m-Torquer system

*Sample preparation:* For the recording of HEK293 cells, a Piezo1 expressing HEK293 cellular line was generated using a PB transposon system following the manufacturer's protocol with slight modification. Briefly, 0.5 $\mu$g of super PiggyBac transposase (SBI #PB210PA-1) and 2.5 $\mu$g of Myc897-Piezo1 encoding PB transposon vector were transfected to 1x10$^6$ of HEK293 cells in a 6-well plate using Lipofectamine. After three days, puromycin selection was done to remove non-integrated cells. The HEK293 cell culture of the cellular line was maintained in Dulbecco's modified Eagle's medium with 4.5g/L glucose (DMEM; Gibco) supplemented with 10% fetal bovine serum (FBS; Gibco) and 1% penicillin-streptomycin (Gibco). A day before recording, cells were transferred to Poly-D-Lysine coated cover glass and incubated overnight. Subsequently, cells were treated with 8 $\mu$l of m-Torquer (50 mg/ml) diluted to 1 mL with culture medium for m-Torquer labeling. After 1 hr incubation at 37 °C, cells were washed with complete medium to remove unbound m-Torquers. Then, the cover glass with cells was loaded on a nonmagnetic bath chamber and placed upon electrophysiology equipment with the MMG stimulator system.

*Patch-clamp equipment with MMG stimulator system:* Measurements were performed with a 700A Multiclamp Axon Amplifier and the signals were digitized with a Digidata 1440A using pClamp 11 software (all Molecular Devices). To apply MMG stimulation, we assembled 6 NdFeB magnets (2.5 $\times$ 3 x 4 cm) caged by a 3D printed holder, which arrangement was set by the simulation to generate a uniform magnetic field ($|B| \approx 25$ mT) at the center point of 3 cm apart. The assembled magnets were mounted above the electrophysiological recording space, and the motorized rotation of the magnet was controlled by customized Arduino (Namil Optical Instruments) connected with Digidata 1440A and thus synchronized with measurement. The assembled magnets were held by a separate pole apart from the faraday cage to avoid transmission of vibration generated by the rotation of magnets. The head stage was set apart 10 cm from the chamber loader to avoid noise and controlled by Motorized Micromanipulator (MM-500, RWD Life Science). The electrode holder (World Precision Instruments) was engaged with the head stage through a customized nonmagnetic holder.

*Electrophysiological recording of MMG-induced current:* For the recording of MMG-evoked currents, whole-cell patch-clamp recording was performed with voltage-clamped condition ($V_h$ = -60 mV). Cells were perfused in the bath solution with the following composition (in mM): 150 NaCl, 3 KCl, 10 HEPES, 5.5 glucose, 2 $MgCl_2$, 2 $CaCl_2$ with pH adjusted to 7.3 by NaOH (300 mOsmol/kg). Patch pipettes (5-8 M$\Omega$) were fabricated from borosilicate glass and filled with the inner solution with the following composition (in mM): 135 $CeMeSO_4$, 8 NaCl, 10 HEPES, 0.25 EGTA with pH adjusted to 7.3 by KOH (290 mOsmol/kg). All measurements were carried out at room temperature (23 °C). All data were reduced 100 times by data reduction of Clampfit software.

### 2-7. In situ calcium imaging in live primary neurons with the m-Torquer system

A solution containing 2 $\mu$l of X-Rhod-1 (5 mM, dispersed in 20% (w/v) Pluronic F-127 in DMSO, Thermo Fisher Scientific) was diluted in 1 ml cell culture medium and applied to primary neurons. Cells were incubated for 30 min at 37°C, 5% $CO_2$. A 2 $\mu$l volume of m-Torquer (10 mg·ml$^{-1}$) was diluted to 200 $\mu$l with culture medium and applied to primary neurons. After a 1 h incubation at 37 °C, unbound particles were removed and 1 ml of HBSS containing 2 mM calcium chloride was added. Then, the cell culture dish was placed under the microscope. The CMA was positioned in the middle of the in-house-built plastic sample stage with a live-cell chamber (37 °C, 5% $CO_2$). The fluorescence signal of X-Rhod-1 was recorded with an EMCCD camera while the rotating magnetic field was applied (imaging rate: 20 frames per second).

### 2-8. Animals

All experiments were approved by the Yonsei University Institutional Animal Care and Use Committee (IACUC) and all procedures involving the handling of animals were in accordance with the National Institutes of Health guidelines. Male mice (>6 weeks old) were used for all behavioral and molecular studies. Mice were maintained in a temperature-controlled room (22 $\pm$ 1°C) on a 12 hr light-dark cycle with *ad libitum* access to food and water, and specific pathogen-free conditions. All WT C57/BL6 mice were acquired from Orient Bio and Central Lab. Animal Inc. Vgat::IRES-Cre: Slc32a1tm2(cre)Lowl/MwarJ and Vglut2::IRES-Cre: Slc17a6 m2(cre)Lowl/J mice[48,49] were acquired from Jackson Laboratory. All Cre mouse lines are in a wild-type (C57BL/6J) background.

## 2-9. Genotyping

DNA isolated from the tail biopsies was used for mouse genotyping of the Vgat::IRES-Cre or Vglut2::IRES-Cre gene. The DNA extraction process from tissue followed the MyTaq Extract-PCR Kit (Meridian Bioscience). The presence of the Vgat::IRES-Cre allele was verified by PCR amplification using the WT-Vgat and KI-Vgat primer sets. Conditions of PCR were as follows: denaturation steps at 95 °C for 3 min, followed by 35 cycles at 95 °C for 15 s, 60.5 °C for 15 s, and an elongation step at 72 °C for 20 s. The genotyping primers used for the RT-PCR assays are as follows: WT-Vgat primers, Forward 5'-CTTCGTCATCGGCGGCATCTG-3'; Reverse 5'-CAGGGCGATGTGGAATAGAAA-3'. KI-Vgat primers, Forward 5'-CACCCTGTTACGTATAGCCG-3'; Reverse 5'-GAGTCAT CCTTAGCGCCGTA-3'. The presence of the Vglut2::IRES-Cre allele was verified by PCR amplification using the WT-Vglut2 and KI-Vglut2 primer sets. Conditions of PCR were as follows: denaturation step at 95 °C for 3 min, followed by 35 cycles at 95 °C for 15 s, 60 °C for 15 s, and an elongation step at 72 °C for 20 s. The genotyping primers used for the RT-PCR assays are as follows: WT-Vglut2 primers, Forward 5'-AAGAAGGTGCGCAAGACG-3'; Reverse 5'- CTG CCACAG ATTGCA CTTGA-3'; KI-Vglut2 primers, Forward 5'- AAGAAGGTGCGCAAGACG-3', Reverse 5'- ACACCG GCCTTATTCCAAG-3'.

## 2-10. Surgical procedures

*General:* All surgeries were performed on mice under aseptic conditions and body temperature was maintained with a heating pad. Mice were anesthetized intraperitoneally with a ketamine and xylazine solution (100 mg·kg$^{-1}$ of ketamine and 10 mg·kg$^{-1}$ of xylazine), and placed into a stereotaxic frame (David Kopf Instruments, Tujunga, CA, USA). All measurements were made relative to bregma for virus and m-Torquer surgeries. Corneas were protected from drying using eye gel (Puralube Vet Ointment, Dechra). After cleaning the periosteum, the vertical coordinates of bregma and lambda were measured to align in the same plane (level head). A craniotomy (~1 mm in diameter) was made above the injection site. The viral injection was performed using a beveled 33-gauge microinjection needle with a 10 µl microsyringe (UMP3; WPI, Sarasota, FL, USA), delivering the virus at a rate of 100 nl·min$^{-1}$ using a microsyringe pump and a controller (Micro4; WPI, Sarasota, FL, USA). After injection, the needle was left in place for 5 min to pass before withdrawing the needle 50-100 µm and leaving it for an additional 10 min to allow diffusion of the viral solution into the brain tissues before the needle was slowly withdrawn completely. After surgery, mice recovered from anesthesia under a heating pad. Animal experiments were carried out in accordance with the recommendations in the Guide for the Care and Use of Laboratory Animals of the Yonsei University Institutional Animal Care and Use Committee. The protocol was approved by the Committee on the Ethics of Animal Experiments of Yonsei University (approval number IACUC-A-202107-1299-05) and by the Institutional Biosafety Committee of Yonsei University (approval number IBC-A-202108-286-01).

*Viral injection for Cre-dependent Piezo1 expression:* For Myc897-Piezo1 expression, all experimental mice underwent stereotaxic injections at the age of 8 weeks. For cell-type specific MMG stimulation targeting lateral hypothalamus (LH), Vgat::IRES-Cre or Vglut2::IRES-Cre mice were injected bilaterally with 1.0 µl adenovirus serotype 5 (AV5) encoding Myc897-Piezo1 under a double-floxed inverted open-reading frame construct (AV-hSyn-FLEX-Myc897-Piezo1) (1 x 10$^{12}$ VP (viral particles)/ml, KOMABIOTECH Inc.) For the control group without Piezo1 expression, a null version of the virus only carrying Myc897-Piezo1 without FLEX (AV-hSyn-Myc897-Piezo1) was injected into the LH using the following coordinate relative to lambda: AP (anteroposterior), -1.4; ML (mediolateral), ±0.9; DV (dorsoventral), -5.35.

*M-Torquer introduction:* 3 weeks after viral injection, Vgat::IRES-Cre or Vglut2::IRES-Cre mice were injected bilaterally with M-torquer (50 mg·ml$^{-1}$) 1.0 µL at the following coordinate relative to lambda: AP (anteroposterior), -1.4; ML (mediolateral), ±0.9; DV (dorsoventral), -5.35. M-Torquer injection was performed using a beveled 33-gauge microinjection needle with 10 µl microsyringe (UMP3; WPI, Sarasota, FL, USA), delivering virus at a rate of 100 nl·min$^{-1}$ using a microsyringe pump and a controller (Micro4; WPI, Sarasota, FL, USA). After injection, the needle was left in place for 5 min to pass before withdrawing the needle 50-100 µm and leaving it for an additional 10 min to allow diffusion of the m-Torquer into the brain tissues before the needle was slowly withdrawn completely. After surgery, mice recovered from anesthesia under a heating pad.

## 2-11. *In vivo* MMG stimulation

Mice were anesthetized as previously described. Using the LH coordinate, 1 µl of AV-hSyn-FLEX-Myc897-Piezo1 (titer) was injected bilaterally into the LH of 8 week old mice. For Piezo1(-) controls, the same volume of saline was injected instead of adenovirus. Three weeks later, 2.5 µl of 100 mg ml m-Torquers were injected into the same LH area under isoflurane anesthesia. For m-Torquer (-) controls, the same volume of saline was injected instead of nanoparticles. After 2-3 days of recovery, the mice were placed within the 60 cm MMG apparatus and magnetic arena. Mice received a uniform magnetic field, field gradient ∇B < 10 T/m, and a magnitude of field |B| ≥ 20 mT for 30 min while allowed to freely roam around and behave naturally in all behavioral assays used in this study, including open chamber feeding assay, long-term MMG stimulation, and social interaction tests. *In vivo* MMG stimulation experiment was approved by the Committee on the Ethics of Animal Experiments of Yonsei University (approval number IACUC-A-202107-1299-05) and by the Institutional Biosafety Committee of Yonsei University (approval number IBC-A-202108-286-01).

## 2-12. Behavioral tests: Open chamber, free-access feeding task

Animals were placed in a custom circular, single-chamber (300 mm x 250 mm) arena with four food traps to quantify the amount of food consumed and assess the time spent in a designated food zone arena. The arena contained four accessible food traps. For short-term MMG experiments, pellets of standard mouse chow were placed into one of the chambers (designated food zone) while the other three traps remained empty. Mice were allowed to freely explore for 1 min and then received 30 min of continuous magnetic stimulation during which they were allowed to freely move and explore around the arena. To test on-demand, reversible activation of feeding, well-fed mice were monitored for 10 min pre-stimulation, 10 min magnetic stimulation, and 10 min post-stimulation. Their spatial locations, time spent at the food zone, and velocity were recorded via a CCD camera at 15 fps interfaced with EthoVision XT 10 (Noldus, Leesburg, VA) for individual mice in a semi-dark condition. All behavioral tests were performed under low light conditions, and animals were allowed to acclimate to the behavior room for a least 1 hr before the beginning of behavioral testing. Mice were habituated at least 3 days before with magnetic field sounds without stimulation for 30 min.

## 2-13. Behavioral tests: Long-term MMG stimulation for diet-induced obese mice

For the diet-induced obese (DIO) mouse study, Vgat::IRES-Cre or Vglut2::IRES-Cre male mice at the age of 6 weeks were placed on a 60 kcal % high-fat diet (HFD) for 8 weeks. Vgat- or Vglut2- DIO mice on HFD were stereotaxically injected with Ad-hSyn-FLEX-Myc897-Piezo1. After 3 weeks to induce Piezo1 expression, a second injection delivered m-Torquer (1 $\mu$l, 50 mg·ml$^{-1}$) using the same coordinate of the LH. Food intake and weight change of Vgat- or Vglut2-DIO mice were measured while applying magnetic field stimulation for 1 hr every day for 10-14 days. Change in body weight over time was normalized as a percentage of the day 1 initial starting weight for each animal. Fat mass from inguinal and gonadal white adipose tissues were measured from the mice after 10 days of MMG stimulation.

## 2-14. Behavioral tests: Three-chamber sociability and social novelty

Social interaction behaviors modulated by MMG stimulation were examined using the three-chambered social interaction assay as previously described[50] with some modifications for MMG stimulation. The three-chambered apparatus, designed to assess the animal's preference for a social stimulus over a non-social stimulus and measure social novelty, had the dimension of (w) 510 × (D) 300 × (H) 250 mm) divided into 3 equal compartments ((w) 170 × (D) 300 × (H) 250 mm) by plastic dividers with a 2-inch opening in the middle. Vgat::IRES-Cre mice injected with Ad-hSyn-FLEX-Myc897-Piezo1 were assigned into one of the two groups: MMG group with m-Torquer (1 $\mu$l, 50 mg·ml$^{-1}$) injected into the LH$_A$ and the control group without m-Torquer. The experimental mice were habituated to the arena for 5 min in the middle chamber without access to the side chambers. After all chambers were open, the mice were allowed to freely explore the entire apparatus for 5 min before a new mouse and object were placed into the arena. Only the mice which were not biased to either side were tested further for sociality and social novelty. A juvenile male (4-5 weeks of age, Vgat::IRES-Cre) mouse was placed into one of the chambers, and a 5 min recording session, during which mice were MMG stimulated (0.5 Hz), was initiated. A video camera was positioned above the magnetic arena recorded each trial, and mouse location and velocity were tracked. The videos, recorded at 15 fps, were analyzed using Ethovision XT software. For both control and MMG groups, the total amount of time spent investigating a novel mouse during magnetic field (MF) ON epochs was analyzed.

## 2-15. Behavioral tests: Multiple animal social interaction test

Social interaction test involving multiple animals in the same physical area was conducted using the same three-chambered apparatus previously described. For paired-mice social-interaction experiments, Vgat::IRES-Cre male mice born in the litter were bred in a cage and kept in a laminar airflow cabinet maintained at 22 ± 1 °C on a 12 hr light-dark cycle with *ad libitum* access to food and water, and specific pathogen-free conditions. Two Vgat::IRES-Cre mice injected with Ad-hSyn-FLEX-Myc897-Piezo1 were assigned into one of the two groups: MMG group with m-Torquer (1 $\mu$l, 50 mg·ml$^{-1}$) injected into the LH$_A$, and the control group without m-Torquer. The experimental mice were habituated to the arena for 5 min in the middle chamber without access to the side chambers. After all chambers were open, the mice were allowed to freely explore the entire apparatus for 5 min before a new mouse and object were placed into the arena. Only the mice which were not biased to either side were tested further for sociality and social novelty. A juvenile male (4-5 weeks of age, Vgat::IRES-Cre) mouse was placed into one of the chambers, and a 3 min recording session, during which mice were MMG stimulated (0.5 Hz), was initiated. A video camera was positioned above the magnetic arena recorded each trial, and mice location and velocity were tracked. The videos, recorded at 15 fps, were analyzed using Ethovision XT software. For both control and MMG groups, the total amount of time spent investigating a novel mouse during magnetic field (MF) ON epochs was analyzed.

## 2-16. Analysis of animal behavioral tests

A python program was manually developed to analyze behavioral tests (code is available at https://github.com/DHSHINN/Magnetogenetics). Raw data were collected by a CCD camera interfaced with EthoVision XT 10 (Noldus, Leesburg, VA) and primarily processed using Microsoft Excel to give out coordinates of mouse movement per time frame, and then was subsequently processed with Seaborn 0.11.0, Matplotlib 3.5.1, and Pandas 1.4.1 to generate heatmaps to visualize and track time spent on each region of the stage. Python code was modified for each unique dataset to normalize and process the coordinates to produce a heatmap and calculate the time spent on a defined area for each trial.

## 2-17. Immunohistochemistry

For immunohistochemical analysis, animals were perfused with phosphate-buffered saline (PBS, pH 7.4) followed by 4% paraformaldehyde in PBS for 1.5 hr after being exposed to a rotating magnetic field (0.5 Hz). Then, the brains were removed and post-fixed in the same fixative for 24 h at 4 °C, frozen and sectioned coronally at 40 $\mu$m thickness using a sliding microtome (Leica Microsystems GmbH, Germany) and stored in cryoprotectant at 4 °C. Brain slices were blocked for 1 hr with 3% bovine serum albumin (BSA) in PBS containing 0.1% Triton X-100 and 5% normal goat serum to prevent nonspecific binding. The subsequent immunostaining was performed with primary antibodies in PBS containing 5% normal goat serum overnight at 4 °C (anti-Myc (Cell Signaling Technology, Inc), 1:300; anti-c-Fos (Cell Signaling Technology, Inc), 1:200; anti-VGAT (Invitrogen), 1:200; anti-Vglut2 (Sigma-Aldrich), 1:200; anti-GFAP (DAKO), 1:200; anti-Iba1 (Wako chemicals, Inc), 1:200; and anti-NeuN (Abcam), 1:200). On the following day, tissues were washed three times with PBS, appropriate secondary antibodies (anti-rabbit IgG (Alexa Fluor 488, Abcam, polyclonal goat antibody), 1:200; anti-mouse IgG (Alexa Fluor 488, Abcam, polyclonal goat antibody), 1:200; anti-rabbit IgG (Alexa Fluor 647, Abcam, polyclonal goat antibody), 1:200; anti-rabbit IgG (Alexa Fluor 647, Abcam, polyclonal donkey antibody), 1:200) were applied for 4 hr at room temperature and stained with 4',6-Diamidino-2-phenylindole dihydrochloride (DAPI) (Invitrogen). Subsequently, sections were washed three times with PBS and mounted on a Crystal Mount™ Aqueous Mounting Medium (Sigma-Aldrich). A confocal microscope (Leica, Germany) was used to observe fluorescence using LasX software. All imaging parameters were constant across all samples. Z-stack and tiled images of mounted brain sections were captured with a 10x, 25x, and 63x objective. For c-Fos quantification, Fiji or ImageJ with thresholding and segmentation functions was used to quantify the number of c-Fos-labeled neurons among all DAPI-stained neurons within the LH. For Piezo1 colocalization with Vgat or Vglut2 neurons, the number of Piezo1-, Vgat-, and Vglut2-expressing neurons were counted and quantified using a similar analysis.

## 2-18. *In vitro* 3D human brain phantom experiment

HEK293 cell culture was maintained in Dulbecco's modified Eagle's medium with 4.5g/L glucose (DMEM; Gibco) supplemented with 10% fetal bovine serum (FBS; Gibco) and 1% penicillin-streptomycin (Gibco). For MMA stimulation in a 3D human brain-like phantom, HEK293 cells were co-transfected with pcDNA3.1-pCMV-FLEX-Myc897-Piezo1 and pCMV-Cre (Addgene #123133) expression plasmids using 10 ul lipofectamine 3000 with 5000 ng of total DNA in Opti-MEM medium (Gibco). After 2 days of recovery and Piezo1 expression, the cells were treated with 8 $\mu$l of m-Torquer (50 mg/ml) diluted to 1 ml with culture medium for m-Torquer labeling. After a 3 hr incubation at 37 °C, cells were washed with complete medium to remove unbound m-Torquers. A total of $5 \times 10^5$ cells were transferred to and embedded in the Matrigel matrix (size = 1.8 cm x 1.8 cm x 1.8 cm, volume = 120 ul). MMG stimulation was provided for 30 min with 0.5 Hz frequency using the MMG-60 apparatus. The samples were analyzed using RT-PCR for *c-fos, Piezol, Cre,* and *β-actin* mRNA expression.

**RT-PCR:** Total RNA was extracted from cells using the RNAeasy Mini Kit (QIAGEN, Hilden, Germany). cDNAs were synthesized using 3 $\mu$g of total RNA, oligo dT (10 pmol), and SuperScript reverse transcriptase III (200 units/$\mu$l) in a total reaction volume of 20 $\mu$l. The primer sequences used for *c-fos, Piezol, Cre, and β-actin.* The primers used for the RT-PCR assays are as follows: *c-fos* primers, Forward 5'-CAAGCGGAGACAGACCAACT-3'; Reverse 5'-AGTCAG ATCAAGGGAAGCCA-3'; *Piezo1* primers, Forward 5'-TTCTTCGGGTTGGAGAGGTA-3', Reverse 5'-TGTCACCATGT GGTTAAGGATG-3; *Cre* primers, Forward 5'-GCCTGCATTACCGGTCGA TGCAAC-3', Reverse 5'-CGTATATCCTGG CAGCGATCGC-3; *β-actin* primers, Forward 5'-GCACCACACCTTCT ACAATG-3', Reverse 5'-TGCTTGCTGATCCAC ATCTG-3.

**IVIS optical imaging:** HEK293 cells were co-transfected with pCMV-Luc and pcDNA3.1-pCMV-Myc897-Piezo1 expression plasmids using 10 $\mu$l Lipofectamine 3000 with 5000 ng of total DNA in Opti-MEM medium (Gibco). After 2 days of recovery and Piezo1 expression, the cells were treated with 8 $\mu$l of m-Torquer (50 mg/ml) diluted to 1 ml with culture medium for m-Torquer labeling. After a 3-hr incubation at 37 °C, cells were washed with complete medium to remove unbound m-Torquers. A total of $5 \times 10^5$ cells were transferred to and embedded in the Matrigel matrix (size = 1.8 cm x 1.8 cm x 1.8 cm, volume = 120 ul). MMG stimulation was provided for 30 min with 0.5 Hz frequency using the MMG-60 apparatus. Cells embedded in the Matrigel were treated with 150 $\mu$g/ml D-luciferin diluted in media for 30 min. Chemi-luminescence signals from luciferase expression were captured using the IVIS Lumina III In Vivo Imaging System (PerkinElmer). Total radiant efficiency of the samples was measured using Living Image software 4.7.4 (PerkinElmer).

*Luciferase reporter assay:* For luciferase assay, $Ca^{2+}$ dependent luciferase reporter-expressing HEK293 cell line was generated using lentiviral transduction. Briefly, lentivirus for CSN-mCMV-eGFP-2A-FLuc were applied to $5 \times 10^4$ of HEK293 cells cultured in a 6-well plate. Luciferase reporter-expressing HEK293 cells were co-transfected with pcDNA3.1-pCMV-FLEX-Myc897-Piezo1 (2000 ng) and pCMV-Cre (Addgene #123133, 4000 ng) expression plasmids using 10 $\mu$l Lipofectamine 3000 in Opti-MEM medium (Gibco). After 2 days of recovery and Piezo1 expression, cells were treated with 8 $\mu$l of m-Torquer (50 mg/ml) diluted to 1 ml with culture medium for m-Torquer labeling. In vitro MMG stimulation was performed using a 35-cm MMG apparatus with 0.5 Hz for 24 hrs. Then, cells were washed with complete medium to remove unbound m-Torquers and analyzed for luciferase activity using a EnSight Multimode Microplate Reader (PerkinElmer). All reactions were performed in triplicate. Reporter activity was normalized to total protein amount to determine transfection efficiency.

### 2-19. Statistical analyses

Statistical analysis was performed using GraphPad Prism 9.3.1 software (GraphPad) or Microsoft Excel.

**[Results ]**

### 1. Cell-type specific MMG neuromodulation in freely behaving animals

For the MMG stimulation of the target neurons in freely behaving animals the MMG apparatus was constructed as follows: a rotational magnetic force generator (MFG), a CMOS camera for behavior monitoring (behavior CAM), and an Arduino controller (Fig 20a, Fig. 24a). MFGs with different sizes can be constructed depending on applications: 16-cm for *in vitro* (cells), 35-cm for a single mouse, and 60-cm for multiple mice and a large animal **(Fig 20a, Fig. 24b).** The 60-cm MFG has a magnetic arena of 60 cm in diameter and 20 cm in height with NdFeB magnets for a uniform magnetic field, $|B| \approx$ 20 mT and field gradient, $\nabla B < 10$ Tm$^{-1}$, rotating at slow speed (0.5 Hz) (*c.f,* 100 kHz for MTG setup) **(Fig 20a).** The nanomagnetic torque generator (m-Torquer) is a 200 nm magnetic nanoparticle for torque generation under a rotating magnetic field **(Fig. 24c).** When the rotating magnetic field is ON, MFG generates ~2 pN of force over the entire magnetic arena to actuate Piezo1 channel for calcium entry and subsequently neural excitation **(Fig 20a).**

To examine whether Piezo1 ion channel gating by MMG stimulation induces the changes in membrane potential and current, Cre-dependent Piezo1-expressing HEK293 (Piezo1+) cells were electrophysiologically recorded under whole-cell, voltage-clamped mode at -60 mV. A novel patch-clamp system capable of synchronized electrophysiological recording under a uniform, rotating magnetic field ($|B| \approx$ 25 mT, VB < 10 Tm$^{-1}$) was constructed **(Fig** 20b). Application of a rotating magnetic field (0.5 Hz, 1 sec) induced large and rapid depolarizing currents in Piezo1+ cells, which reached a maximum rise rate of 128 $\pm$ 28 pA/ms (mean $\pm$ s.d., n = 9, **Fig** 20c). Mean whole-cell inward currents were significantly larger in Piezo1+ cells ($|I_{max}|$ = 516 $\pm$ 83 pA) than in controls transfected with empty vector, which showed negligible responses ($|I_{max}|$ = 35 $\pm$ 7 pA) **(Fig** 20c, **inset).** Both electrophysiology and imaging of $Ca^{2+}$ influx using X-Rhod-1 showed a response latency of ~100 ms, which is within the range of Piezo1 gating kinetics[29,30] but slower than the kinetics of opsins[2] **(Fig** 20c, **Fig. 25).** The force transfer from m-Torquer to the channel is direct and thus immediate, resulting in relatively faster kinetics than the other magnetogenetics approaches including MTG where the channel gating is mediated by indirect heat diffusion through space. In addition, a train of magnetic pulses (0.5 Hz, 6 pulses, with each pulse of 1-sec duration, and with an interval of 9-sec) elicited a series of current spikes with reliable timing and amplitude in Piezo1+ cells while the controls do not show any current **(Fig** 20d). Repeated stimulations induced currents with variable amplitudes, consistent with a previous report[31] that repetitive mechanical stimulation of Piezo1 in HEK293 cells invokes current peaks with varying amplitudes.

To achieve neuron-specific MMG stimulation *in vivo,* two Cre-dependent mouse lines, Cre-vesicular $\gamma$-amino-butyric acid transporter (Vgat) and Cre-vesicular glutamate transporter 2 (Vglut2) with selective expression of mechanosensitive Piezo1 channels were established. Vgat and Vglut2 are the promoters specifically expressed in GABAergic and glutamatergic neurons, respectively, which have distinct physiological roles in regulating neural networks. Mechanosensitive Piezo1 ion channel modified with a Myc tag for labeling of m-Torquers was packaged into the adenovirus for Cre-dependent neuron-specific expression (Ad-hSyn-FLEX-Myc897-Piezo1)**(Fig 20e**). The adenovirus was stereotaxically delivered to the target brain region of Cre mice to selectively express Piezo1 in GABAergic or glutamatergic neurons, respectively.

Neural cell-type specific expression of Piezo1 was confirmed by strong co-localization fluorescence signals of Piezo1 (green) and Vgat or Vglut2 (red) **(Fig 20 f, g**). The majority of the Piezo1-expressing neurons were Vgat- (93%) and Vglut2-immunopositive (90%), indicating robust and selective neuron-type specific expression of Piezo1 **(Fig** 20**h**). In addition, Piezo1 signals were primarily detected in the lateral hypothalamus (LH) region **(Fig. 26a)** and primarily localized to the cell membrane **(Fig. 26b),** indicating region-specific and membrane-specific expression. The m-Torquers labeled these Piezo1-expressing neurons, showing fluorescence signals of m-Torquers (red) were co-localized with Piezo1 (green) **(Fig** 20**i**).

## 2. **MMG stimulation of lateral hypothalamus neurons for bidirectional modulation of feeding**

After expressing Piezo1 followed by m-Torquer introduction into the brain, *in vivo* neuromodulation with m-Torquer-based MMG was demonstrated by stimulating two distinct neuronal populations, i.e., GABAergic and glutamatergic neurons in the LH. Located in the deep brain, the LH contains the GABAergic and glutamatergic neurons with complementary functions that together form neural circuits regulating complex behaviors including feeding and reward[34-38]. Adenovirus for Cre-dependent neuron-specific Piezo1 expression (hSyn-FLEX-Myc897-Piezo1) was stereotaxically injected into the LH in both hemispheres (X= $\pm$0.9 mm; Y= -1.4 mm; Z= -5.35 mm) of either Cre-Vgat or -Vglut2 mice. Three weeks after the Piezo1 expression, m-Torquers were injected at the same location using the defined injection condition (1.0 $\mu$l at 100 nl·min[-1] rate) **(Fig 21a, left).** These mice were habituated in a MMG setup for 2-3 days and stimulated by the rotating magnetic field with varying periods. Neuronal activation by Yoda1 (an agonist for Piezo1) or MMG stimulation was examined by histological analysis of c-Fos, a marker of neural activity **(Fig. 26c, d**). MMG stimulation triggered neural activity, confirmed by increased c-Fos expression (red) in the LH **(Fig 21b, Fig. 27a, b**). A significantly higher proportion of c-Fos-positive cells was observed in both Vgat-Piezo1 (27 %) **(Fig 21b, c**) and Vglut2-Piezo1 (17%) **(Fig. 27a**, **b**) mice than in controls without m-Torquer (0.12% in Vgat-Piezo1, 0.14% in Vglut2-Piezo1).

We chose, as a first example of complex animal behaviors, the bidirectional control of mouse feeding behaviors by cell-type specific MMG neuromodulation. We conducted the real-time food intake assay with a designated food zone in a MMG setup where the mouse was placed and monitored for feeding during 30 min MMG stimulation **(Fig 21a, right).** The heatmaps of mice trajectories show that the MMG stimulation elicited acute feeding behavior in Vgat-Piezo1 mice with m-Torquer (Vgat-MMG mice) but not in controls (Vgat-Piezo1 mice without m-Torquer) **(Fig 21d).** Food intake duration of Vgat-MMG mice increased significantly (20.4 min) in response to a magnetic field. It is >2-fold increase compared to control groups without m-Torquers (8.5 min), Piezo1 (10.5 min), and both (8.8 min) **(Fig 21e).** Vgat-MMG mice exhibited diminished locomotion by ~50% compared with control groups, possibly due to increased staying time at the designated food zone **(Fig 21f).** This feeding behavior by magnetic neuromodulation was also reversible. A 30 min home cage feeding task, comprised of three 10 min epochs (pre-stimulation, stimulation, and post-stimulation) was conducted, and the mice displayed significantly increased food intake during the stimulation (6.2 min) and then ceased food intake after the stimulation (2.3 min), returning to the level of pre-stimulation (3.0 min) **(Fig 21g**, **h**).

After confirmation of the increased feeding behavior of MMG-stimulated Vgat mice, we tested the Vglut2 mice in the same experimental condition. Stimulations of Vgat and Vglut2 are known to be bidirectional in neural circuitry toward animal behaviors, and the excitation of Vglut2 should deactivate the feeding. After m-Torquer delivery into Vglut2-Piezo1 mice to create Vglut2-MMG mice, the mice were allowed to feed naturally and performed a real-time feeding assay during magnetic stimulation for 30 min. In response to a magnetic field, Vglut2-MMG mice showed an acute response: the feeding behavior ceased, and the mouse moved away from the food zone, as shown in the heatmaps **(Fig 21i).** Overall food intake time of Vglut2-MMG mice was decreased significantly in response to a magnetic field (5.4 min), compared to control groups without m-Torquers (10.6 min), Piezo1 (9.8 min), and both (11.2 min) **(Fig 21j).** The locomotion of mice, shown as mean velocity, did not vary much between the stimulated and the control groups, indicating that the reduced feeding did not affect locomotion **(Fig 21k).** Based on a 30-min feeding task, Vglut2 neuron-specific stimulation was also reversible as Vglut2-MMG mice exhibited reduced feeding behavior during the magnetic field ON (2.0 min) while turning the magnetic field OFF promoted feeding behavior (3.5 min), returning to the level of pre-stimulation (3.7 min) **(Fig 21l**, **m**). These data support the findings that excitatory and inhibitory neuronal populations in the LH produce opposite effects in the regulation of mice feeding [34,36,39]. In sum, our results confirm our MMG system is a reliable and important addition to the repertoire of neuromodulation for examining the roles of different neuronal cell types or circuits.

## 3. **Application of MMG for long-term obesity modulation)**

While long-term, chronic brain stimulation is critical for modeling behaviors and diseases, current methods show challenges due to the physical tethers and thereby restriction in animal behaviors [40]. We applied long-term neuromodulation using MMG in the complex syndrome of obesity to interrogate the effects of prolonged modulation of mice feeding behaviors. Vgat- or Vglut2-Cre mice were maintained in an 8-week high-fat diet (HFD) to generate a mouse model of diet-induced obesity (DIO) and allowed for Cre-dependent Piezo1 expression to create either Vglut2-Piezo1 or Vgat-Piezo1 lines **(Fig 22a).** After m-Torquer delivery at week 11, the mice were treated with daily 1 hr, 0.5 Hz MMG stimulation in a magnetic arena with access to food for 2 weeks, and their body weight and food intake were monitored **(Fig 22a).** In the case of HFD Vglut2-Piezo1 mice, after the session, the results showed substantial weight loss and alleviation of obesity, while controls did not **(Fig 22b**, **c**). Gradual weight reductions on a daily basis were observed (**Fig 22c**), resulting in a significant body-weight reduction by ~4.3 g on average (from 49.2 g on day 1 to 44.9 g on day 10), which is equivalent to 10 % body weight loss, while the control groups without m-Torquers, Piezo1, or both maintained obesity albeit MMG stimulation (**Fig 22d**). The total amount of food intake was also significantly decreased in MMG stimulated Vglut2 mice (by 25.2 g) compared to the control groups without m-Torquers (50.8 g), Piezo1 (48.2 g), and both (53.5 g) (**Fig. 28a**). Anatomical examinations showed reduced body fat: the sizes of inguinal and gonadal white adipose tissue (iWAT and gWAT) were reduced compared to the control groups (by 47% and 21% for iWAT and gWAT, respectively) (**Fig 22e, Fig. 28c**).

In the case of HFD Vgat-Piezo1 mice, on the contrary, substantial weight gains and aggravation of obesity were observed after two weeks of stimulation **(Fig 22f, g)**. Body weight was increased by 7.5 g on average (from 39.2 g on day 1 to 46.8 g on day 10), being equivalent to 18 % body weight gain compared to the controls **(Fig 22g, h**). Total food intake was also increased considerably (40 g), compared to control groups without m-Torquers (25.4 g), Piezo1 (25.1 g), and both (27.4 g) **(Fig. 28b),** and both iWAT and gWAT were noticeably enlarged compared to the control groups (120% and 61% enlargement, respectively) **(Fig 22i, Fig. 28d).** Histological analysis showed negligible expressions of GFAP and Iba1 (markers for tissue inflammation), and a normal level of NeuN (a marker for neurons), suggesting long-term MMG stimulation did not induce any cytotoxicity or inflammatory responses **(Fig. 29a-c).** Overall, these results show that long-term, neuron-specific neuromodulation is possible by MMG, controlling the animal behaviors bidirectionally. These results imply the therapeutic potential for alleviating physiological disorders while allowing animals to behave freely during stimulation without implanted devices otherwise needed in opto- and electro-genetics.

## 4. **Wireless control of social behavior in multiple animals)**

With capabilities for wireless and large-area neuromodulation in freely moving animals, this MMG technology can be used for examining the circuitry that underlies complex social behaviors [6,41-43]. The three-chamber cage assay for assessing sociality and social novelty was conducted with the MMG setup **(Fig 23a).** The stimulation of GABAergic neurons in the lateral hypothalamus area (LH$_A$) projects inhibitory inputs to VTA GABAergic neurons, which in turns reinforces/promotes social interaction **(Fig 23a).** Adenovirus of hSyn-FLEX-myc897-Piezo1 was injected into the LH$_A$ region (X= $\pm$0.9 mm; Y= -1.4 mm; Z= -5.35 mm) of Vgat-Cre mice, and subsequently m-Torquer for MMG stimulation. To assess sociality, the first novel mouse (M1; male, 4-5 weeks juvenile) was placed into one of the chambers the in MMG setup where the test mouse (Vgat-MMG) was habituated. Then, during the magnetic stimulation (0.5 Hz rotating magnetic field, 5 min), the time spent by the Vgat-Piezo1 mouse engaging in an empty chamber or M1 was measured **(Fig 23b).** The heatmap analyses show Vgat-MMG mice spent significantly more time (red, ~200 s) interacting with M1 than controls (Vgat-Piezo1 mice without m-Torquer; ~100 s), while spending less time investigating the empty chamber (~31 s) than control (~69 s), reflecting increased sociality by LH Vgat neuron stimulation **(Fig 23c**, **d**). Then, the test of social novelty was performed by placing the second stranger mouse (M2) into another chamber, and the interaction of Vgat-MMG mice with M1 (a familiar mouse) and M2 (a novel mouse, social novelty) was measured **(Fig 23e).** The heatmap results show Vgat-Piezo1 mice exhibited an increased preference for social novelty, spending significantly more time in the M2 chamber (red, ~199 s) than control mice without m-Torquer (~112 s) **(Fig 23f**, **g**).

Using MMG capable of non-tethered animal behavioral testing in a large magnetic arena, social interactions of multiple individual animals could be simultaneously modulated within the same physical space [6,44]. Two Vgat-MMG mice were placed in the abovementioned three-chamber arena, and they were simultaneously stimulated and monitored for their social behaviors in response to a magnetic field for 3 min **(Fig 23h-k).** Interestingly, in the sociality assay, the mice trajectory shows that both Vgat-MMG mice showed an increased preference for interaction with a novel mouse (M1) in a synchronized fashion, while controls (Vgat-Piezo1 without m-Torquers) exhibited more random activity without preference for a novel mouse **(Fig 23h**, **i**). Similarly, in the social novelty test where another novel mouse (M2) was introduced to the chamber, both Vgat-MMG mice tended concurrently again to spend more time with M2 than M1, while controls showed no such social novelty preference **(Fig 23j**, **k**). Altogether, these results clearly support that regulating LH$_A$-VTA inhibitory neurons promotes social interactions in mice [45].

## 5. **Remote cell-type specific ion channel gating in a pseudo-human brain**

The long-working distance of the MMG system could enable deep brain stimulation of larger animal models such as primates in a non-contact manner. However, experiments with live primates are currently not available due to the cost, regulation, and ethical issues. As a proof-of-concept study for reliable MMG application in larger animals, we created a large-scale brain phantom (size = 16 cm $\times$ 14.5 cm $\times$ 8.5 cm) mimicking the human brain using non-magnetic, light-impermeable resins. Two vacant wells were engrafted inside the 3D human-brain phantom for cell cultures **(Fig. 30a**, **b**). HEK293 cells were co-transfected with Cre and FLEX-Piezo1 and FACS enriched for robust Cre-dependent Piezo1 expression. The sorted cells were labeled with m-Torquer conjugated with anti-Myc antibody, embedded in the Matrigel matrix, which has similar mechanical properties as neural tissues, and cultured inside the brain phantom. After the phantom was placed inside the 60 cm MMG setup, we provided 1 hr MMG stimulation and analyzed the cells for $Ca^{2+}$ influx-dependent activities by measuring *c-fos* mRNA expression. The MMG stimulation elicited a 2.6-fold increase in *c-fos* levels compared to the control group without m-Torquer **(Fig. 30c)**. In addition, the $Ca^{2+}$-dependent luciferase reporter assay resulted in a significantly higher (3.8-fold) expression of luciferase in Piezo1-expressing cells in response to MMG stimulation than the controls in the absence of m-Torquer **(Fig. 30d**, **e**). With the working distance of a sub-meter scale (up to 60 cm), which is significantly greater than that of MTG, our MMG system allows for wireless neural stimulation at clinically relevant tissue depth in any part of the brain, with potential for the study of larger animals such as primates and translational applications.

In summary, this example demonstrates that the m-Torquer conjugate-based drug of the present invention enables deep brain stimulation without implantation or tethering in freely moving mice. This is achieved through the targeted administration of the m-Torquer conjugate-based drug, which activates lateral hypothalamic (LH) neurons ectopically expressing the mechanosensitive transient receptor Piezo1. The Piezo1-expressing neurons in the LH of mice can be activated by a rotating circular magnet array (CMA) positioned over 70 cm above the animal's head, a distance greater than the bore size of standard human magnetic resonance imaging (MRI) devices, in conjunction with the m-Torquer conjugate-based drug, which functions as a nanoscale magnetic torque actuator within the magnetic field. Through this approach, we identified neural circuits in the LH that play a critical role in feeding-related behaviors. Specifically, the m-Torquer conjugate-based drug enables Piezo1-mediated stimulation of LH neurons, which in turn modulates feeding behavior. Vgat-positive inhibitory neurons in the LH promote feeding, whereas Vglut2-positive neurons induce feeding aversion. Furthermore, the m-Torquer conjugate-based drug demonstrates that the activation of its drug target proteins in the deep brain via magnetic stimulation can regulate other behaviors, such as social interaction. Additionally, in diet-induced obese (DIO) mice, we confirmed that Vglut2-specific magnetogenetic neuromodulation mediated by the m-Torquer conjugate-based drug suppresses food intake and alleviates obesity.

## Claims

1. An m-Torquer conjugate-based drug comprising:

   (a) a magnetic nanoparticle that generates rotational force upon application of a controlled rotating magnetic field, wherein the working distance from the rotating magnetic field generator is at least 1 cm, preferably at least 2 cm, 10 cm, more preferably at least 20 cm, 30 cm, and most preferably at least 60 cm or 70 cm;
   (b) a binding moiety that binds to a drug target protein present in the biological lipid membrane; and
   (c) a linker or a direct attachment between the magnetic nanoparticle and the binding moiety,

   wherein, when the m-Torquer conjugate binds to the drug target protein, the rotational force generated by the magnetic nanoparticle upon application of a controlled rotating magnetic field is transmitted to the drug target protein, inducing a conformational change in the drug target protein.

2. The m-Torquer conjugate-based drug of claim 1, wherein the magnetic nanoparticles that generate rotational force in the m-Torquer conjugate have a working distance of at least 20 cm from the rotating magnetic field generator.

3. The m-Torquer conjugate-based drug of claim 1, wherein the conformational change or modulation of the biological activity of the drug target protein, induced by the rotational force of the magnetic nanoparticles upon application of a controlled rotating magnetic field, is reversible, and wherein the activity of the drug target protein is regulated with a desired level of temporal precision and resolution by delivering a magnetic stimulation pattern that governs the conformational change of the drug target protein bound to the m-Torquer conjugate.

4. The m-Torquer conjugate-based drug of claim 1, wherein the conformational change or modulation of the biological activity of the drug target protein, induced by the rotational force of the magnetic nanoparticles upon application of a controlled rotating magnetic field, is reversible, and wherein the pharmacological efficacy of the m-Torquer conjugate-based drug is regulated by delivering magnetic stimulation to the drug target protein according to a predetermined pattern of the rotating magnetic field.

5. The m-Torquer conjugate-based drug of claim 1, wherein, when the drug target protein is expressed on the cell membrane of a neuronal cell, the m-Torquer conjugate bound to the drug target protein modulates neural circuit activity or behavior in a freely moving animal within the applied rotating magnetic field.

6. The m-Torquer conjugate-based drug of claim 1, wherein, when the drug target protein is expressed on the cell membrane of a specific neuronal cell within a defined brain region, the magnetic stimulation pattern, generated by the application of a controlled rotating magnetic field, selectively activates and/or inhibits the neuronal cell, thereby regulating the release and concentration of neurotransmitters that mediate neural signals to other brain regions.

7. The m-Torquer conjugate-based drug of claim 1, wherein the m-Torquer conjugate-based drug targets a naturally or artificially expressed ion-transport membrane protein, and upon application of a controlled rotating magnetic field, the ion-transport membrane protein is activated and/or inhibited, thereby modulating the electrical polarization of the cell membrane.

8. The m-Torquer conjugate-based drug of claim 1, wherein the m-Torquer conjugate-based drug targets a naturally or artificially expressed drug target protein in a postsynaptic cell and, upon application of a controlled rotating magnetic field in a predetermined pattern, it magnetically stimulates the drug target protein to modulate the firing patterns of action potentials in the postsynaptic cell.

9. The m-Torquer conjugate-based drug of claim 1, wherein the m-Torquer conjugate-based drug targets excitatory or inhibitory neurotransmitter receptors naturally or artificially expressed in postsynaptic neurons, and upon application of a controlled rotating magnetic field in a predetermined pattern, activates and/or inhibits the excitatory or inhibitory neurotransmitter receptor.

10. The m-Torquer conjugate-based drug of claim 1, wherein the m-Torquer conjugate-based drug targets excitatory neurotransmitter receptors naturally or artificially expressed in postsynaptic neurons, and upon application of a controlled rotating magnetic field in a predetermined pattern, inhibits the stimulation of excitatory neurotransmitter receptors to prevent neuronal death or injury in postsynaptic neurons.

11. The m-Torquer conjugate-based drug of claim 1, wherein the m-Torquer conjugate-based drug selectively activates and/or inhibits glutamatergic neurons, GABAergic neurons, cholinergic neurons, and/or dopaminergic neurons upon application of a controlled rotating magnetic field in a predetermined pattern.

12. The m-Torquer conjugate-based drug of claim 1, wherein the m-Torquer conjugate-based drug targets voltage-gated calcium ion channels expressed in presynaptic neurons and, upon the application of a controlled rotating magnetic field in a predetermined pattern, magnetically stimulates the voltage-gated calcium ion channels, thereby inducing the release of neurotransmitters into the postsynaptic neuron or target cell to trigger or inhibit neural activity in the postsynaptic neuron or target cell.

13. The m-Torquer conjugate-based drug of claim 1, wherein, for neuromodulation, the m-Torquer conjugate-based drug targets specific neurons in the brain and/or spinal cord, and, upon the application of a controlled rotating magnetic field in a predetermined pattern, magnetically stimulates them to form neural circuit loops associated with the desired brain function.

14. The m-Torquer conjugate-based drug of claim 1, wherein, upon the application of a controlled rotating magnetic field in a predetermined pattern, the m-Torquer conjugate-based drug modulates animal behavior or performs long-term neuron-specific neuromodulation for the treatment of physiological disorders or diseases.

15. The m-Torquer conjugate-based drug of claim 1, wherein, for neuromodulation, the m-Torquer conjugate-based drug targets, via the binding moiety (b), (i) central nervous system (CNS) neurons that regulate metabolism by releasing endocrine neural signals, or (ii) peripheral nervous system (PNS) neurons that relay CNS-derived neural signals to organs, wherein a controlled rotating magnetic field applied in a predetermined pattern induces magnetic stimulation of the targeted neurons.

16. The m-Torquer conjugate-based drug of claim 1, wherein, upon the non-invasive application of a rotating magnetic field, the m-Torquer conjugate induces magnetic stimulation of targeted neurons, secretory cells, muscle cells, or cancer cells via mechanical rotational force, thereby functioning as a biophysiological modulation agent, behavioral regulation agent, or anticancer agent.

17. The m-Torquer conjugate-based drug of claim 1, wherein, upon the non-invasive application of a rotating magnetic field, the m-Torquer conjugate induces magnetic stimulation of spinal cord neurons, thereby functioning as a pain-modulating agent or behavior-modulating agent.

18. The m-Torquer conjugate-based drug of claim 1, wherein, when the m-Torquer conjugate-based drug targets a drug target protein present in the vagus nerve, which detects and regulates systemic inflammation, or in a postsynaptic target cell synapsing with the vagus nerve, the non-invasive application of a rotating magnetic field induces magnetic stimulation of the drug target protein, thereby modulating cytokine production.

19. The m-Torquer conjugate-based drug of claim 1, wherein, when the m-Torquer conjugate-based drug targets a drug target protein present in the vagus nerve innervating the pancreas or in a postsynaptic target cell synapsing with the vagus nerve, the non-invasive application of a rotating magnetic field induces magnetic stimulation of the drug target protein, thereby regulating insulin secretion, pancreatic β-cell proliferation, and/or insulin sensitivity.

20. The m-Torquer conjugate-based drug of claim 1, wherein the drug target protein present in the biological lipid membrane is a receptor undergoing a conformational change upon ligand binding.

21. The m-Torquer conjugate-based drug of claim 1, wherein, upon the controlled application of a rotating magnetic field in a predetermined pattern, the conformational change induced in the m-Torquer-conjugated drug target protein enables the m-Torquer conjugate-based drug to function as an agonist, antagonist, activator, inhibitor, or blocker of the drug target protein, thereby preventing or treating a disease associated with the drug target protein.

22. The m-Torquer conjugate-based drug of claim 1, wherein the binding moiety (b) of the m-Torquer conjugate is a ligand, receptor, antibody, antigen-binding domain, lipibody, aptamer, or a protein tag-binding moiety, wherein the binding moiety is specific to a natural or genetically engineered drug target protein.

23. The m-Torquer conjugate-based drug of claim 1, wherein the m-Torquer conjugate-based drug modulates the gating of an ion channel as the drug target protein.

24. The m-Torquer conjugate-based drug of claim 1, wherein the binding moiety (b) is configured to selectively bind to a drug target protein expressed on a specific type of brain cell located in deep brain regions.

25. The m-Torquer conjugate-based drug of claim 1, wherein the drug target protein selectively targeted by the m-Torquer conjugate-based drug is naturally or artificially expressed in a specific type of brain cell.

26. The m-Torquer conjugate-based drug of claim 1, wherein the m-Torquer conjugate-based drug regulates feeding behavior and food intake in animals by modulating neuronal activity in the lateral hypothalamus (LH), the central hub of appetite regulation.

27. The m-Torquer conjugate-based drug of claim 1, wherein the m-Torquer conjugate-based drug enables sustained magnetic stimulation for a period exceeding 10 days.

28. The m-Torquer conjugate-based drug of any one of claims 1 to 27, wherein the m-Torquer conjugate, upon binding to a drug target protein, enables non-invasive, tetherless, and localized magnetic stimulation of cells expressing the drug target protein within the rotating magnetic field space of a freely moving animal.

29. The m-Torquer conjugate-based drug of any one of claims 1 to 27, wherein the drug target protein, to which the m-Torquer conjugate binds, is expressed in specific cell types via a gene delivery carrier that enables targeted gene expression in a specific tissue region and cell population in vivo, thereby modulating the physiological or molecular functions of the target cells.

30. A pharmaceutical composition for neuromodulation, comprising the m-Torquer conjugate-based drug of any one of claims 1 to 27.

31. The pharmaceutical composition for neuromodulation of claim 30, wherein the composition is designed to inhibit neural activity by activating an anion transport membrane protein.

32. The pharmaceutical composition for neuromodulation of claim 30, wherein the composition is formulated for the treatment of epilepsy, attention-deficit/hyperactivity disorder (ADHD), chronic pain disorders, or the suppression of pain perception.

33. A pharmaceutical composition for inducing weight loss or treating obesity, comprising the m-Torquer conjugate-based drug of any one of claims 1 to 27.

34. A pharmaceutical composition for regulating physiological functions or modulating behavior, comprising the m-Torquer conjugate-based drug of any one of claims 1 to 27.

35. A pharmaceutical composition for the prevention or treatment of metabolic diseases, comprising the m-Torquer conjugate-based drug of any one of claims 1 to 27, wherein the m-Torquer conjugate-based drug is configured to remotely and reversibly deliver mechanical torque-based magnetic stimulation over an extended period upon application of a controlled rotating magnetic field generated by a rotating magnetic field generator.

36. A pharmaceutical composition for inducing intracellular anion influx, comprising the m-Torquer conjugate-based drug of any one of claims 1 to 27.

37. The pharmaceutical composition of any one of claims 30 to 36, wherein the m-Torquer conjugate-based drug is co-administered with a carrier that delivers the gene encoding a drug target protein capable of binding to the m-Torquer conjugate, thereby enabling targeted expression of the drug target protein in a specific cell type within a specific anatomical region.

38. The pharmaceutical composition of any one of claims 30 to 36, wherein the composition is co-administered with a viral vector engineered to introduce the gene information of an ion transport membrane protein into target cells, thereby enabling its expression.

39. A kit comprising:

(i) the m-Torquer conjugate-based drug of any one of claims 1 to 27, and
(ii) a carrier engineered to deliver a gene encoding a drug target protein, to which the m-Torquer conjugate can bind, wherein the carrier enables selective expression of the drug target protein in a specific population of cells within a defined region in vivo.

[Fig. 1]

[Fig. 2]

[Fig. 3]

Magnetic particles generating
rotational force
(100-3000 nm)

Magnet arrangement
(multiple permanent magnets or electromagnets)

[Fig. 4]

[Fig. 5]

Neuromodulation methods

Neuronal activity

Yang et al., Adv Mater, 2021

**Optogenetics**

**Magnetogenetics**

Induction of neural activity

Inhibition of neural activity

Induction of neural activity

Inhibition of neural activity

Deisseroth, Nat. Neuro., 2015

Lee et al, Nat. Materials., 2021

[Fig. 6]

[Fig. 7]

Magnetic particle

Rotational force

membrane

Anion transport membrane protein

Anion

Examples of binding mechanisms :
Antigen-antibody reaction
Chemical covalent bonding
Chemical coordination bonding
Receptor-ligand binding
Enzymatic reaction binding
Electrostatic interactions, etc.

Changes in cell polarization

**Neuronal inhibition**

RMF stimulation

Current injection

Silence

In culture neuron

20mV
10s

~70% AP silenced

INO (mv)
20
0
-20
-40
-60
11000 111000
Time (ms)

INO (mv)
20
0
-20
-40
-60
91100 91200
Time (ms)

Current injection
RMF stimulation

[Fig. 8]

[Fig. 9]

nerve cell

Anion level

Neural activity

Magnetic field

Regulation of feeding behavior, etc.

[Fig. 10]

Dionaea muscipula (Fly Catcher)

FlyC1 channel

A  DmFLYC1

-70 mmHg

Cell-attached

20 pA

500 ms

Procko et al., eLife, 2021

- A novel **mechanosensitive chloride channel FlyC1** has recently identified from **touch sensory cell** of Fly Catcher

- FlyC1 mechanically **trans-conduct [Cl⁻] ions** and **hyperpolarize membrane potential** (i.e. inhibit action potential)

- Gene of FlyC1 has **further mutated for better mechanosensitivity (V656L, T659L)**

[Fig. 11]

[Fig. 12]

Candidate binding sites for MNP (Magnetic Nanoparticle)

eLife

Stretch-activated ion channels identified in the touch-sensitive structures of carnivorous Droseraceae plants

Carl Procko, Swetha Murthy, William T Keenan, Seyed Ali Reza Mousavi, Tsegaye Dabi, Adam Coombs, Erik Procko, Lisa Baird, Ardem Patapoutian, Joanne Chory

MNP

Extracellular

Intracellular
· conserved
· high similarity
· weak similarity
· Predicted pore
COOH  domain

Cell          DmFLYC-MNP binding

[Fig. 13]

[Fig. 14]

[Fig. 15]

Naïve DmFLYC1 + MNP     DmFLYC1-Myc + MNP

Extracellular

Intracellular

RFP

Red: DmFLYC1-mCherry    Green: MNP-Alx647 (no pemeabilization)

[Fig. 16]

[Fig. 17]

[Fig. 18]

## 1. Cre-Loxp System for cell-specific magnetic control

Viral injection

Cre+ Cre- Cre-

lox2722 lox2722

EF1α eYFP ChR2

Reversible Cre-mediated recombination

loxP loxP

EF1α ChR2 eYFP

Permanent Cre-mediated lox site excision

EF1α ChR2 eYFP

loxP lox2722

EF1α ChR2 eYFP

Transgenic parvalbumin::Cre

eYFP

Parvalbumin

Overlay

30µm

Fenno, *Annu. Rev. Neurosci.* (2011)

## 2. Long-term, Deep brain neuromodulation

Piezo1   m-Torquer

**LH: lateral hypothalamus**
Important for diverse behaviors including motivation, rewards...

LH

## 3. Magnetic control of feeding in freely moving mice

LH Vglut2 neurons

$Ca^{2+}$   m-Torquer
Myc
Piezo1
$Ca^{2+}$

↓ Food consumption
↓ Weight changes

LH Vgat neurons

$Ca^{2+}$   m-Torquer
Myc
Piezo1
$Ca^{2+}$

↑ Food consumption
↑ Weight changes

SN food SN

MG stimulation

LH Vglut2
(Glutamate) neurons

⚡ activate

VTA GABA
neurons ↑

VTA Dopamine
neurons ↓

Feeding ↓

LH Vgat
(GABA) neurons

⊥ inhibit

VTA GABA
neurons ↓

VTA Dopamine
neurons ↑

Feeding ↑

## EP 4 574 132 A1

[Fig. 19]

a. Generation of Cre-mice

b. Cell-specific MG (Piezo1, m-Torquer)

c. *in vivo* feeding control

d. *in vivo* social behavior control

[Fig. 20]

66

[Fig. 21]

[Fig. 22]

[Fig. 23]

**a** Social behavior test

Vgat-cre mice
60 cm
hSyn loxN/lox2722
LH
VTA
LHA
Vgat specific
MMG stimulation

**b** Sociality
Empty    M1
Novel mouse 1

**c**
Piezo1+ m-Torquer− MF+    Piezo1+ m-Torquer+ MF+
Empty    M1    Empty    M1
6 cm
short ▬▬▬ long
Time spent

**d**
Piezo1+ m-Torquer− MF+
Piezo1+ m-Torquer+ MF+
Interaction time (s)
Empty    Mouse1
* **

**e** Social novelty
M2    M1
Novel mouse 2    Familar mouse

**f**
Piezo1+ m-Torquer− MF+    Piezo1+ m-Torquer+ MF+
M2    M1    M2    M1
6 cm
short ▬▬▬ long
Time spent

**g**
Piezo1+ m-Torquer− MF+
Piezo1+ m-Torquer+ MF+
Interaction time (s)
Mouse1    Mouse2
* **

**h** Sociality
Empty    Novel mouse 1
Multiple animals

**i**
Piezo1+ m-Torquer− MF+    Piezo1+ m-Torquer+ MF+
Empty    M1    Empty    M1
── mouse 1   ── mouse 2

**j** Social novelty
Novel mouse 2    Familar mouse
Multiple animals

**k**
Piezo1+ m-Torquer− MF+    Piezo1+ m-Torquer+ MF+
M2    M1    M2    M1
── mouse 1   ── mouse 2

70

[Fig. 24]

[Fig. 25]

[Fig. 26]

[Fig. 27]

[Fig. 28]

[Fig. 29]

| | Piezo1+<br>m-Torquer– | | Piezo1+<br>m-Torquer+ | |
|---|---|---|---|---|
| | W/O RMF | W/ RMF 2 week | W/O RMF | W/ RMF 2 week |

[Fig. 30]

[Fig. 31]

[Fig. 32]

[Fig. 33]

(a)

(b)

(c)

(a)

(b)

## INTERNATIONAL SEARCH REPORT

| International application No. |
| --- |
| **PCT/KR2023/012209** |

| A. | CLASSIFICATION OF SUBJECT MATTER |
| --- | --- |

**A61K 9/00**(2006.01)i; **A61K 41/00**(2006.01)i; **A61P 25/00**(2006.01)i; **A61P 3/04**(2006.01)i; **A61P 25/08**(2006.01)i

According to International Patent Classification (IPC) or to both national classification and IPC

| B. | FIELDS SEARCHED |
| --- | --- |

Minimum documentation searched (classification system followed by classification symbols)

A61K 9/00(2006.01); A61K 41/00(2006.01); A61N 2/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 자기토크(magnetic torque), 약물 표적 단백질(drug target protein), 작동거리 (working distance), 신경조절(neuromodulation), 이온 채널(ion channel)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | DEL SOL-FERNANDEZ, S. et al. Magnetogenetics: remote activation of cellular functions triggered by magnetic switches. Nanoscale. 15 November 2021 (published online), vol. 14, pp. 2091-2118. | 1-39 |
| A | WILLIS, A. J. et al. Rotating Magnetic Nanoparticle Clusters as Microdevices for Drug Delivery. International Journal of Nanomedicine. 2020, vol. 15, pp. 4105-4123.<br>　　See entire document. | 1-39 |
| A | SONG, M. et al. Development of Magnetic Torque Stimulation (MTS) Utilizing Rotating Uniform Magnetic Field for Mechanical Activation of Cardiac Cells. Nanomaterials. 2020, vol. 10, no. 1684, inner pp. 1-13.<br>　　See entire document. | 1-39 |
| A | HUSSAIN, S. I. et al. Parallel Multichannel Assessment of Rotationally Manipulated Magnetic Nanoparticles. Nanotechnology, Science and Applications. 19 April 2022, vol. 15, inner pp. 1-15.<br>　　See entire document. | 1-39 |

☑ Further documents are listed in the continuation of Box C.    ☑ See patent family annex.

| | | |
| --- | --- | --- |
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
| --- | --- |
| **20 November 2023** | **21 November 2023** |

| Name and mailing address of the ISA/KR | Authorized officer |
| --- | --- |
| **Korean Intellectual Property Office**<br>**Government Complex-Daejeon Building 4, 189 Cheongsa-**<br>**ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

International application No.

**PCT/KR2023/012209**

### C. DOCUMENTS CONSIDERED TO BE RELEVANT

| Category* | Citation of document, with indication, where appropriate, of the relevant passages | Relevant to claim No. |
|---|---|---|
| A | US 2016-0367668 A1 (MEMORIAL SLOAN KETTERING CANCER CENTER) 22 December 2016 (2016-12-22)<br>    See entire document. | 1-39 |

## INTERNATIONAL SEARCH REPORT

| International application No. |
| --- |
| **PCT/KR2023/012209** |

**Box No. I          Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

  a.  ☑ forming part of the international application as filed.

  b.  ☐ furnished subsequent to the international filing date for the purposes of international search (Rule 13*ter*.1(a)),

       ☐ accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

**INTERNATIONAL SEARCH REPORT**
Information on patent family members

International application No.

**PCT/KR2023/012209**

| Patent document cited in search report | | | Publication date (day/month/year) | Patent family member(s) | | | Publication date (day/month/year) |
|---|---|---|---|---|---|---|---|
| US | 2016-0367668 | A1 | 22 December 2016 | EP | 3113760 | A1 | 11 January 2017 |
| | | | | EP | 3344334 | A1 | 11 July 2018 |
| | | | | US | 10912947 | B2 | 09 February 2021 |
| | | | | WO | 2015-134620 | A1 | 11 September 2015 |
| | | | | WO | 2017-040915 | A1 | 09 March 2017 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• KR 1020220100260

**Non-patent literature cited in the description**

• Nature Materials (2022). *Nat Mater*, July 2021, vol. 20 (7), 1029-1036